# EUROPEAN PATENT APPLICATION

(11) **EP 4 215 212 A1**
(43) Date of publication of application: **26.07.2023**
(21) Application number: 21869419.8
(22) Date of filing: 16.09.2021
(51) Int. Cl.: A61K 45/00, A61K 48/00, A61P 3/04, A61P 3/10, A61P 25/16, A61P 25/28, A61P 27/02, A61P 27/06, A61P 31/12, A61P 43/00, C12N 15/12, C12N 15/31, C12N 15/62, A61K 38/16, A61K 38/17

(54) **COMPOSITION FOR TREATING OR PREVENTING DISEASES, DISORDERS, OR CONDITIONS ASSOCIATED WITH ENDOPLASMIC RETICULUM STRESS OR ALL-TRANS-RETINAL, PROTECTING RETINAL THICKNESS, OR SUPPRESSING REDUCTION IN RETINAL THICKNESS OR PROGRESS OF REDUCTION IN RETINAL THICKNESS**

(30) Priority: 17.09.2020 JP 2020156757
(71) Applicant: Restore Vision Inc., Tokyo, 105-6415 (JP)
(72) Inventor: KURIHARA, Toshihide, Tokyo 160-8582 (JP); KATADA, Yusaku, Tokyo 160-8582 (JP); TSUBOTA, Kazuo, Tokyo 160-8582 (JP)
(74) Representative: Uexküll & Stolberg
(86) International application number: PCT/JP2021/034143
(87) International publication number: WO 2022/059736

(57) **Abstract**

The present disclosure provides: a composition having an effect of preventing, suppressing the progress of, or treating a disease associated with an endoplasmic reticulum stress, such as a vesicle transport disorder, and a disorder such as cell death due to all-trans-retinal toxicity; and/or a composition for use in protecting the retina. A composition for use in treating or preventing a disease, disorder or symptom associated with an endoplasmic reticulum stress, the composition comprising a retinal modulating agent; a composition for use in treating or preventing a disease, disorder or symptom associated with all-trans-retinal, the composition comprising a retinal modulating agent; and a composition for use in protecting the retina, the composition comprising a retinal modulating agent, are provided. Use of a retinal modulating agent modulates a cis/trans ratio of retinal, thereby reducing the amount or concentration of all-trans-retinal resulting from photoreception of rhodopsin, where a therapeutic effect for diseases associated with endoplasmic reticulum stress, etc., or a disease associated with all-trans-retinal, and a protective effect for the retina can be expected.

## Description

### [Technical Field]

The present disclosure relates to techniques and methods for treating or preventing diseases, disorders or symptoms associated with an endoplasmic reticulum stress, and compositions therefor. The present disclosure also relates to techniques and methods for treating or preventing diseases, disorders or symptoms associated with all-trans-retinal, and compositions therefor. The present disclosure further relates to techniques and methods for protecting retinal thickness or suppressing atrophy, or progress of atrophy, of a retinal thickness, and compositions therefor.

### [Background Art]

Rhodopsin is a photosensitive receptor with a seven-time-transmembrane structure in the retina of humans and animals, and rhodopsin is also applied in medicine.

### [Summary of Invention]

### [Solution to Problem]

The present inventors have found that adjusting the amount, concentration, or cis/trans ratio of retinal, which constitutes rhodopsin, has preventive, progress-suppressing, or therapeutic effects on diseases associated with an endoplasmic reticulum stress, such as vesicle transport disorders, and disorders such as cell death due to all-trans-retinal toxicity. The present inventors have also found that a light absorbing substance has preventive, progress-suppressing, or therapeutic effects on diseases associated with an endoplasmic reticulum stress, disorders such as cell death due to all-trans-retinal toxicity, and diseases, disorders, etc., associated with retinal thickness. The present inventors have also found that a neural activity signaling agent or device has preventive, progress-suppressing, or therapeutic effects on diseases associated with an endoplasmic reticulum stress, disorders such as cell death due to all-trans-retinal toxicity, and diseases, disorders, etc., associated with retinal thickness. Furthermore, the present inventors have found that opsins (rhodopsin), such as microbial opsins, that do not release retinal upon photoreception, protect retinal thickness or suppress atrophy, or the progress of atrophy, of a retinal thickness, and therefore, the present inventors have completed the present invention.

Accordingly, the present disclosure provides the following:
(Item 1)
   A composition for use in treating or preventing a disease, disorder or symptom associated with an endoplasmic reticulum stress, the composition comprising a retinal modulating agent.
(Item 2)
   A composition for use in treating or preventing a disease, disorder or symptom associated with an endoplasmic reticulum stress, the composition comprising a light absorbing substance.
(Item 3)
   A composition for use in treating or preventing a disease, disorder or symptom associated with an endoplasmic reticulum stress, the composition comprising a neural activity signaling agent or device.
(Item 4)
   A composition for use in reducing or eliminating an endoplasmic reticulum stress, the composition comprising a light absorbing substance.
(Item 5)
   A composition for use in reducing or eliminating an endoplasmic reticulum stress, the composition comprising a neural activity signaling agent or device.
(Item 6)
   A composition for use in treating or preventing a disease, disorder or symptom associated with an endoplasmic reticulum stress, the composition comprising an opsin or a derivative thereof, or a nucleic acid molecule encoding the same.
(Item 7)
   A composition for use in ameliorating a rhodopsin transport disorder, the composition comprising a retinal modulating agent.
(Item 8)
   A composition for use in ameliorating a rhodopsin transport disorder, the composition comprising a light absorbing substance.
(Item 9)
   A composition for use in ameliorating a rhodopsin transport disorder, the composition comprising a neural activity signaling agent or device.
(Item 10)
   The composition of any one of the above Items, wherein the retinal modulating agent, the light absorbing substance, the neural activity signaling agent or device, or the opsin or derivative thereof modulates a cis/trans ratio of retinal in an organism body of interest.
(Item 11)
   The composition of any one of the above Items, wherein the retinal modulating agent, the light absorbing substance, the neural activity signaling agent or device, or the opsin or derivative thereof modulates a retinal concentration in an organism body of interest.
(Item 12)
   The composition of any one of the above Items, wherein the disease, disorder or symptom associated with the endoplasmic reticulum stress includes vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease.
(Item 13)
   The composition of any one of the above Items, characterized in that the composition is for treating or preventing a disease, disorder or symptom associated with an endoplasmic reticulum stress, for reducing or eliminating an endoplasmic reticulum stress, or for ameliorating a rhodopsin transport disorder, in the retina.
(Item 14)
   The composition of any one of the above Items, characterized in that the composition is administered to a subject prior to or after onset of the disease, disorder or symptom.
(Item 15)
   The composition of any one of the above Items, characterized in that the composition is administered once during a treatment period.
(Item 16)
   The composition of any one of the above Items, wherein the retinal modulating agent, the light absorbing substance, the neural activity signaling agent or device, or the opsin or derivative thereof is a rhodopsin that does not release retinal upon photoreception.
(Item 17)
   The composition of any one of the above Items, wherein the retinal modulating agent, the light absorbing substance, the neural activity signaling agent or device, or the opsin or derivative thereof is an ion-transporting receptor rhodopsin.
(Item 18)
   The composition of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item 19)
   The composition of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item 20)
   The composition of any one of the above Items, wherein the ion-transporting receptor rhodopsin is derived from cyanobacteria (blue-green bacteria).
(Item 21)
   The composition of any one of the above Items, wherein the G protein-coupled receptor rhodopsin is derived from a mammal.
(Item 22)
   A composition for use in treating or preventing a disease, disorder or symptom associated with all-trans-retinal, the composition comprising a retinal modulating agent.
(Item 23)
   A composition for use in treating or preventing a disease, disorder or symptom associated with all-trans-retinal, the composition comprising a light absorbing substance.
(Item 24)
   A composition for use in treating or preventing a disease, disorder or symptom associated with all-trans-retinal, the composition comprising a neural activity signaling agent or device.
(Item 25)
   A composition for use in reducing or eliminating all-trans-retinal toxicity, the composition comprising a light absorbing substance.
(Item 26)
   A composition for use in reducing or eliminating all-trans-retinal toxicity, the composition comprising a neural activity signaling agent or device.
(Item 27)
   A composition for use in treating or preventing a disease, disorder or symptom associated with all-trans-retinal, the composition comprising an opsin or a derivative thereof, or a nucleic acid molecule encoding the same.
(Item 28)
   A composition for use in inhibiting cell death or apoptosis, the composition comprising a retinal modulating agent.
(Item 29)
   A composition for use in inhibiting cell death or apoptosis, the composition comprising a light absorbing substance.
(Item 30)
   A composition for use in inhibiting cell death or apoptosis, the composition comprising a neural activity signaling agent or device.
(Item 31)
   A composition for use in inhibiting cell death or apoptosis, the composition comprising an opsin or a derivative thereof, or a nucleic acid molecule encoding the same.
(Item 32)
   The composition of any one of the above Items, wherein the retinal modulating agent, the light absorbing substance, the neural activity signaling agent or device, or the opsin or derivative thereof modulates a cis/trans ratio of retinal in an organism body of interest.
(Item 33)
   The composition of any one of the above Items, wherein the retinal modulating agent, the light absorbing substance, the neural activity signaling agent or device, or the opsin or derivative thereof modulates a retinal concentration in an organism body of interest.
(Item 34)
   The composition of any one of the above Items, wherein the disease, disorder or symptom associated with all-trans-retinal comprises cell death or apoptosis.
(Item 35)
   The composition of any one of the above Items, characterized in that the composition is for treating or preventing a disease, disorder or symptom associated with all-trans-retinal, for reducing or eliminating all-trans-retinal toxicity, or for inhibiting cell death or apoptosis.
(Item 36)
   The composition of any one of the above Items, characterized in that the composition is administered to a subject prior to or after onset of the disease, disorder or symptom.
(Item 37)
   The composition of any one of the above Items, characterized in that the composition is administered once during a treatment period.
(Item 38)
   The composition of any one of the above Items, wherein the retinal modulating agent, the light absorbing substance, the neural activity signaling agent or device, or the opsin or derivative thereof is a rhodopsin that does not release retinal upon photoreception.
(Item 39)
   The composition of any one of the above Items, wherein the retinal modulating agent, the light absorbing substance, the neural activity signaling agent or device, or the opsin or derivative thereof is an ion-transporting receptor rhodopsin.
(Item 40)
   The composition of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item 41)
   The composition of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item 42)
   The composition of any one of the above Items, wherein the ion-transporting receptor rhodopsin is derived from cyanobacteria (blue-green bacteria).
(Item 43)
   The composition of any one of the above Items, wherein the G protein-coupled receptor rhodopsin is derived from a mammal.
(Item 44)
   A composition for use in protecting a retinal thickness, the composition comprising a retinal modulating agent.
(Item 45)
   A composition for use in protecting a retinal thickness, the composition comprising a light absorbing substance.
(Item 46)
   A composition for use in protecting a retinal thickness, the composition comprising a neural activity signaling agent or device.
(Item 47)
   A composition for use in suppressing atrophy, or progress of atrophy, of a retinal thickness, the composition comprising a retinal modulating agent.
(Item 48)
   A composition for use in suppressing atrophy, or progress of atrophy, of a retinal thickness, the composition comprising a light absorbing substance.
(Item 49)
   A composition for use in suppressing atrophy, or progress of atrophy, of a retinal thickness, the composition comprising a neural activity signaling agent or device.
(Item 50)
   A composition for use in protecting a retinal thickness, the composition comprising an opsin or a derivative thereof, or a nucleic acid molecule encoding the same.
(Item 51)
   A composition for use in suppressing atrophy, or progress of atrophy, of a retinal thickness, the composition comprising an opsin or a derivative thereof, or a nucleic acid molecule encoding the same.
(Item 52)
   The composition of any one of the above Items, wherein the retinal modulating agent, the light absorbing substance, the neural activity signaling agent or device, or the opsin or derivative thereof modulates a cis/trans ratio of retinal in an organism body of interest.
(Item 53)
   The composition of any one of the above Items, wherein the retinal modulating agent, the light absorbing substance, the neural activity signaling agent or device, or the opsin or derivative thereof modulates a retinal concentration in an organism body of interest.
(Item 54)
   The composition of any one of the above Items, wherein the protection of the retinal thickness, or the suppression of atrophy, or progress of atrophy, of the retinal thickness, comprises: protection of an inner retinal layer thickness; or suppression of atrophy, or progress of atrophy, of an inner retinal layer thickness.
(Item 55)
   The composition of any one of the above Items, characterized in that the composition is administered to a subject prior to or after onset of atrophy of a retinal thickness.
(Item 56)
   The composition of any one of the above Items, characterized in that the composition is administered once during a treatment period.
(Item 57)
   The composition of any one of the above Items, wherein the retinal modulating agent, the light absorbing substance, the neural activity signaling agent or device, or the opsin or derivative thereof is a rhodopsin that does not release retinal upon photoreception.
(Item 58)
   The composition of any one of the above Items, wherein the retinal modulating agent, the light absorbing substance, the neural activity signaling agent or device, or the opsin or derivative thereof is an ion-transporting receptor rhodopsin.
(Item 59)
   The composition of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item 60)
   The composition of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item 61)
   The composition of any one of the above Items, wherein the ion-transporting receptor rhodopsin is derived from cyanobacteria (blue-green bacteria).
(Item 62)
   The composition of any one of the above Items, wherein the G protein-coupled receptor rhodopsin is derived from a mammal.
(Item A1)
   A method for treating or preventing a disease, disorder or symptom associated with an endoplasmic reticulum stress in a subject, the method comprising the step of administering an effective amount of a retinal modulating agent to the subject in need thereof.
(Item A2)
   A method for treating or preventing a disease, disorder or symptom associated with an endoplasmic reticulum stress in a subject, the method comprising the step of administering an effective amount of a light absorbing substance to the subject in need thereof.
(Item A3)
   A method for treating or preventing a disease, disorder or symptom associated with an endoplasmic reticulum stress in a subject, the method comprising the step of administering an effective amount of a neural activity signaling agent or device to the subject in need thereof.
(Item A4)
   A method for reducing or eliminating an endoplasmic reticulum stress in a subject, the method comprising the step of administering an effective amount of a light absorbing substance to the subject in need thereof.
(Item A5)
   A method for reducing or eliminating an endoplasmic reticulum stress in a subject, the method comprising the step of administering an effective amount of a neural activity signaling agent or device to the subject in need thereof.
(Item A6)
   A method for treating or preventing a disease, disorder or symptom associated with an endoplasmic reticulum stress in a subject, the method comprising the step of administering an effective amount of an opsin or a derivative thereof, or a nucleic acid molecule encoding the same, to the subject in need thereof.
(Item A7)
   A method for ameliorating a rhodopsin transport disorder in a subject, the method comprising the step of administering an effective amount of a retinal modulating agent to the subject in need thereof.
(Item A8)
   A method for ameliorating a rhodopsin transport disorder in a subject, the method comprising the step of administering an effective amount of a light absorbing substance to the subject in need thereof.
(Item A9)
   A method for ameliorating a rhodopsin transport disorder in a subject, the method comprising the step of administering an effective amount of a neural activity signaling agent or device to the subject in need thereof.
(Item A10)
   The method of any one of the above Items, wherein the retinal modulating agent, the light absorbing substance, the neural activity signaling agent or device, or the opsin or derivative thereof modulates a cis/trans ratio of retinal in an organism body of interest.
(Item A11)
   The method of any one of the above Items, wherein the retinal modulating agent, the light absorbing substance, the neural activity signaling agent or device, or the opsin or derivative thereof modulates a retinal concentration in an organism body of interest.
(Item A12)
   The method of any one of the above Items, wherein the disease, disorder or symptom associated with the endoplasmic reticulum stress includes vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease.
(Item A13)
   The method of any one of the above Items, characterized in that the method is for treating or preventing a disease, disorder or symptom associated with an endoplasmic reticulum stress, for reducing or eliminating an endoplasmic reticulum stress, or for ameliorating a rhodopsin transport disorder, in the retina.
(Item A14)
   The method of any one of the above Items, characterized by administering the retinal modulating agent, the light absorbing substance, the neural activity signaling agent or device, or the opsin or derivative thereof, to a subject prior to or after onset of the disease, disorder or symptom.
(Item A15)
   The method of any one of the above Items, characterized by administering the retinal modulating agent, the light absorbing substance, the neural activity signaling agent or device, or the opsin or derivative thereof, once during a treatment period.
(Item A16)
   The method of any one of the above Items, wherein the retinal modulating agent, the light absorbing substance, the neural activity signaling agent or device, or the opsin or derivative thereof is a rhodopsin that does not release retinal upon photoreception.
(Item A17)
   The method of any one of the above Items, wherein the retinal modulating agent, the light absorbing substance, the neural activity signaling agent or device, or the opsin or derivative thereof is an ion-transporting receptor rhodopsin.
(Item A18)
   The method of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item A19)
   The method of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item A20)
   The method of any one of the above Items, wherein the ion-transporting receptor rhodopsin is derived from cyanobacteria (blue-green bacteria).
(Item A21)
   The method of any one of the above Items, wherein the G protein-coupled receptor rhodopsin is derived from a mammal.
(Item A22)
   A method for treating or preventing a disease, disorder or symptom associated with all-trans-retinal in a subject, the method comprising the step of administering an effective amount of a retinal modulating agent to the subject in need thereof.
(Item A23)
   A method for treating or preventing a disease, disorder or symptom associated with all-trans-retinal in a subject, the method comprising the step of administering an effective amount of a light absorbing substance to the subject in need thereof.
(Item A24)
   A method for treating or preventing a disease, disorder or symptom associated with all-trans-retinal in a subject, the method comprising the step of administering an effective amount of a neural activity signaling agent or device to the subject in need thereof.
(Item A25)
   A method for reducing or eliminating all-trans-retinal toxicity in a subject, the method comprising the step of administering an effective amount of a light absorbing substance to the subject in need thereof.
(Item A26)
   A method for reducing or eliminating all-trans-retinal toxicity in a subject, the method comprising the step of administering an effective amount of a neural activity signaling agent or device to the subject in need thereof.
(Item A27)
   A method for treating or preventing a disease, disorder or symptom associated with all-trans-retinal in a subject, the method comprising the step of administering an effective amount of an opsin or a derivative thereof, or a nucleic acid molecule encoding the same, to the subject in need thereof.
(Item A28)
   A method for inhibiting cell death or apoptosis in a subject, the method comprising the step of administering an effective amount of a retinal modulating agent to the subject in need thereof.
(Item A29)
   A method for inhibiting cell death or apoptosis in a subject, the method comprising the step of administering an effective amount of a light absorbing substance to the subject in need thereof.
(Item A30)
   A method for inhibiting cell death or apoptosis in a subject, the method comprising the step of administering an effective amount of a neural activity signaling agent or device to the subject in need thereof.
(Item A31)
   A method for inhibiting cell death or apoptosis in a subject, the method comprising the step of administering an effective amount of an opsin or a derivative thereof, or a nucleic acid molecule encoding the same, to the subject in need thereof.
(Item A32)
   The method of any one of the above Items, wherein the retinal modulating agent, the light absorbing substance, the neural activity signaling agent or device, or the opsin or derivative thereof modulates a cis/trans ratio of retinal in an organism body of interest.
(Item A33)
   The method of any one of the above Items, wherein the retinal modulating agent, the light absorbing substance, the neural activity signaling agent or device, or the opsin or derivative thereof modulates a retinal concentration in an organism body of interest.
(Item A34)
   The method of any one of the above Items, wherein the disease, disorder or symptom associated with all-trans-retinal comprises cell death or apoptosis.
(Item A35)
   The method of any one of the above Items, characterized in that the method is for treating or preventing a disease, disorder or symptom associated with all-trans-retinal, for reducing or eliminating all-trans-retinal toxicity, or for inhibiting cell death or apoptosis, in the retina.
(Item A36)
   The method of any one of the above Items, characterized by administering the retinal modulating agent, the light absorbing substance, the neural activity signaling agent or device, or the opsin or derivative thereof, to a subject prior to or after onset of the disease, disorder or symptom.
(Item A37)
   The method of any one of the above Items, characterized by administering the retinal modulating agent, the light absorbing substance, the neural activity signaling agent or device, or the opsin or derivative thereof, once during a treatment period.
(Item A38)
   The method of any one of the above Items, wherein the retinal modulating agent, the light absorbing substance, the neural activity signaling agent or device, or the opsin or derivative thereof is a rhodopsin that does not release retinal upon photoreception.
(Item A39)
   The method of any one of the above Items, wherein the retinal modulating agent, the light absorbing substance, the neural activity signaling agent or device, or the opsin or derivative thereof is an ion-transporting receptor rhodopsin.
(Item A40)
   The method of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item A41)
   The method of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item A42)
   The method of any one of the above Items, wherein the ion-transporting receptor rhodopsin is derived from cyanobacteria (blue-green bacteria).
(Item A43)
   The method of any one of the above Items, wherein the G protein-coupled receptor rhodopsin is derived from a mammal.
(Item A44)
   A method for protecting a retinal thickness in a subject, the method comprising the step of administering an effective amount of a retinal modulating agent to the subject in need thereof.
(Item A45)
   A method for protecting a retinal thickness in a subject, the method comprising the step of administering an effective amount of a light absorbing substance to the subject in need thereof.
(Item A46)
   A method for protecting a retinal thickness in a subject, the method comprising the step of administering an effective amount of a neural activity signaling agent or device to the subject in need thereof.
(Item A47)
   A method for suppressing atrophy, or progress of atrophy, of a retinal thickness in a subject, the method comprising the step of administering an effective amount of a retinal modulating agent to the subject in need thereof.
(Item A48)
   A method for suppressing atrophy, or progress of atrophy, of a retinal thickness in a subject, the method comprising the step of administering an effective amount of a light absorbing substance to the subject in need thereof.
(Item A49)
   A method for suppressing atrophy, or progress of atrophy, of a retinal thickness in a subject, the method comprising the step of administering an effective amount of a neural activity signaling agent or device to the subject in need thereof.
(Item A50)
   A method for protecting a retinal thickness in a subject, the method comprising the step of administering an effective amount of an opsin or a derivative thereof, or a nucleic acid molecule encoding the same, to the subject in need thereof.
(Item A51)
   A method for suppressing atrophy, or progress of atrophy, of a retinal thickness in a subject, the method comprising the step of administering an effective amount of an opsin or a derivative thereof, or a nucleic acid molecule encoding the same, to the subject in need thereof.
(Item A52)
   The method of any one of the above Items, wherein the retinal modulating agent, the light absorbing substance, the neural activity signaling agent or device, or the opsin or derivative thereof modulates a cis/trans ratio of retinal in an organism body of interest.
(Item A53)
   The method of any one of the above Items, wherein the retinal modulating agent, the light absorbing substance, the neural activity signaling agent or device, or the opsin or derivative thereof modulates a retinal concentration in an organism body of interest.
(Item A54)
   The method of any one of the above Items, wherein the protection of the retinal thickness, or the suppression of atrophy, or progress of atrophy, of the retinal thickness, comprises: protection of an inner retinal layer thickness; or suppression of atrophy, or progress of atrophy, of an inner retinal layer thickness.
(Item A55)
   The method of any one of the above Items, characterized by administering the retinal modulating agent, the light absorbing substance, the neural activity signaling agent or device, or the opsin or derivative thereof, to a subject prior to or after onset of a retinal thickness.
(Item A56)
   The method of any one of the above Items, characterized by administering the retinal modulating agent, the light absorbing substance, the neural activity signaling agent or device, or the opsin or derivative thereof, once during a treatment period.
(Item A57)
   The method of any one of the above Items, wherein the retinal modulating agent, the light absorbing substance, the neural activity signaling agent or device, or the opsin or derivative thereof is a rhodopsin that does not release retinal upon photoreception.
(Item A58)
   The method of any one of the above Items, wherein the retinal modulating agent, the light absorbing substance, the neural activity signaling agent or device, or the opsin or derivative thereof is an ion-transporting receptor rhodopsin.
(Item A59)
   The method of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item A60)
   The method of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item A61)
   The method of any one of the above Items, wherein the ion-transporting receptor rhodopsin is derived from cyanobacteria (blue-green bacteria).
(Item A62)
   The method of any one of the above Items, wherein the G protein-coupled receptor rhodopsin is derived from a mammal.
(Item B1)
   Use of a retinal modulating agent for manufacture of a composition for use in treating or preventing a disease, disorder or symptom associated with an endoplasmic reticulum stress.
(Item B2)
   Use of a light absorbing substance for manufacture of a composition for use in treating or preventing a disease, disorder or symptom associated with an endoplasmic reticulum stress.
(Item B3)
   Use of a neural activity signaling agent or device for manufacture of a composition for use in treating or preventing a disease, disorder or symptom associated with an endoplasmic reticulum stress.
(Item B4)
   Use of a light absorbing substance for manufacture of a composition for use in reducing or eliminating an endoplasmic reticulum stress.
(Item B5)
   Use of a neural activity signaling agent or device for manufacture of a composition for use in reducing or eliminating an endoplasmic reticulum stress.
(Item B6)
   Use of an opsin or a derivative thereof, or a nucleic acid molecule encoding the same, for manufacture of a composition for use in treating or preventing a disease, disorder or symptom associated with an endoplasmic reticulum stress.
(Item B7)
   Use of a retinal modulating agent for manufacture of a composition for use in ameliorating a rhodopsin transport disorder.
(Item B8)
   Use of a light absorbing substance for manufacture of a composition for use in ameliorating a rhodopsin transport disorder.
(Item B9)
   Use of a neural activity signaling agent or device for manufacture of a composition for use in ameliorating a rhodopsin transport disorder.
(Item B10)
   The use of any one of the above Items, wherein the retinal modulating agent, the light absorbing substance, the neural activity signaling agent or device, or the opsin or derivative thereof modulates a cis/trans ratio of retinal in an organism body of interest.
(Item B11)
   The use of any one of the above Items, wherein the retinal modulating agent, the light absorbing substance, the neural activity signaling agent or device, or the opsin or derivative thereof modulates a retinal concentration in an organism body of interest.
(Item B12)
   The use of any one of the above Items, wherein the disease, disorder or symptom associated with the endoplasmic reticulum stress includes vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease.
(Item B13)
   The use of any one of the above Items, characterized in that the composition is for treating or preventing a disease, disorder or symptom associated with an endoplasmic reticulum stress, for reducing or eliminating an endoplasmic reticulum stress, or for ameliorating a rhodopsin transport disorder, in the retina.
(Item B14)
   The use of any one of the above Items, characterized in that the composition is administered to a subject prior to or after onset of the disease, disorder or symptom.
(Item B15)
   The use of any one of the above Items, characterized in that the composition is administered once during a treatment period.
(Item B16)
   The use of any one of the above Items, wherein the retinal modulating agent, the light absorbing substance, the neural activity signaling agent or device, or the opsin or derivative thereof is a rhodopsin that does not release retinal upon photoreception.
(Item B17)
   The use of any one of the above Items, wherein the retinal modulating agent, the light absorbing substance, the neural activity signaling agent or device, or the opsin or derivative thereof is an ion-transporting receptor rhodopsin.
(Item B18)
   The use of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item B19)
   The use of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item B20)
   The use of any one of the above Items, wherein the ion-transporting receptor rhodopsin is derived from cyanobacteria (blue-green bacteria).
(Item B21)
   The use of any one of the above Items, wherein the G protein-coupled receptor rhodopsin is derived from a mammal.
(Item B22)
   Use of a retinal modulating agent for manufacture of a composition for use in treating or preventing a disease, disorder or symptom associated with all-trans-retinal.
(Item B23)
   Use of a light absorbing substance for manufacture of a composition for use in treating or preventing a disease, disorder or symptom associated with all-trans-retinal.
(Item B24)
   Use of a neural activity signaling agent or device for manufacture of a composition for use in treating or preventing a disease, disorder or symptom associated with all-trans-retinal.
(Item B25)
   Use of a light absorbing substance for manufacture of a composition for use in reducing or eliminating all-trans-retinal toxicity.
(Item B26)
   Use of a neural activity signaling agent or device for manufacture of a composition for use in reducing or eliminating all-trans-retinal toxicity.
(Item B27)
   Use of an opsin or a derivative thereof, or a nucleic acid molecule encoding the same, for manufacture of a composition for use in treating or preventing a disease, disorder or symptom associated with all-trans-retinal.
(Item B28)
   Use of a retinal modulating agent for manufacture of a composition for use in inhibiting cell death or apoptosis.
(Item B29)
   Use of a light absorbing substance for manufacture of a composition for use in inhibiting cell death or apoptosis.
(Item B30)
   Use of a neural activity signaling agent or device for manufacture of a composition for use in inhibiting cell death or apoptosis.
(Item B31)
   Use of an opsin or a derivative thereof, or a nucleic acid molecule encoding the same, for manufacture of a composition for use in inhibiting cell death or apoptosis.
(Item B32)
   The use of any one of the above Items, wherein the retinal modulating agent, the light absorbing substance, the neural activity signaling agent or device, or the opsin or derivative thereof modulates a cis/trans ratio of retinal in an organism body of interest.
(Item B33)
   The use of any one of the above Items, wherein the retinal modulating agent, the light absorbing substance, the neural activity signaling agent or device, or the opsin or derivative thereof modulates a retinal concentration in an organism body of interest.
(Item B34)
   The use of any one of the above Items, wherein the disease, disorder or symptom associated with all-trans-retinal comprises cell death or apoptosis.
(Item B35)
   The use of any one of the above Items, characterized in that the composition is for treating or preventing a disease, disorder or symptom associated with all-trans-retinal, for reducing or eliminating all-trans-retinal toxicity, or for inhibiting cell death or apoptosis, in the retina.
(Item B36)
   The use of any one of the above Items, characterized in that the composition is administered to a subject prior to or after onset of the disease, disorder or symptom.
(Item B37)
   The use of any one of the above Items, characterized in that the composition is administered once during a treatment period.
(Item B38)
   The use of any one of the above Items, wherein the retinal modulating agent, the light absorbing substance, the neural activity signaling agent or device, or the opsin or derivative thereof is a rhodopsin that does not release retinal upon photoreception.
(Item B39)
   The use of any one of the above Items, wherein the retinal modulating agent, the light absorbing substance, the neural activity signaling agent or device, or the opsin or derivative thereof is an ion-transporting receptor rhodopsin.
(Item B40)
   The use of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item B41)
   The use of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item B42)
   The use of any one of the above Items, wherein the ion-transporting receptor rhodopsin is derived from cyanobacteria (blue-green bacteria).
(Item B43)
   The use of any one of the above Items, wherein the G protein-coupled receptor rhodopsin is derived from a mammal.
(Item B44)
   Use of a retinal modulating agent for manufacture of a composition for use in protecting a retinal thickness.
(Item B45)
   Use of a light absorbing substance for manufacture of a composition for use in protecting a retinal thickness.
(Item B46)
   Use of a neural activity signaling agent or device for manufacture of a composition for use in protecting a retinal thickness.
(Item B47)
   Use of a retinal modulating agent for manufacture of a composition for use in suppressing atrophy, or progress of atrophy, of a retinal thickness.
(Item B48)
   Use of a light absorbing substance for manufacture of a composition for use in suppressing atrophy, or progress of atrophy, of a retinal thickness.
(Item B49)
   Use of a neural activity signaling agent or device for manufacture of a composition for use in suppressing atrophy, or progress of atrophy, of a retinal thickness.
(Item B50)
   Use of an opsin or a derivative thereof, or a nucleic acid molecule encoding the same, for manufacture of a composition for use in protecting a retinal thickness.
(Item B51)
   Use of an opsin or a derivative thereof, or a nucleic acid molecule encoding the same, for manufacture of a composition for use in suppressing atrophy, or progress of atrophy, of a retinal thickness.
(Item B52)
   The use of any one of the above Items, wherein the retinal modulating agent, the light absorbing substance, the neural activity signaling agent or device, or the opsin or derivative thereof modulates a cis/trans ratio of retinal in an organism body of interest.
(Item B53)
   The use of any one of the above Items, wherein the retinal modulating agent, the light absorbing substance, the neural activity signaling agent or device, or the opsin or derivative thereof modulates a retinal concentration in an organism body of interest.
(Item B54)
   The use of any one of the above Items, wherein the protection of the retinal thickness, or the suppression of atrophy, or progress of atrophy, of the retinal thickness, comprises: protection of an inner retinal layer thickness; or suppression of atrophy, or progress of atrophy, of an inner retinal layer thickness.
(Item B55)
   The use of any one of the above Items, characterized in that the composition is administered to a subject prior to or after onset of atrophy of the retinal thickness.
(Item B56)
   The use of any one of the above Items, characterized in that the composition is administered once during a treatment period.
(Item B57)
   The use of any one of the above Items, wherein the retinal modulating agent, the light absorbing substance, the neural activity signaling agent or device, or the opsin or derivative thereof is a rhodopsin that does not release retinal upon photoreception.
(Item B58)
   The use of any one of the above Items, wherein the retinal modulating agent, the light absorbing substance, the neural activity signaling agent or device, or the opsin or derivative thereof is an ion-transporting receptor rhodopsin.
(Item B59)
   The use of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item B60)
   The use of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item B61)
   The use of any one of the above Items, wherein the ion-transporting receptor rhodopsin is derived from cyanobacteria (blue-green bacteria).
(Item B62)
   The use of any one of the above Items, wherein the G protein-coupled receptor rhodopsin is derived from a mammal.
(Item C1)
   A retinal modulating agent for use in treating or preventing a disease, disorder or symptom associated with an endoplasmic reticulum stress.
(Item C2)
   A light absorbing substance for use in treating or preventing a disease, disorder or symptom associated with an endoplasmic reticulum stress.
(Item C3)
   A neural activity signaling agent or device for use in treating or preventing a disease, disorder or symptom associated with an endoplasmic reticulum stress.
(Item C4)
   A light absorbing substance for use in reducing or eliminating an endoplasmic reticulum stress.
(Item C5)
   A neural activity signaling agent or device for use in reducing or eliminating an endoplasmic reticulum stress.
(Item C6)
   An opsin or a derivative thereof, or a nucleic acid molecule encoding the same, for use in treating or preventing a disease, disorder or symptom associated with an endoplasmic reticulum stress.
(Item C7)
   A retinal modulating agent for use in ameliorating a rhodopsin transport disorder.
(Item C8)
   A light absorbing substance for use in ameliorating a rhodopsin transport disorder.
(Item C9)
   A neural activity signaling agent or device for use in ameliorating a rhodopsin transport disorder.
(Item C10)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof modulates a cis/trans ratio of retinal in an organism body of interest.
(Item C11)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof modulates a retinal concentration in an organism body of interest.
(Item C12)
   The retinal modulating agent, light absorbing substance, neural activity signaling agent or device, or opsin or derivative thereof, of any one of the above Items, wherein the disease, disorder or symptom associated with the endoplasmic reticulum stress includes vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease.
(Item C13)
   The retinal modulating agent, light absorbing substance, neural activity signaling agent or device, or opsin or derivative thereof, of any one of the above Items, wherein a composition comprising: the retinal modulating agent; the light absorbing substance; the neural activity signaling agent or device; or the opsin or derivative thereof, is for treating or preventing a disease, disorder or symptom associated with an endoplasmic reticulum stress, for reducing or eliminating an endoplasmic reticulum stress, or for ameliorating a rhodopsin transport disorder, in the retina.
(Item C14)
   The retinal modulating agent, light absorbing substance, neural activity signaling agent or device, or opsin or derivative thereof, of any one of the above Items, characterized in that a composition comprising: the retinal modulating agent; the light absorbing substance; the neural activity signaling agent or device; or the opsin or derivative thereof, is administered to a subject prior to or after onset of the disease, disorder or symptom.
(Item C15)
   The retinal modulating agent, light absorbing substance, neural activity signaling agent or device, or opsin or derivative thereof, of any one of the above Items, characterized in that a composition comprising: the retinal modulating agent; the light absorbing substance; the neural activity signaling agent or device; or the opsin or derivative thereof, is administered once during a treatment period.
(Item C16)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof, is a rhodopsin that does not release retinal upon photoreception.
(Item C17)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof, is an ion-transporting receptor rhodopsin.
(Item C18)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item C19)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item C20)
   The retinal modulating agent, light absorbing substance, neural activity signaling agent or device, or opsin or derivative thereof, of any one of the above Items, wherein the ion-transporting receptor rhodopsin is derived from cyanobacteria (blue-green bacteria).
(Item C21)
   The retinal modulating agent, light absorbing substance, neural activity signaling agent or device, or opsin or derivative thereof, of any one of the above Items, wherein the G protein-coupled receptor rhodopsin is derived from a mammal.
(Item C22)
   A retinal modulating agent for use in treating or preventing a disease, disorder or symptom associated with all-trans-retinal.
(Item C23)
   A light absorbing substance for use in treating or preventing a disease, disorder or symptom associated with all-trans-retinal.
(Item C24)
   A neural activity signaling agent or device for use in treating or preventing a disease, disorder or symptom associated with all-trans-retinal.
(Item C25)
   A light absorbing substance for use in reducing or eliminating all-trans-retinal toxicity.
(Item C26)
   A neural activity signaling agent or device for use in reducing or eliminating all-trans-retinal toxicity.
(Item C27)
   An opsin or a derivative thereof, or a nucleic acid molecule encoding the same, for use in treating or preventing a disease, disorder or symptom associated with all-trans-retinal.
(Item C28)
   A retinal modulating agent for use in inhibiting cell death or apoptosis.
(Item C29)
   A light absorbing substance for use in inhibiting cell death or apoptosis.
(Item C30)
   A neural activity signaling agent or device for use in inhibiting cell death or apoptosis.
(Item C31)
   An opsin or a derivative thereof, or a nucleic acid molecule encoding the same, for use in inhibiting cell death or apoptosis.
(Item C32)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof modulates a cis/trans ratio of retinal in an organism body of interest.
(Item C33)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof modulates a retinal concentration in an organism body of interest.
(Item C34)
   The retinal modulating agent, light absorbing substance, neural activity signaling agent or device, or opsin or derivative thereof, of any one of the above Items, wherein the disease, disorder or symptom associated with all-trans-retinal comprises cell death or apoptosis.
(Item C35)
   The retinal modulating agent, light absorbing substance, neural activity signaling agent or device, or opsin or derivative thereof, of any one of the above Items, wherein a composition comprising: the retinal modulating agent; the light absorbing substance; the neural activity signaling agent or device; or the opsin or derivative thereof, is for treating or preventing a disease, disorder or symptom associated with all-trans-retinal, for reducing or eliminating all-trans-retinal toxicity, or for inhibiting cell death or apoptosis, in the retina.
(Item C36)
   The retinal modulating agent, light absorbing substance, neural activity signaling agent or device, or opsin or derivative thereof, of any one of the above Items, characterized in that a composition comprising: the retinal modulating agent; the light absorbing substance; the neural activity signaling agent or device; or the opsin or derivative thereof, is administered to a subject prior to or after onset of the disease, disorder or symptom.
(Item C37)
   The retinal modulating agent, light absorbing substance, neural activity signaling agent or device, or opsin or derivative thereof, of any one of the above Items, characterized in that a composition comprising: the retinal modulating agent; the light absorbing substance; the neural activity signaling agent or device; or the opsin or derivative thereof, is administered once during a treatment period.
(Item C38)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof, is a rhodopsin that does not release retinal upon photoreception.
(Item C39)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof, is an ion-transporting receptor rhodopsin.
(Item C40)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item C41)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item C42)
   The retinal modulating agent, light absorbing substance, neural activity signaling agent or device, or opsin or derivative thereof, of any one of the above Items, wherein the ion-transporting receptor rhodopsin is derived from cyanobacteria (blue-green bacteria).
(Item C43)
   The retinal modulating agent, light absorbing substance, neural activity signaling agent or device, or opsin or derivative thereof, of any one of the above Items, wherein the G protein-coupled receptor rhodopsin is derived from a mammal.
(Item C44)
   A retinal modulating agent for use in protecting a retinal thickness.
(Item C45)
   A light absorbing substance for use in protecting a retinal thickness.
(Item C46)
   A neural activity signaling agent or device for use in protecting a retinal thickness.
(Item C47)
   A retinal modulating agent for use in suppressing atrophy, or progress of atrophy, of a retinal thickness.
(Item C48)
   A light absorbing substance for use in suppressing atrophy, or progress of atrophy, of a retinal thickness.
(Item C49)
   A neural activity signaling agent or device for use in suppressing atrophy, or progress of atrophy, of a retinal thickness.
(Item C50)
   An opsin or a derivative thereof, or a nucleic acid molecule encoding the same, for use in protecting a retinal thickness.
(Item C51)
   An opsin or a derivative thereof, or a nucleic acid molecule encoding the same, for use in suppressing atrophy, or progress of atrophy, of a retinal thickness.
(Item C52)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof modulates a cis/trans ratio of retinal in an organism body of interest.
(Item C53)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof modulates a retinal concentration in an organism body of interest.
(Item C54)
   The retinal modulating agent, light absorbing substance, neural activity signaling agent or device, or opsin or derivative thereof, of any one of the above Items, wherein the protection of the retinal thickness, or the suppression of atrophy, or progress of atrophy, of the retinal thickness, comprises: protection of an inner retinal layer thickness; or suppression of atrophy, or progress of atrophy, of an inner retinal layer thickness.
(Item C55)
   The retinal modulating agent, light absorbing substance, neural activity signaling agent or device, or opsin or derivative thereof, of any one of the above Items, characterized in that a composition comprising: the retinal modulating agent; the light absorbing substance; the neural activity signaling agent or device; or the opsin or derivative thereof, is administered to a subject prior to or after onset of atrophy of a retinal thickness.
(Item C56)
   The retinal modulating agent, light absorbing substance, neural activity signaling agent or device, or opsin or derivative thereof, of any one of the above Items, characterized in that a composition comprising: the retinal modulating agent; the light absorbing substance; the neural activity signaling agent or device; or the opsin or derivative thereof, is administered once during a treatment period.
(Item C57)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof, is a rhodopsin that does not release retinal upon photoreception.
(Item C58)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof, is an ion-transporting receptor rhodopsin.
(Item C59)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.
(Item C60)
   The retinal modulating agent, light absorbing substance, neural activity signaling agent or device, or opsin or derivative thereof, of any one of the above Items, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.
(Item C61)
   The retinal modulating agent, light absorbing substance, neural activity signaling agent or device, or opsin or derivative thereof, of any one of the above Items, wherein the ion-transporting receptor rhodopsin is derived from cyanobacteria (blue-green bacteria).
(Item C62)
   The retinal modulating agent, light absorbing substance, neural activity signaling agent or device, or opsin or derivative thereof, of any one of the above Items, wherein the G protein-coupled receptor rhodopsin is derived from a mammal.

In the present disclosure, it is intended that the above one or more features may be provided in further combinations, in addition to the explicit combinations. Still further embodiments and advantages of the present disclosure will be appreciated by those skilled in the art upon reading and understanding the following detailed description as necessary.

### [Advantageous Effects of Invention]

The present disclosure has confirmed that: compositions or pharmaceuticals comprising a retinal modulating agent, such as a microbial opsin or a chimeric rhodopsin, and/or a light absorbing substance and/or a neural activity signaling agent or device; opsins; etc., can effectively treat or prevent a disease associated with an endoplasmic reticulum stress, such as a vesicle transport disorder, a disorder, such as cell death due to all-trans-retinal toxicity, or other diseases, and can also protect a retinal thickness or suppress atrophy, or progress of atrophy, of a retinal thickness.

### [Brief Description of Drawings]

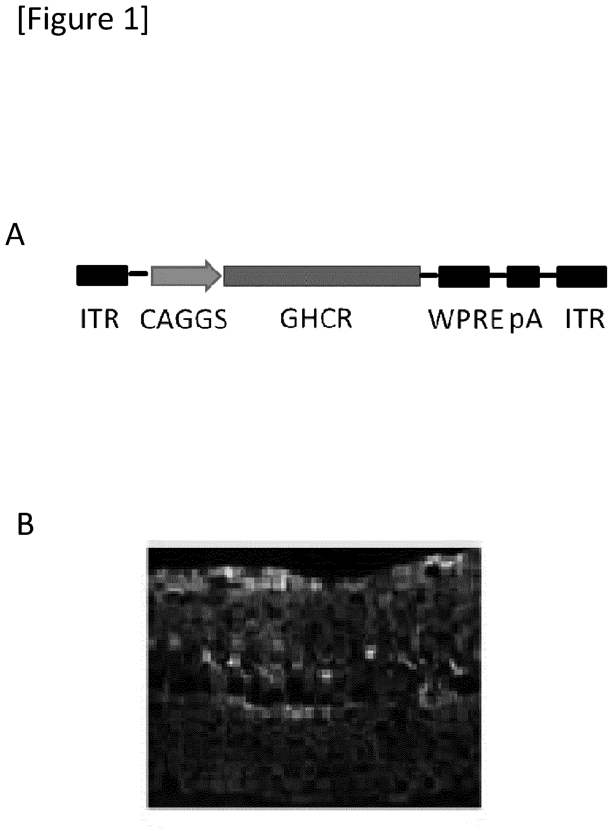

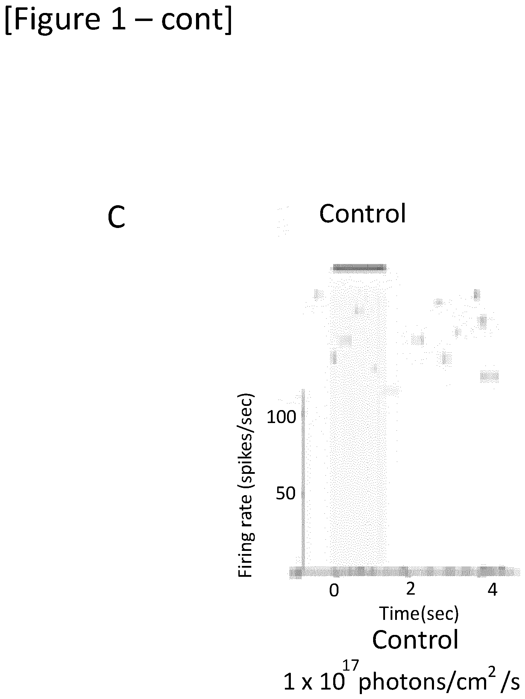

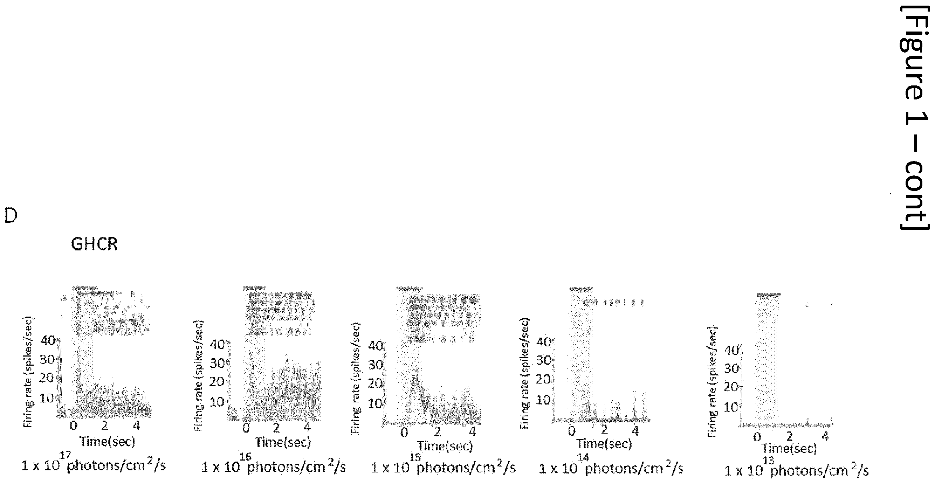

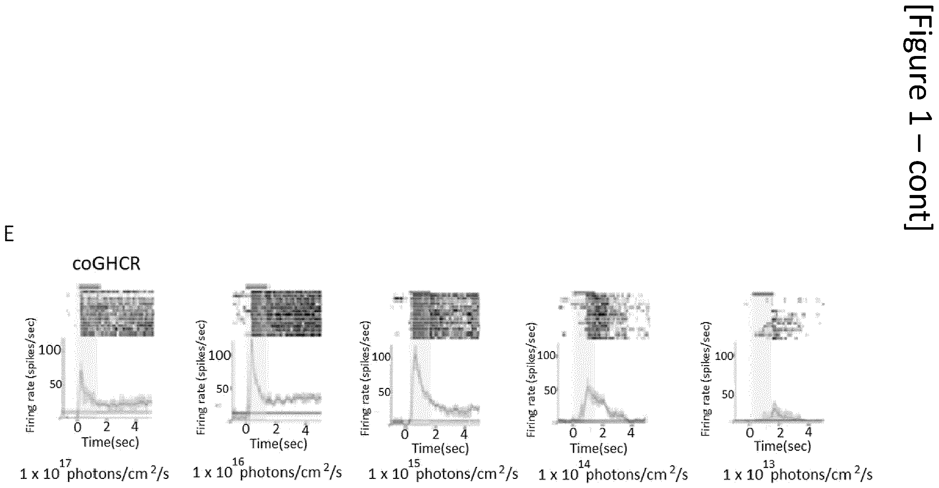

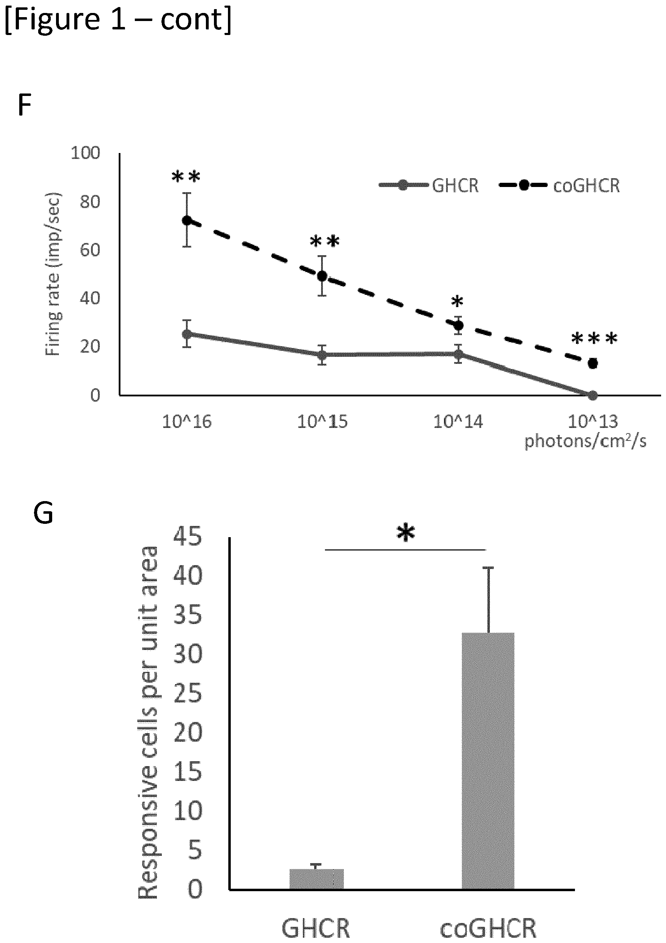

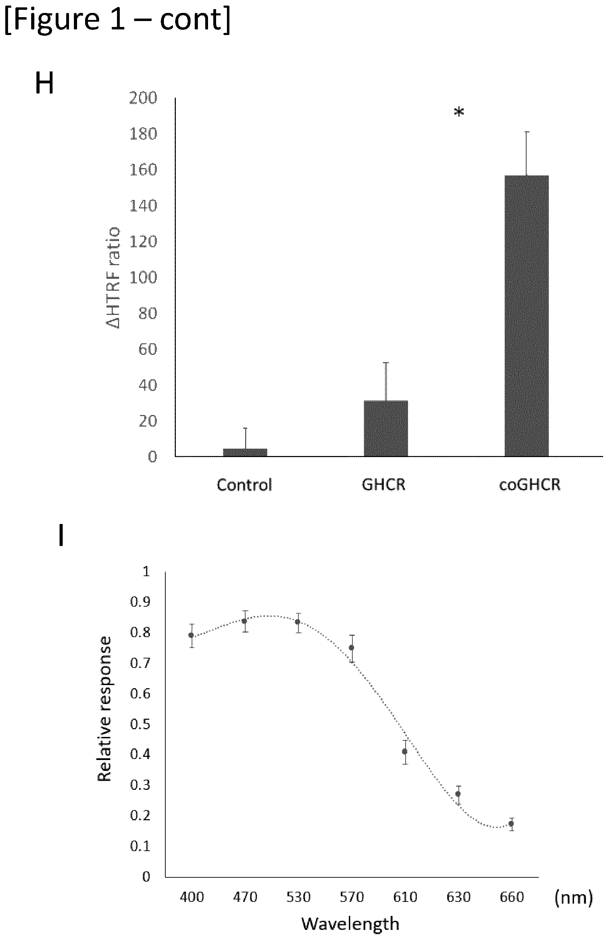

[Figure 1] Figure **1A** is a schematic view of a DNA expression cassette delivered to the retina by an AAV-DJ vector. The GHCR coding sequence is driven by a hybrid CMV enhancer/chicken β-actin (CAGGS) promoter. The sequence is flanked by inverted terminal repeats (ITRs) and stabilized by a polyadenylation signal sequence (pA) and a woodchuck hepatitis posttranscriptional regulatory element (WPRE). Figure **1B** is a cross-sectional confocal image of a C57BL/6 retina two months after AAV-DJ-CAGGS-EGFP intravitreal injection. DAPI nuclear counterstaining is shown in blue. The scale bar is 20 µm. Figures 1C-1E show raster plots of photostimulation and PSTH from control mice (AAV-DJ-CAGGS-EGFP) (C), GHCR-treated mice (AAV-DJ-CAGGS-GHCR) (D), and coGHCR-treated mice (AAV-DJ-CAGGS-coGHCR) (E). The response of a white LED with varying light intensity to 1.0 second illumination was recorded. The gray area around the averaged trace shows the SEM. Figure **1F** shows a quantitative plot of the firing rate of GHCR- and coGHCR-treated retinal ganglion cells at each light intensity. Figure **1G** is a histogram showing the number of light-responsive retinal ganglion cells per unit area (2.6 mm²) of retina in GHCR- and coGHCR-treated mice (n=3 each). Figure **1H** is a histogram showing ΔHTRF detecting changes in cAMP consumption in response to Gi/o-coupled GPCR activation in HKE293T cells transfected with GHCR and coGHCR (n=3 each). Figure **1I** shows the spectral sensitivity of coGHCR. The Error bar indicates SEM. * p<0.05, ** p<0.01, and *** p<0.001. Student's two-tailed t-test.

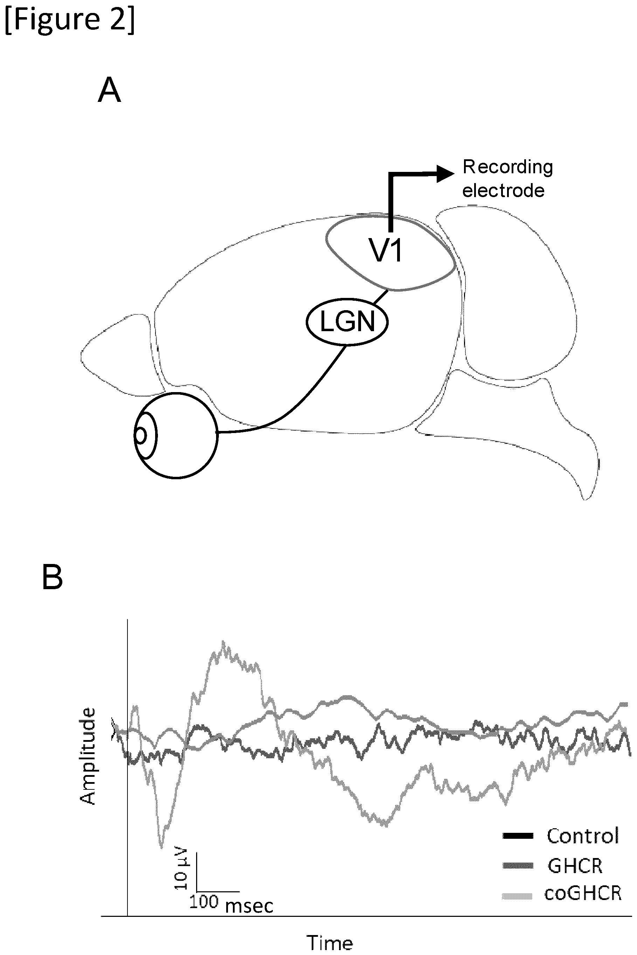

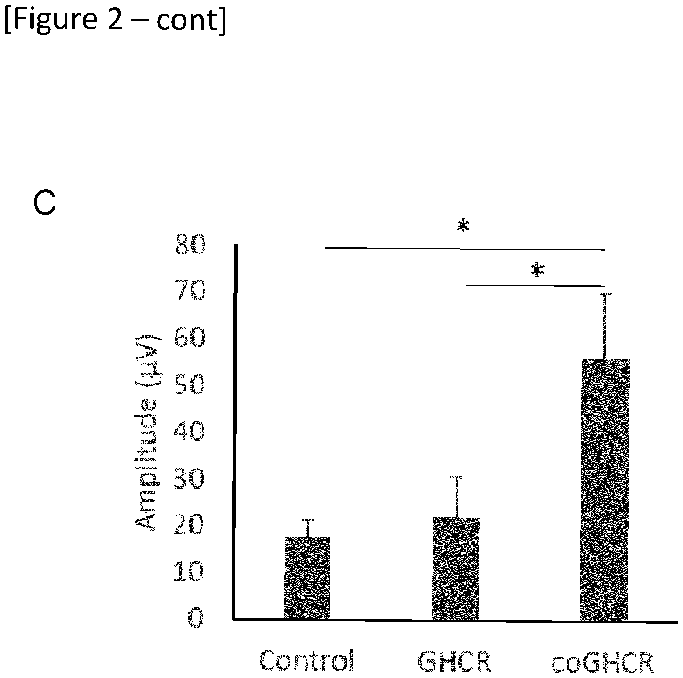

[Figure 2] Figure **2A** is a schematic diagram showing the results of visual evoked potentials (VEP). Figure **2B** shows representative VEP results in GHCR-treated, coGHCR-treated, and control mice. Figure **2C** is a graph showing the average amplitude of VEP in control mice (AAV-DJ-CAGGS-EGFP), GHCR-treated mice (AAV-DJ-CAGGS-GHCR), and coGHCR-treated mice (AAV-DJ-CAGGS-coGHCR). Stimulation was provided with white LED 3cds/m² flash stimulation, the signal was low-pass filtered at 300 Hz, and an average value of 60 times was taken. The error bar indicates SEM. *p<0.05, one-way analysis of variance and Tukey's multiple comparison test.

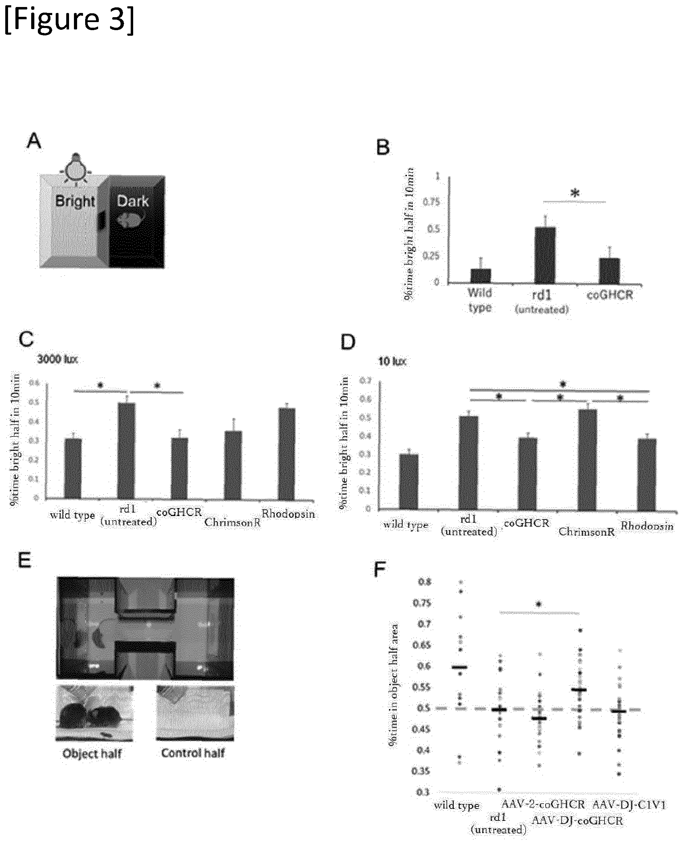

[Figure 3] Figure **3A** is a schematic view of the light-dark chamber transfer test (LDT). The test was conducted with a 30 × 45 × 30 cm box. There is a bright room and a dark room of the same size, adjacent to each other with a 5 × 5 cm opening, and mice can move freely between them. Sighted mice are more anxious in bright light, so they spend less time in a bright room. Figure **3B** is a graph showing percentage of time spent in the bright room of wild type mice (n=4), control mice (AAV-DJ-CAGGS-EGFP) (n=7), and coGHCR-treated mice (AAV-DJ-CAGGS-coGHCR) (n=6) as measured by 10 min LDT at 10 lux illumination. Figures **3C** and **3D** are graphs showing percentage of time spent in the bright room of wild-type mice (n=6), control mice (AAV-6-CAGGS-EGFP) (n=8), coGHCR-treated mice (AAV-6-CAGGS-coGHCR) (n=6), ChrimsonR-treated mice (AAV-6-CAGGS-ChrimsonR) (n=6), and human rhodopsin-treated mice (AAV-6-CAGGS-rhodopsin) (n=6) as measured by 10 min LDT at 10 lux illumination (C) and at 3,000 lux illumination (D), respectively. Figure **3E** is an image of the VRT. One half of the room played a fighting video, the other playing an empty cage video as a control, and the time spent in each area (blue and red) was measured. Figure **3F** is a graph showing the distribution of percentage of time spent in the fighting video area of wild-type mice (n=6), control mice (rd1 mice without treatment), AAV-2-coGHCR-treated mice (AAV-2-CAGGS-coGHCR) (n=6), AAV-DJ-coGHCR-treated mice (AAV-DJ-CAGGS-coGHCR) (n=6), and AAV-DJ-C1V1-treated mice (AAV-DJ-CAGGS-C1V1) (n=6) as measured by LDT at 10 lux (C) illumination and at 3,000 lux (D) illumination. The black lines indicate mean values. * p<0.05, ** p<0.01, and *** p<0.001. One-way analysis of variance and Tukey's multiple comparison test.

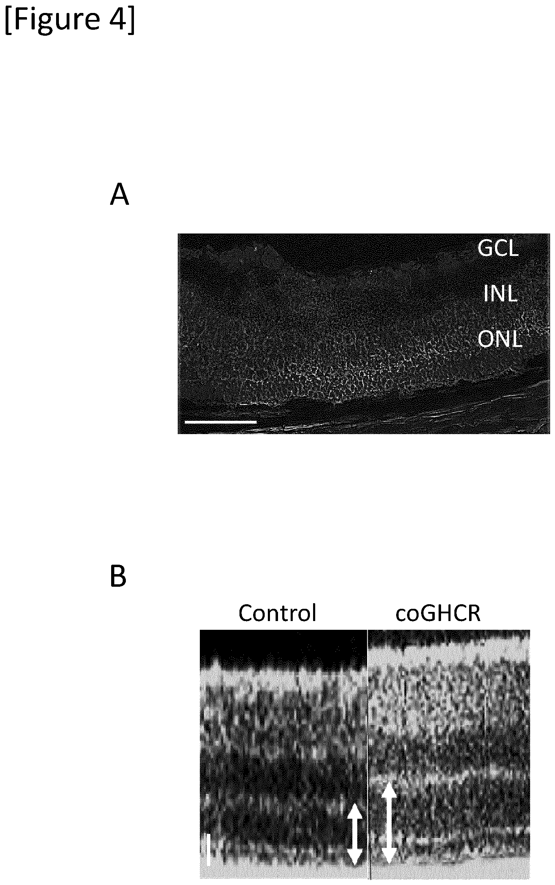

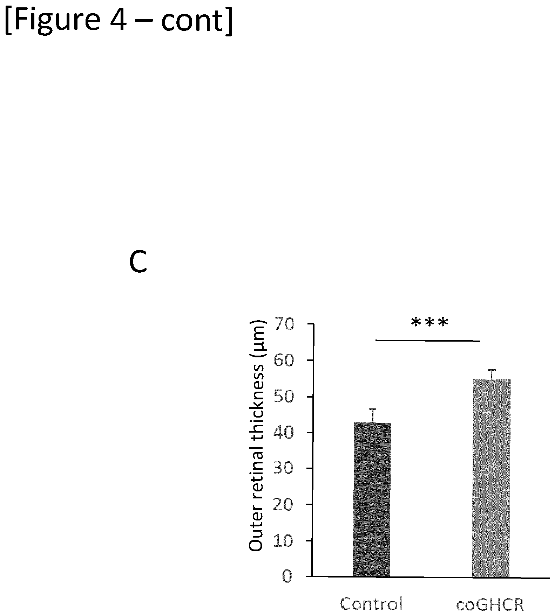

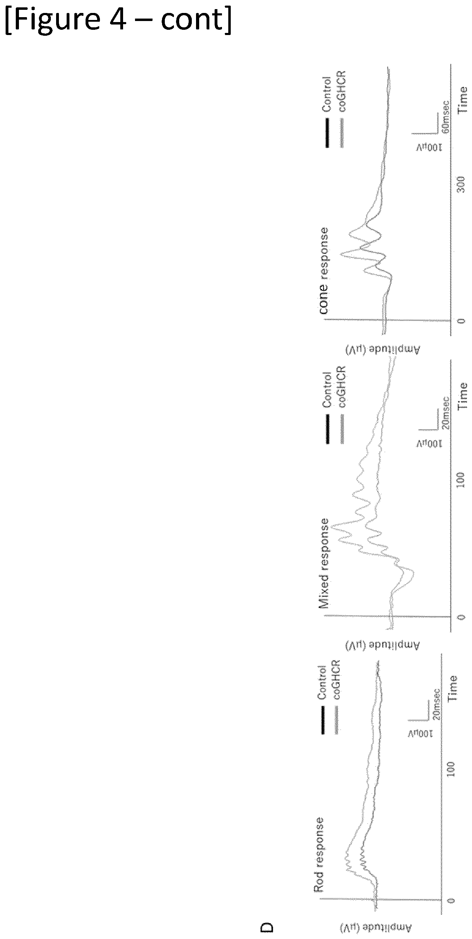

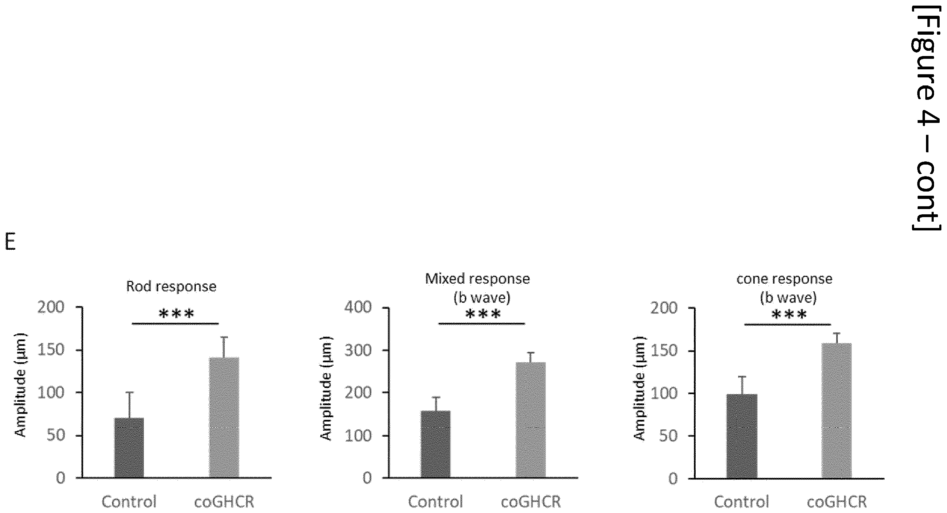

[Figure 4] Figure **4A** is a cross-sectional confocal image of P23H retina two months after AAV-DJ-CAGGS-coGHCR subretinal injection. The FLAG tag fused to the C-terminus of coGHCR is shown in green. DAPI nuclear counterstain is shown in blue. The scale bar is 100 µm. Figure **4B** shows cross-sectional images of OCT retinas at PND30 of coGHCR-treated and control mice (AAV-DJ-CAGGS-EGFP subretinal injection). The white arrows indicate the measured outer retinal thickness (ONL to COS). The scale bar is 20 µm. Figure **4C** shows a histogram of the measured outer retinal thickness of coGHCR-treated and control mice at PND30. Figures **4D** and **4E** show: representative ERG waveforms (rod response, mixed response, cone response) of coGHCR-treated mice and control mice at PND30 (D); and histograms of the ERG amplitudes of the rod response, mixed b-wave, and cone b-wave (E). The error bar indicates SEM. *** p<0.001. Unpaired t-test.

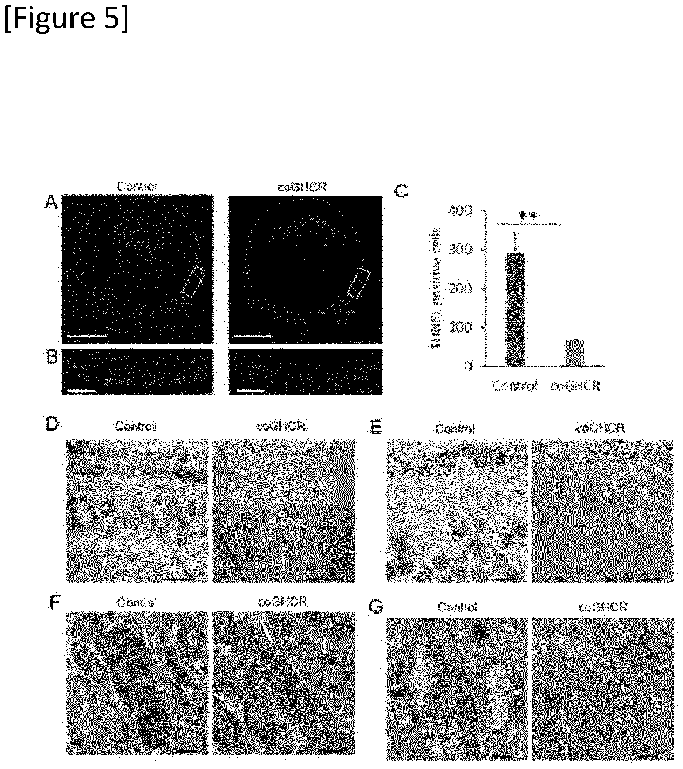

[Figure 5] Figures **5A** and **5B** show TNDEL-stained cross sections of coGHCR-treated mice and control mice (AAV-DJ-CAGGS-EGFP subretinal injection) at PND31 (A) and magnified image of the white rectangular portions (B). TUNEL-positive cells fluoresce in red. DAPI nuclear counterstaining is shown in blue. The scale bar is 1,000 um in Figure **5A** and 100 um in Figure **5B.** Figure **5C** is a histogram of TUNEL-positive cells from coGHCR-treated and control mice at PND31. The error bar indicates SEM. ** p<0.01. Unpaired t-test. Figures **5D** to **5G** are TEM images of cross-sections of coGHCR-treated and control mice at PND31. The outer retinal layer (D), the outer segment at low magnification (E), the outer segment at high magnification (F), and the inner segment (G) are shown, respectively. The arrows indicate swelling of the endoplasmic reticulum. The scale bars are 20 um in Figure 5D, 5 um in Figure 5E, 1 um in Figure 5F and 500 nm in Figure 5G.

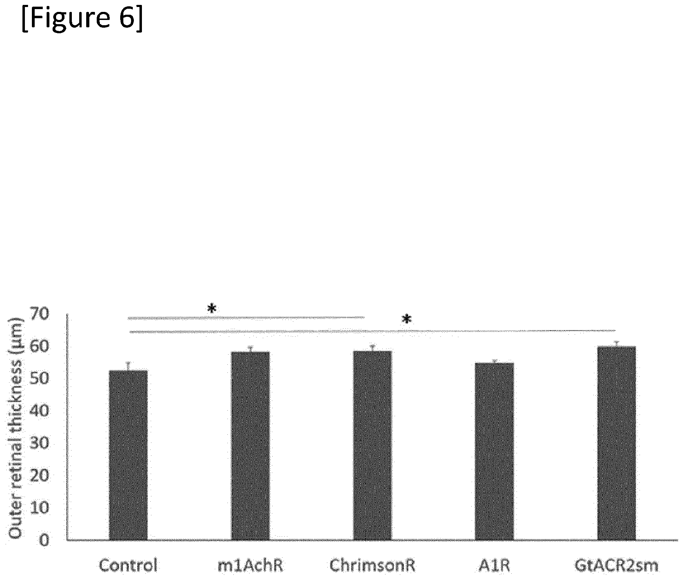

[Figure 6] Figure **6** is a graph showing outer retinal thickness at PND30 in AAV DJ-CAGGS-GRsm/m1AchR-3rd-Venus-injected mice, AAV DJ-CAGGS-GRsm/A1R-3rd-Venus injected mice, AAV DJ-CAGGS-EGFP-GtACR2sm/BvRh-double injected mice, or AAV 6-CAGGS-ChrimsonR-tdT injected mice.

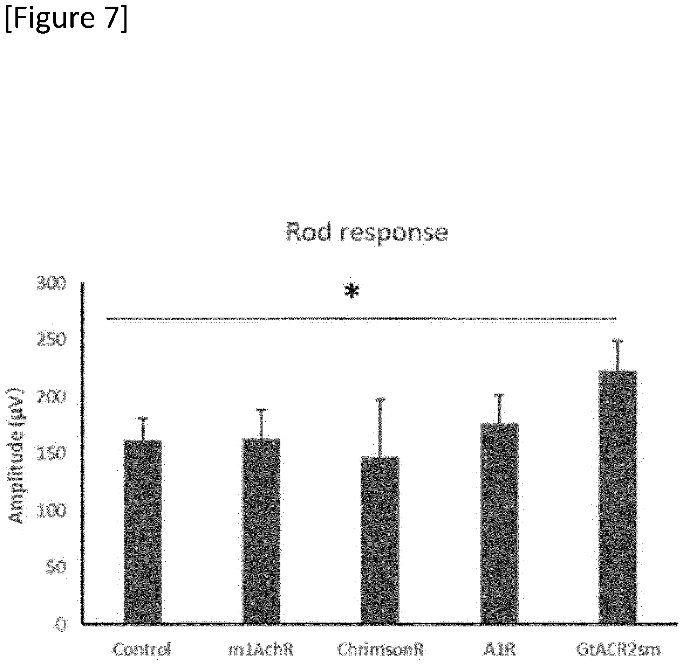

[Figure 7] Figure **7** is a graph showing electroretinogram amplitudes at PND30 in rod responses in AAV DJ-CAGGS-GRsm/m1AchR-3rd-Venus injected mice, AAV DJ-CAGGS-GRsm/A1R-3rd-Venus injected mice, AAV DJ-CAGGS-EGFP-GtACR2sm/BvRh-double injected mice, or AAV 6-CAGGS-ChrimsonR-tdT injected mice.

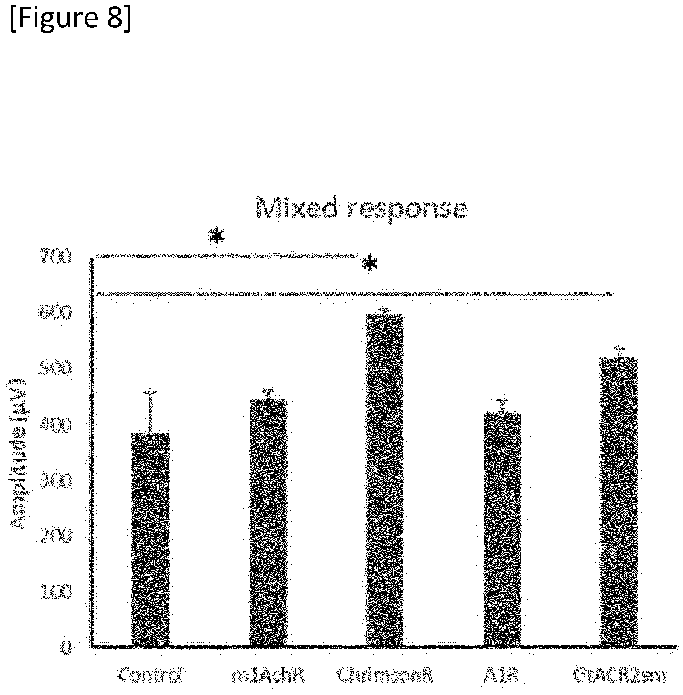

[Figure 8] Figure **8** is a graph showing electroretinogram amplitudes at PND30 in mixed responses in AAV DJ-CAGGS-GRsm/m1AchR-3rd-Venus injected mice, AAV DJ-CAGGS-GRsm/A1R-3rd-Venus injection mice, AAV DJ-CAGGS-EGFP-GtACR2sm/BvRh-double injected mice, or AAV 6-CAGGS-ChrimsonR-tdT injected mice.

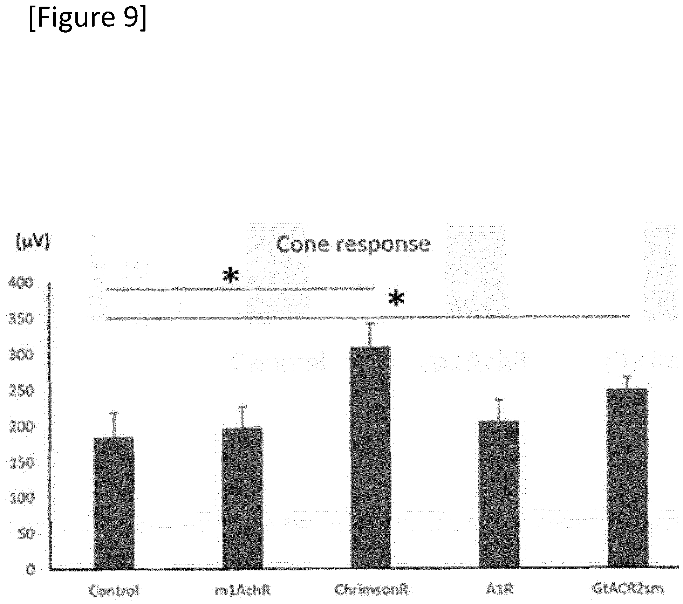

[Figure 9] Figure **9** is a graph showing electroretinogram amplitudes at PND30 in cone responses in AAV DJ-CAGGS-GRsm/m1AchR-3rd-Venus injected mice, AAV DJ-CAGGS-GRsm/A1R-3rd-Venus injection mice, AAV DJ-CAGGS-EGFP-GtACR2sm/BvRh-double injected mice, or AAV 6-CAGGS-ChrimsonR-tdT injected mice.

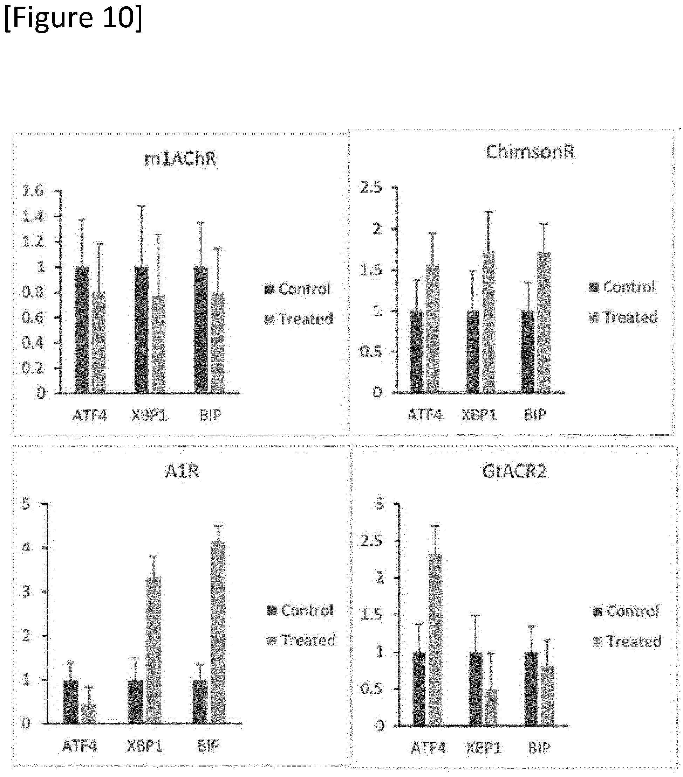

[Figure 10] Figure **10** is a graph showing the results of Western blotting of ATF4, XBP1 and BIP at PND30 in AAV DJ-CAGGS-GRsm/m1AchR-3rd-Venus injected mice, AAV DJ-CAGGS-GRsm/A1R-3rd-Venus injected mice, AAV DJ-CAGGS-EGFP-GtACR2sm/BvRh-double injected mice, or AAV 6-CAGGS-ChrimsonR-tdT injected mice.

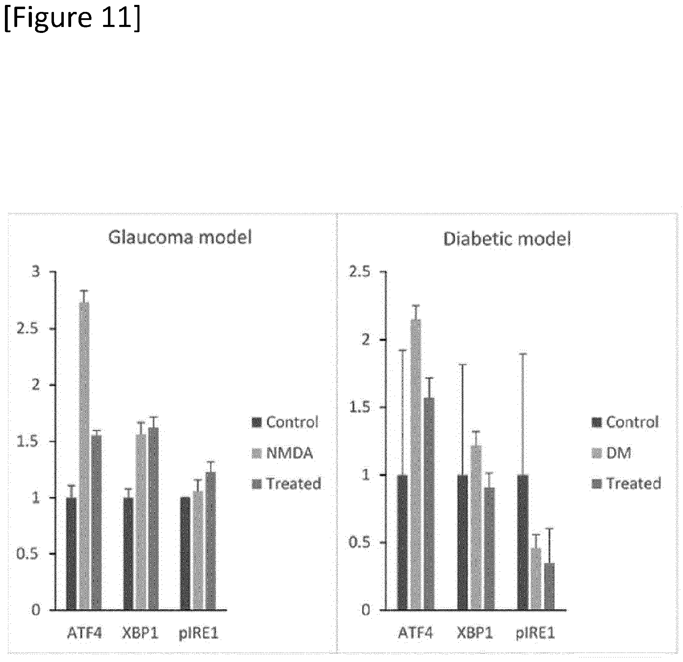

[Figure 11] Figure **11** is a graph showing the results of Western blotting on the retina of glaucoma and diabetes-induced model mice after transfection of GHCR into the retina.

### [Description of Embodiments]

Hereinafter, the present disclosure will be described while showing the best mode. Throughout the present specification, it should be understood that the representation of a singular form also includes the concept of a plural form thereof, unless otherwise stated. It should thus be understood that singular articles (e.g., "a", "an", "the", etc. in the English language) also include the concept of a plural form thereof, unless otherwise stated. It should also be understood that the terms used herein are used in the meaning commonly used in the art, unless otherwise stated. Thus, unless otherwise defined, all technical terms and scientific terms used herein have the same meaning as commonly understood by those skilled in the art to which the present disclosure pertains. In case of conflict, the present specification (including definitions) takes precedence.

### (Definitions etc.)

The definitions and/or basic technical contents of terms particularly used in the present specification will be described below as appropriate.

As used herein, "opsin" or "opsins" refers to a protein that binds to retinal, a pigment, to form rhodopsin, a visual substance. The type of opsin varies depending on the species of organism and the type of visual cell, and examples thereof can include rod opsin (rhodopsin) and cone opsin (e.g., blue opsin, green opsin, red opsin). In addition, the opsins, as used herein, include, but are not limited to, melanopsin, encephalopsin, panopsin, and peropsin. For example, animal opsins are G protein-coupled receptors (GPCRs) with a seven-time transmembrane structure, and form rhodopsins that exhibit photosensitivity when retinal, a pigment, binds to them. Opsin activates a trimeric G-protein upon photoreception, thereby transmitting external light signals into cells. As used herein, "opsin" and "rhodopsin" described below can be used interchangeably depending on the context. Microbial opsins are often called rhodopsins because they do not release the chromophore retinal upon photoreception. On the other hand, among the animal opsins, rhodopsin is narrowly defined as the animal-type rod visual substance. For distinction, those other than animal-type rod visual substances are often referred to as opsins, but those skilled in the art can understand what is meant according to the context.

Opsins (rhodopsins) can be broadly classified into microbial opsins and animal opsins, and animal opsins can be further classified into vertebrate visual opsins, vertebrate non-visual opsins, and invertebrate opsins. Opsins (rhodopsins) can also be classified into Type I opsins and Type II opsins. Vertebrate non-visual opsins and invertebrate opsins are called bistable opsins, and include opsins that use all-trans-retinal as a chromophore, like microbial opsins. The microbial opsins as well as some of the vertebrate non-visual opsins and invertebrate opsins are opsins that do not release retinal upon photoreception (rhodopsins) and can be used as opsins of the present disclosure. Opsins (rhodopsins) that can be advantageously used in the present disclosure are opsins (rhodopsins) that do not release retinal upon photoreception, including, for example, microbial opsins as well as some of vertebrate non-visual opsins and invertebrate opsins. Bistable opsins, including insect opsins, can also be included therein. Even if the opsins used in the present disclosure do not strictly correspond to microbial opsins, vertebrate non-visual opsins, or invertebrate opsins, such opsins can be advantageously used as long as they are functional equivalents having equivalent functions to these types of opsins that are advantageously used.

As used herein, "rhodopsin" is a protein having a pigment called retinal inside, which is activated by receiving light and transmits visual signals to the brain. Ion-transporting receptor rhodopsin, typified by those derived from microorganisms, can be repeatedly activated by absorbing light because retinal is not released by light irradiation; however, the ion-transporting receptor rhodopsin is unable to activate G protein, unlike G protein-coupled receptor rhodopsin typified by those derived from animals. The chimeric rhodopsin with an ion-transporting receptor rhodopsin and a G protein-coupled receptor rhodopsin, as preferably utilized in the present disclosure, is thought to have enhanced functions compared to the conventional rhodopsin. In particular, the ion-transporting receptor rhodopsin can preferably be of microbial origin, and those that can be repeatedly used are utilized. Furthermore, when the G protein-coupled receptor rhodopsin of animal origin, preferably of mammalian origin, is utilized, high activity via an endogenous G protein can be obtained while the function of repeated activation is retained. Without wishing to be bound by theory, the chimeric protein utilized in the present disclosure is expressed in mammals, such as rodents and primates, while retaining sufficient activity, as demonstrated by the animal models; thus, the chimeric protein is capable of achieving preventive and progress-suppressing effects for diseases, disorders or symptoms of the retina, and in particular, the prevention or suppression of progress of retinitis pigmentosa, or providing improvement in visual cognitive behavioral functions (e.g., improvement in light-dark determination functions, improvement in bright spot evading functions, and/or crisis avoidance functions), or exerting effects for augmenting visual functions, such as improvement in visual acuity.

As used herein, an "ion-transporting receptor rhodopsin" refers to any rhodopsin having a function of transporting ions, and examples thereof can include an ion pumping receptor rhodopsin and an ion channeling receptor rhodopsin.

With regard to the ion-transporting receptor rhodopsin, the conformational compatibility and the membrane transfer efficiency with the G protein activation loop are considered to be important. In particular, the ion-transporting receptor rhodopsins of algal or microbial origin have good conformational compatibility and membrane transfer efficiency with the G protein activation loop, and among them, those pertaining to the genus Gloeobacter or genus Guillardia are preferable. In particular, Gloeobacter violaceus, among the microorganisms pertaining to the genus Gloeobacter, and Guillardia theta of the genus Guillardia are preferable. It is also preferable to combine and utilize the rhodopsin (e.g., SEQ ID NO: 13 or 14) of microorganisms pertaining to the genus Gloeobacter, or the rhodopsin (e.g., SEQ ID NO: 15 or 16) of microorganisms pertaining to the genus Guillardia, with a G protein-coupled receptor rhodopsin of mammalian origin, and preferably a G protein-coupled receptor rhodopsin of Artiodactyla, such as cow (e.g., SEQ ID NO: 11 or 12), or primates such as humans (e.g., SEQ ID NO: 9 or 10), among the G protein-coupled receptor rhodopsins of animal origin. The genus Gloeobacter, as well as the algae of the genus Guillardia etc., are also preferable in terms of having an important property of being expressed well in E. coli, which are eubacteria, and human cells, which are eukaryotes.

As used herein, an "ion pumping receptor rhodopsin" refers to any pumping rhodopsin having a function of transporting ions. When such a rhodopsin is sensitive to light, it functions by actively transporting ions, such as hydrogen ions, chloride ions or sodium ions, into cells.

As used herein, an "ion channeling receptor rhodopsin" refers to any channeling rhodopsin having a function of transporting ions. When such a rhodopsin is sensitive to light, it functions by allowing ions, such as hydrogen ions, chloride ions or sodium ions, to flow into cells.

As used herein, a "G protein-coupled receptor rhodopsin" refers to a rhodopsin classified as a G protein-coupled receptor, which is a type of receptor existing on the cytoplasmic membrane of eukaryotic cells or on the constituent membrane inside the cell. The G protein-coupled receptor is said to have seven α-helix structures that penetrate the cytoplasmic membrane, with the N-terminal side being extracellular and the C-terminal side being intracellular, and three extracellular loops (ECL1/2/3) and three intracellular loops (ICL1/2/3). The rhodopsin is composed of apoprotein and chromophore retinal, and retinal absorbs light to isomerize and cause structural changes in the protein part, driving the intracellular signal transduction system via the G protein.

As used herein, "retinal" is also called retinaldehyde or retinene, and refers to a substance that constitutes rhodopsin, a visual substance contained in the rods of the retina. The retinal is a kind of vitamin A1 and is reduced to retinol when the retina is exposed to light. Retinal, which binds to opsin to form rhodopsin, has a molecular form of 11-cis-retinal, which is isomerized to all-trans-retinal when exposed to light, and is released from the bond with opsin. All-trans-retinal then reverts to 11-cis-retinal in the visual cycle and binds to opsin. All-trans-retinal is phototoxic and has been implicated in various retinal diseases, including age-related macular degeneration, Stargardt's disease, fundus flavum, and recessive retinitis pigmentosa.

As used herein, a "retinal modulating agent" refers to an agent that modulates the state of retinal, including the in vivo amount, concentration, or cis/trans ratio (ratio of 11-cis-retinal to all-trans-retinal) of retinal as described above. For example, a retinal modulating agent includes vitamin A, and since vitamin A can be a material for retinal, vitamin A can modulate the amount of retinal in the retina. In addition, such retinal modulating agents can include 9-cis-retinal, 9-cis-retinyl-acetate, and 9-cis-β-carotene. These substances bind to opsin to become rhodopsin and receive light, but are metabolized to isorhodopsin instead of all-trans-retinal. Since this isorhodopsin does not enter the regeneration system of the visual cycle, the amount of all-trans-retinal is reduced and the burden on the visual cycle is reduced. This is thought to be particularly effective in retinitis pigmentosa with mutations in the visual cycle-related genes (ABCA4, LRAT, and RPE65). The retinal modulating agents can also include retinylamine and emixustat. These are substances that act as visual cycle modulators by acting on a metabolic circuit called the visual cycle in which all-trans-retinal photoisomerized from 11-cis-retinal to all-trans-retinal is returned to 11-cis-retinal again. By modulating the speed of the circuit, they suppress the accumulation of the toxic substance all-trans-retinal. The retinal modulating agents further include: RBP4-TTRcomplex, which is a vitamin A remover; and Primary Amines, which is an all-trans retinal remover. As the retinal modulating agents, it is also possible to use: microbial opsins that do not release retinal upon photoreception; bistable opsins that are vertebrate non-visual opsins and invertebrate opsins; and chimeric rhodopsins of ion-transporting receptor rhodopsin and G protein-coupled receptor rhodopsin. The retinal modulating agents further include Zuretinolacetate (9-cis-retinol), which functions as a substitute for 11-cis-retinal, thereby substantially functioning as a modulating agent. The modulating agent may be provided in the form of a protein, may be provided as a nucleic acid molecule (genetic medicine) encoding the protein, or may be provided as a substance other than these.

As used herein, a "relative retinal modulating agent" refers to an agent that directly or indirectly acts on retinal to relatively modulate cis-retinal and trans-retinal. For example, vitamin A can be a material for retinal, and thus, it can be said to be a relative retinal modulating agent in that vitamin A modulates the amount of retinal itself. Furthermore, 9-cis-retinal, 9-cis-retinyl-acetate, and 9-cis-β-carotene couple to opsin to form rhodopsin, which is metabolized to isorhodopsin rather than all-trans-retinal upon exposure to light. This thus can reduce the amount of all-trans-retinal. That is, these substances can be considered as relative retinal modulating agents in that they modulate the amount of all-trans-retinal. In addition, retinylamine and emixustat modulate the amount, concentration, or cis/trans ratio of retinal by modulating the speed of the visual cycle. They thus can be considered to act on retinal, and therefore, they can be considered as relative retinal modulating agent. Furthermore, RBP4-TTRcomplex, a vitamin A remover, and Primary Amines, an all-trans-retinal remover, modulate the amount, concentration, or cis/trans ratio of retinal through the removal of vitamin A and all-trans-retinal. These substances can thus be considered to act on retinal, and therefore, they can be considered as relative retinal modulating agent. Furthermore, Zuretinolacetate (9-cis-retinol) can be considered to act on retinal in that it functions as a substitute for 11-cis-retinal, and therefore, it can be considered as a relative retinal modulating agent. As used herein, a "relative retinal modulating agent" may also be referred to as a "metabolic retinal modulating agent" from the viewpoint of its action on the metabolic circuit of retinal in the visual cycle. The relative retinal modulating agent may be provided in the form of a protein, may be provided as a nucleic acid molecule (genetic medicine) encoding the protein, or may be provided as a substance other than these.

As used herein, an "absolute retinal modulating agent" refers to such an agent that acts on rhodopsin itself, not on retinal, to prevent the production of toxic metabolites such as all-trans-retinal, thereby absolutely modulating the amount, concentration, or cis/trans ratio of retinal. That is, in the first place, the absolute retinal modulating agent can modulate the amount, concentration, or cis/trans ratio of retinal without producing all-trans-retinal, unlike the natural rhodopsin, in which 11-cis-retinal released by photoreception undergoes photoisomerization to all-trans-retinal, and the all-trans-retinal is converted back to 11-cis-retinal for use as a cofactor. For example, the chimeric rhodopsin of the present disclosure can be considered to be an absolute retinal modulating agent in that it becomes a rhodopsin that does not produce all-trans-retinal by the gene introduction thereof into the opsin that constitutes rhodopsin. In addition, it is also possible to use: microbial opsins that do not release retinal upon photoreception; and bistable opsins that are vertebrate non-visual opsins and invertebrate opsins, as absolute retinal modulating agents. As used herein, the "absolute retinal modulating agent" may also be referred to as a "photocompensatory retinal modulating agent" or a "photoreceptive retinal modulating agent", from the viewpoint that the absolute retinal modulating agent is activated by absorbing light even if it receives light. The absolute retinal modulating agent may be provided in the form of a protein, may be provided as a nucleic acid molecule (genetic medicine) encoding the protein, or may be provided as a substance other than these.

As used herein, a "light absorbing substance" refers to a substance that receives light in place of endogenous rhodopsin. By taking over the photoreception by endogenous rhodopsin, the light absorbing substance can, for example, reduce the amount of all-trans-retinal that is originally produced due to the photoreception by rhodopsin. In addition, the light absorbing substance can also include an autologous light absorbing substance. In addition, the light absorbing substance is preferably a non-intrinsic light absorbing substance. For example, the light absorbing substance can include: microbial opsins; bistable opsins that are vertebrate non-visual opsins and invertebrate opsins; and chimeric rhodopsins of ion-transporting receptor rhodopsin and G protein-coupled receptor rhodopsin. The light absorbing substance used in the present disclosure is preferably, but not limited to, a biocompatible light absorbing substance. The light absorbing substance used in the present disclosure may be, but is not limited to, a light absorbing substance that has neural activity signaling activity. The light absorbing substance may be provided in the form of a protein, may be provided as a nucleic acid molecule (genetic medicine) encoding the protein, or may be provided as a substance other than these.

In order to determine whether a certain substance is a light absorbing substance for use in the present disclosure, the substance can be measured using a spectrophotometer and determined by the absorbance thereof. For example, in order to determine whether a substance is a light absorbing substance, advantageous is such a substance that absorbs light with a wavelength at any point in the band from about 300 to about 800 nm, preferably at any point in the band from about 400 to about 600 nm, and more preferably at about 500 nm.

As used herein, a "biocompatible light absorbing substance" refers to a light absorbing substance having biocompatibility among light absorbing substances that are photoreceptive substances instead of endogenous rhodopsin. Such light absorbing substances having biocompatibility can include, for example, any biological substances (such as nucleic acids, proteins, peptides, lipids, carbohydrates, and complexes thereof). In addition, the biocompatible light absorbing substance can also include an autologous biocompatible light absorbing substance. In addition, the biocompatible light absorbing substance is preferably a non-intrinsic biocompatible light absorbing substance. As for the non-intrinsic biocompatible light absorbing substance, it may be advantageous, but not limited, to use a non-antigenic, light absorbing substance. For example, the biological light absorbing substance can include: microbial opsins; bistable opsins that are vertebrate non-visual opsins and invertebrate opsins; and chimeric rhodopsins of ion-transporting receptor rhodopsin and G protein-coupled receptor rhodopsin.

As used herein, a "neural activity signaling agent or device" refers to an agent, substance, or device capable of transmitting a neural activity signal, including electrical stimulation, to nerves on the side closer to the center. Originally, visual cells sense light through the visual transmission system and transmit visual signals to the central nervous system via neurons. However, if visual cells or retinal pigment epithelial cells are degenerated or lost, such as in the case of ablepsia, the neural activity signaling agent or device can transmit neural activity signals to the center. The neural activity signaling agent or device can include those that are autologous. In addition, the neural activity signaling agent or device is preferably a non-intrinsic neural activity signaling agent or device. For example, the neural activity signaling agent or device can include: electrical signal-imparting devices, substances that generate electrical signals, electrical signal generating chips, and other devices; microbial opsins; bistable opsins that are vertebrate non-visual opsins and invertebrate opsins; and chimeric rhodopsins of ion-transporting receptor rhodopsin and G protein-coupled receptor rhodopsin. Further, as used herein, the "neural activity signaling agent or device" can also include cell therapy, and the cell therapy can include: visual cell transplantation (e.g., those derived from iPS cells); and stem cell transplantation. The neural activity signaling agent or device may be provided in the form of a protein, may be provided as a nucleic acid molecule (genetic medicine) encoding the protein, or may be provided as a substance other than these.

The neural activity signaling agent or device used in the present disclosure is preferably, but not limited to, a biocompatible neural activity signaling agent or device. The neural activity signaling agent or device may be, but is not limited to, a neural activity signaling agent or device having a light absorption property.

In order to determine whether a certain substance is a neural activity signaling agent or device for use in the present disclosure, an electrophysiological technique typified by the patch clamp method, an intracellular imaging technique using a fluorescent dye, or the like can be used to measure electrical signals, and the determination can be made based on the result. Advantageously, to determine whether a substance is a neural activity signaling agent or device, it is preferable to be able to measure neural action potentials generated in response to a light stimulation or chemical stimulation or electrical stimulation or mechanical stimulation, and it is more preferable to be able to measure neural action potentials generated in response to a light stimulation. However, the present invention is not limited to this. For example, a response to a light stimulation can occur within about 500 milliseconds, but is not so limited.

As used herein, a "biocompatible neural activity signaling agent or device" refers to those that have biocompatibility among the neural activity signaling agents or devices that are agents, substances or devices capable of transmitting neural activity signals, including electrical stimulation, to nerves on the side closer to the center. Those that have biocompatibility include, for example, devices constituted of any biological substances (such as nucleic acids, proteins, peptides, lipids, carbohydrates, and complexes thereof) or biocompatible substances. Originally, visual cells sense light through the visual transmission system and transmit visual signals to the central nervous system via neurons. However, if visual cells or retinal pigment epithelial cells are degenerated or lost, such as in the case of ablepsia, the biocompatible neural activity signaling agent or device can transmit neural activity signals to the center while being biocompatible. The biocompatible neural activity signaling agent or device can include those that are autologous. In addition, the neural activity signaling agent or device is preferably a non-intrinsic and biocompatible neural activity signaling agent or device. It may be advantageous that the non-intrinsic and biocompatible neural activity signaling agent or device is a non-antigenic one. For example, the biocompatible neural activity signaling agent or device can include: electrical signal-imparting devices, at least the body-contacting surface of which is composed of a biocompatible substance; substances that generate electrical signals, composed of biocompatible substance; electrical signal-generating chips, or other devices, composed of biocompatible substance; microbial opsins; bistable opsins that are vertebrate non-visual opsins and invertebrate opsins; and chimeric rhodopsins of ion-transporting receptor rhodopsin and G protein-coupled receptor rhodopsin. In addition, as used herein, the "neural activity signaling agent or device" can also include cell therapy, and the cell therapy can include: visual cell transplantation (e.g., those derived from iPS cells); and stem cell transplantation.

As used herein, an "endoplasmic reticulum stress" refers to a state in which proteins are no longer folded normally in the lumen of the endoplasmic reticulum and accumulate as defective proteins when cells are exposed to various internal or external environmental changes. When endoplasmic reticulum stress occurs *in vivo,* cells perform UPR (unfolded protein response) to improve the state and eliminate defective proteins that are not folded normally. However, when the endoplasmic reticulum stress occurs excessively or persistently, even the UPR cannot cope with it, causing the cells to undergo apoptosis. It has been suggested that this apoptosis results in cell shedding or tissue dysfunction, leading to the development of various diseases.

As used herein, a "disease, disorder or symptom associated with an endoplasmic reticulum stress" refers to a disease, disorder or symptom that is directly or indirectly associated with, or caused by, the endoplasmic reticulum stress as described above. For example, the disease, disorder or symptom associated with the endoplasmic reticulum stress includes, but not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

As used herein, a "disease, disorder or symptom associated with all-trans-retinal" refers to a disease, disorder or symptom associated with the cytotoxicity of all-trans-retinal or the toxicity due to its reactants such as A2E. For example, included are those that develop due to apoptosis of visual cells due to the cytotoxicity of all-trans-retinal or the toxicity due to its reactants such as A2E. In addition, the disease, disorder or symptom associated with all-trans-retinal includes, but is not limited to, glaucoma and various retinal diseases including age-related macular degeneration, Stargardt's disease, fundus flavum, and recessive retinitis pigmentosa. In fact, any disease, disorder or symptom that is directly or indirectly associated with all-trans-retinal toxicity is included in the disease, disorder or symptom.

As used herein, a "retinal disease" refers to any disease, disorder or symptom related to the retina. The examples thereof include retinal degenerative diseases (retinitis pigmentosa, age-related macular degeneration, etc.), retinopathy (e.g., diabetic retinopathy, proliferative retinopathy, simple retinopathy, etc.), floater, retinal tear, retinal detachment (e.g., rhegmatogenous retinal detachment, non-rhegmatogenous retinal detachment, etc.), and the like. Also included are retinal degenerative diseases, age-related macular degeneration, myopic maculopathy, macular dystrophy, diabetic retinopathy, retinal detachment, and the like. Examples of the disorder or symptom of these diseases include disorders in visual acuity, contrast sensitivity, light-dark adaptation, color vision, etc., and symptoms associated therewith.

As used herein, "suppression of progress" and "delay of progress" are used interchangeably, and these terms refer to the suppression or delay of progress of a disease (e.g., vesicle transport disorder or apoptosis), where the suppression or delay encompasses a reduction in the rate of exacerbations compared to the absence of treatment, as well as maintenance and improvement in the disease levels. If a certain disease has not developed, it falls under "prevention of onset". As used herein, the "onset" refers to appearance of a subjective symptom of disease from a state in which no such subjective symptom of the disease appears. Examples of the subjective symptoms include symptoms such as night blindness, narrowing of vision, photophobia, decreased visual acuity and defective color vision.

As used herein, the terms, "protein," "polypeptide," "oligopeptide," and "peptide", are used interchangeably with the same meaning, and they refer to polymers of amino acids of any length. The polymer may be linear, branched or cyclic. The amino acids may be natural or non-natural, or may be modified amino acids. The term may also encompass those assembled into a complex of multiple polypeptide chains. The term also encompasses naturally or artificially modified amino acid polymers. Such modifications encompass, for example, disulfide bond formation, glycosylation, lipidation, acetylation, phosphorylation or any other manipulation or modification (e.g., conjugation with a labeling component). The subject definition also encompasses, for example, polypeptides including one or more analogs of amino acids (including, for example, unnatural amino acids), peptide-like compounds (e.g., peptoids) and other modifications known in the art. As used herein, an "amino acid" is a general term for organic compounds having an amino group and a carboxyl group. When the antibody according to the embodiment of the present disclosure includes a "specific amino acid sequence", any amino acid in the amino acid sequence may be a chemically-modified amino acid. Furthermore, any amino acid in the amino acid sequence may form a salt or a solvate. Furthermore, any amino acid in the amino acid sequence may be of L-type or D-type. Even in such cases, the protein according to the embodiment of the present disclosure is considered to include the above-mentioned "specific amino acid sequence". As for chemical modifications that amino acids included in proteins undergo *in vivo,* known are, for example, N-terminal modification (e.g., acetylation, myristoylation, etc.), C-terminal modification (e.g., amidation, glycosylphosphatidylinositol addition, etc.), side chain modifications (e.g., phosphorylation, glycosylation, etc.), or the like. It may be natural or non-natural as long as it satisfies the object of the present disclosure.

As used herein, a "chimera" (e.g., protein, opsin, rhodopsin, and the like) refers to a substance in a state in which genetic information derived from different organisms is mixed with each other in the same entity (in this case, protein, rhodopsin, etc.). The chimeric protein includes gene sequences derived from, for example, two or three or more organisms mixed therein. The sequence information contained in the chimeric protein may include a sequence other than the sequence derived from the organism to be mixed.

As used herein, the terms, "polynucleotide", "oligonucleotide", "nucleic acid", and "nucleic acid molecule", are used interchangeably with the same meaning, and they refer to polymers of nucleotides of any length. The terms also include an "oligonucleotide derivative" or "polynucleotide derivative". The "oligonucleotide derivative" or "polynucleotide derivative" refers to an oligonucleotide or polynucleotide containing a derivative of a nucleotide or having an unusual bond between nucleotides, and the terms are used interchangeably. Specific examples of such oligonucleotides include, for example, 2'-O-methyl-ribonucleotide, an oligonucleotide derivative in which a phosphate diester bond in an oligonucleotide is converted to a phosphorothioate bond, an oligonucleotide derivative in which a phosphate diester bond in an oligonucleotide is converted into an N3'-P5'phospholoamidate bond, an oligonucleotide derivative in which ribose and a phosphodiester bond in an oligonucleotide are converted into a peptide nucleic acid bond, an oligonucleotide derivative in which uracil in an oligonucleotide is substituted by C-5 propynyl uracil, an oligonucleotide derivative in which uracil in an oligonucleotide is substituted by C-5 thiazole uracil, an oligonucleotide derivative in which cytosine in an oligonucleotide is substituted by C-5 propynylcytosine, an oligonucleotide derivative in which cytosine in an oligonucleotide is substituted by phenoxazine-modified cytosine, an oligonucleotide derivative in which ribose in DNA is substituted by 2'-O-propyl ribose, and an oligonucleotide derivative in which ribose in an oligonucleotide is substituted by 2'-methoxyethoxyribose, and the like. Unless otherwise indicated, particular base sequences are also intended to include conservatively modified variants (e.g., degenerate codon substitutes) and complementary sequences thereof, similarly to the explicitly indicated sequences. Note that the sequences of nucleic acids are also referred to as nucleic acid sequences, nucleotide sequences, etc., in addition to base sequences, but they all have the same meaning. Specifically, the degenerate codon substitute may be achieved by creating a sequence in which the third position of one or more selected (or all) codons is substituted by a mixed base and/or deoxyinosine residue (Batzer et al., Nucleic Acid Res. 19: 5081(1991); Ohtsuka et al., J. Biol. Chem. 260: 2605-2608(1985); Rossolini et al., Mol. Cell. Probes 8: 91-98(1994)). In accordance with the context, the "nucleic acid" is also used herein interchangeably with genes, DNA such as cDNA, RNA such as mRNA, oligonucleotides, and polynucleotides. The "nucleotide" herein may be natural or non-natural. The nucleic acids can be DNA or RNA herein.

As used herein, a "gene" refers to a factor that defines a genetic trait, and the "gene" may refer to any of a "polynucleotide", an "oligonucleotide" and a "nucleic acid".

As used herein, the terms, "nucleic acid construct", "construct" and "gene construct", are used interchangeably, and they are nucleic acid molecules containing a vector and nucleic acids isolated from naturally occurring genes or combined and juxtaposed in a non-naturally occurring manner.

As used herein, "homology" of a gene refers to the degree of identity of two or more gene sequences to each other, and the concept of having "homology" generally refers to having a high degree of identity or similarity. The term, "identity", refers to the equivalent degree of sequence of the same amino acid, while the term, "similarity", refers to the equivalent degree of sequence, including amino acids of similar nature, in addition to the same amino acid. Thus, as the degree of the homology of two certain genes increases, the degree of the identity or similarity of their sequences increases. Whether or not two different genes have homology can be examined by direct sequence comparison or, in the case of nucleic acids, hybridization under stringent conditions. In a direct comparison between two gene sequences, those genes are homologous when the DNA sequences are typically at least 50% identical, preferably at least 70% identical, and more preferably at least 80%, 90%, 95%, 96%, 97%, 98% or 99% identical, between the gene sequences thereof. Thus, as used herein, a "homologue" or "homologous gene product" means a protein in another species, preferably a mammal, that exerts the same biological functions as the protein components of the complex further described herein. Such homologues are also sometimes referred to as "ortholog gene products". It is understood that such homologues, homologous gene products, ortholog gene products and the like can also be used as long as these substances meet the object of the present disclosure.

Amino acids can be referred to herein by either their generally known three-letter symbols or the one-letter symbols recommended by the IUPAC-IUB Biochemical Nomenclature Commission. Nucleotides can also be referred to by the generally recognized one-letter codes. Herein, comparison of similarity, identity and homology of amino acid sequences and base sequences is calculated with default parameters using a tool for sequence analysis, BLAST. The identity search can be performed using, for example, NCBI's BLAST 2.2.28 (issued on 4.2.2013) (Proc. Natl. Acad. Sci. USA 90: 5873-5877, 1993). The value of identity herein usually refers to the value obtained by performing alignment under the default conditions using the above BLAST. However, if a higher value is obtained by varying the parameters, the highest value obtained is set as the value for the identity. When identity is evaluated in multiple regions, the highest value among them is set as the value for the identity. Similarity refers to a numerical value that takes into account similar amino acids in addition to identity. Blastp can be used with default settings for the algorithm in the comparison between amino acid sequences in BLAST. The measurement results are quantified as Positives or Identities. The homology of the amino acid sequence and base sequence can be determined by the algorithm BLAST by Karlin and Altschul. Based on this algorithm, programs called BLASTN and BLASTX have been developed (Altschul et al. J. Mol. Biol. 215: 403-410, 1990). When the base sequence is analyzed by BLASTN based on BLAST, the parameters are set as, for example, score = 100 and world length = 12. When the amino acid sequence is analyzed by BLASTX based on BLAST, the parameters are set as, for example, score = 50 and world length = 3. When BLAST and Gapped BLAST programs are used, the default parameters of each program are used. Specific techniques of these analysis methods are known (http: //www.ncbi.nlm.nih.gov.) .

The nucleic acid or protein as used herein may include a sequence in which one or more amino acids or nucleotides are substituted, deleted and/or added in the amino acid or base sequence of interest. In this regard, the term "one or more", in the chimeric protein full-length amino acid sequence, typically means 50 amino acids or less, preferably 30 amino acids or less, and still more preferably 10 amino acids or less (e.g., 5 amino acids or less, 3 amino acids or less, or one amino acid) . Further, "one or more", in an amino acid sequence of a domain, typically means 6 amino acids or less, preferably 5 amino acids or less, and still more preferably 4 amino acids or less (e.g., 3 amino acids or less, 2 amino acids or less, and one amino acid). When maintaining the claimed biological activity of chimeric protein, it is desirable that an amino acid residue to be mutated is mutated to another amino acid which conserves the property of the amino acid side chain. Examples of properties of an amino acid side chain include hydrophobic amino acids (A, I, L, M, F, P, W, Y, V), hydrophilic amino acids (R, D, N, C, E, Q, G, H, K, S, T), amino acids with an aliphatic side chain (G, A, V, L, I, P), amino acids with a hydroxyl group containing side chain (S, T, Y), amino acids with a sulfur atom containing side chain (C, M), amino acids with a carboxylic acid and amide containing side chain (D, N, E, Q), amino acids with a base containing side chain (R, K, H), and amino acids with an aromatic containing side chain (H, F, Y, W) (each symbol within the parenthesis represents the one-letter code of an amino acid). These are also referred to herein as "conservative substitutions". Note that a protein having an amino acid sequence modified by deletion, addition and/or substitution with another amino acid of one or more amino acid residues to the amino acid sequence, is known to maintain the biological activity thereof (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413). Therefore, in one embodiment of the present disclosure, "several" may be, for example, 10, 8, 6, 5, 4, 3, or 2, or may be less than or equal to any one of these numerical values. Chimeric protein with deletion etc. can be produced, for example, by a site-specific mutagenesis method, a random mutagenesis method, biopanning using an antibody phage library, or the like. As a site-specific mutagenesis method, KOD-Plus-Mutagenesis Kit (TOYOBO CO., LTD.), for example, can be used. It is possible to select an antibody having the same activity as the wild type, from the mutant-type antibody into which the deletion or the like has been introduced, by performing various characterizations, such as FACS analysis and ELISA.

In one embodiment of the present disclosure, the amino acid sequence and nucleic acid sequence of the chimeric protein of the present disclosure may have 70% or more, 80% or more, or 90% or more identity or similarity with the reference sequence. Regarding the amino acid sequence or base sequence herein, "70% or more" may be, for example, 70, 75, 80, 85, 90, 95, 96, 97, 98, 99% or more; "80% or more" may be, for example, 80, 85, 90, 95, 96, 97, 98, 99% or more; "90% or more" may be, for example, 90, 95, 96, 97, 98, 99% or more, or may be within the range of any two of the values. As for the "similarity", the proportion of homologous amino acids between two or more amino acid sequences may be calculated according to methods known in the art. Before calculating the proportion, the amino acid sequences of the group of amino acid sequences to be compared are aligned, and gaps are introduced in a part of the amino acid sequences if necessary to maximize the proportion of identical amino acids. Methods for alignment, methods for calculating proportions, comparison methods, and computer programs related thereto have been well known in the art (e.g., BLAST, GENETYX, etc.). The proportion of the same amino acids is calculated in the case of "identity", whereas the proportion of similar amino acids is calculated in the case of "similarity". Similar amino acids include, but are not limited to, amino acids that can be conservatively substituted.

As used herein, a "polynucleotide that hybridizes under stringent conditions" refers to well-known conditions commonly used in the art. Such a polynucleotide can be obtained by using a polynucleotide selected from the polynucleotides of the present disclosure as a probe and using a colony hybridization method, a plaque hybridization method, a Southern blot hybridization method, or the like. Specifically, the polynucleotide as above means such a polynucleotide that can be identified by performing hybridization at 65°C in the presence of 0.7 to 1.0 M NaCl, using a filter with DNA immobilized from colonies or plaques, and then washing the filter under 65°C conditions using a SSC (saline-sodiumcitrate) solution with a concentration of 0.1 to 2-fold (note that the composition of the 1-fold SSC solution is 150 mM sodium chloride and 15 mM sodium citrate). For the "stringent conditions", the following conditions, for example, can be adopted: (1) use of low ionic strength and high temperature for washing (e.g., 0.015 M sodium chloride/0.0015 M sodium citrate/0.1% sodium dodecyl sulfate, at 50°C); (2) use of denaturing agents, such as formamide, during hybridization (e.g., 50% (v/v) formamide and 0.1% bovine serum albumin/0.1% ficoll/0.1% polyvinylpyrrolidone/50 mM sodium phosphate buffer with pH of 6.5, and 750 mM sodium chloride, 75 mM sodium citrate, at 42°C); or (3) incubation in a solution containing 20% formamide, 5xSSC, 50 mM sodium phosphate (pH 7.6), 5x Denhardt's solution, 10% dextran sulfate and 20 mg/ml denatured shear salmon sperm DNA at 37°C overnight, followed by washing the filter with 1 × SSC at about 37-50°C. Note that the formamide concentration may be 50% or higher. The washing time may be 5, 15, 30, 60 or 120 minutes, or more. Multiple factors such as temperature and salt concentration can be considered as factors that affect the stringency of the hybridization reaction, the details of which can be found in Ausubel et al., Current Protocols in Molecular Biology, Wiley Interscience Publishers, (1995). Examples of "highly stringent conditions" are 0.0015M sodium chloride, 0.0015M sodium citrate, at 65-68°C, or 0.015M sodium chloride, 0.0015M sodium citrate and 50% formamide at 42°C. As for hybridization, it can be carried out according to a method described in an experimental document, such as Molecular Cloning 2nd ed., Current Protocols in Molecular Biology, Supplement 1-38, DNA Cloning 1: Core Techniques, A Practical Approach, Second Edition, Oxford University Press (1995), or the like. Here, sequences containing only the A sequence or only the T sequence are preferably excluded from the sequences that hybridize under the stringent conditions. Moderately stringent conditions can be readily determined by one of ordinary skill in the art, based on, for example, the length of the DNA, as shown in Sambrook et al., Molecular Cloning: A Laboratory Manual, No. 3, Vol. 1, 7.42-7.45 Cold Spring Harbor Laboratory Press, 2001. Furthermore, with regard to nitrocellulose filters, included are use of hybridization conditions of 5 × SSC, 0.5% SDS, 1.0 mM EDTA (pH 8.0) prewash solution, about 50% formamide at about 40-50°C, and 2 × SSC-6 × SSC (or other similar hybridization solution, such as Stark's solution, in about 50% formamide at about 42°C), and washing conditions of about 60°C, 0.5 × SSC and 0.1% SDS. Accordingly, the polypeptide used in the present disclosure also includes a polypeptide encoded by a nucleic acid molecule that hybridizes under highly or moderately stringent conditions to the nucleic acid molecule encoding the polypeptide specifically described in the present disclosure.

As used herein, a "purified" substance or biological factor (e.g., nucleic acid or protein) refers to one from which at least some of the factors naturally associated with the substance or biological factor have been removed. Therefore, the purity of the biological factor in the purified biological factor is usually higher (i.e., more enriched) than the purity of the biological factor in the state in which the biological factor is normally present. The term "purified" as used herein means that there are preferably at least 75% by weight, more preferably at least 85% by weight, even more preferably at least 95% by weight, and most preferably at least 98% by weight of biological factors of the same type. The substance or biological factor used in the present disclosure is preferably a "purified" substance. An "isolated" substance or biological factor (e.g., nucleic acid or protein) as used herein refers to one in which a factor naturally associated with the substance or biological factor has been substantially removed. The term "isolated" as used herein varies in accordance with its purpose and therefore does not necessarily have to be expressed in purity, but if necessary, the term means that there are preferably at least 75% by weight, more preferably at least 85% by weight, even more preferably at least 95% by weight, and most preferably at least 98% by weight of biological factors of the same type. The substance used in the present disclosure is preferably an "isolated" substance or biological factor.

As used herein, a "corresponding" amino acid or nucleic acid or moiety refers, in a polypeptide or polynucleotide molecule (e.g., rhodopsin), to an amino acid or nucleotide that has or is expected to have the same effect as a given amino acid or nucleotide or moiety in a polypeptide or polynucleotide that serves as a reference for comparison. In particular, as for an enzyme molecule, it refers to an amino acid that exists at a similar position in the active site and makes a similar contribution to catalytic activity, whereas as for a complex molecule, it refers to a corresponding moiety (e.g., heparan sulfate, etc.). In an antisense molecule, for example, it may be a similar moiety in the ortholog that corresponds to a particular moiety of the antisense molecule. The corresponding amino acid may be, for example, a specific amino acid that is cysteineized, glutathioneized, S-S bond formed, oxidized (e.g., methionine side chain oxidation), formylated, acetylated, phosphorylated, glycosylated, myristylated, and the like. Alternatively, the corresponding amino acid may be the amino acid responsible for dimerization. Such "corresponding" amino acids or nucleic acids may be regions or domains over a range. Thus, in such a case, they are referred to herein as a "corresponding" region or domain. Such a corresponding region or domain is useful when designing a complex molecule in the present disclosure.

As used herein, a "corresponding" gene (in which case it may be a sequence or molecule of polynucleotide, encoding rhodopsin or the like) refers, in a certain species, to a gene (in which case it may be a sequence or molecule of polynucleotide, encoding rhodopsin or the like) that has or is expected to have the same effect as a given gene in the species of reference for comparison. When there are multiple genes having such an action, those having the same evolutionary origin are referred to as the corresponding genes. Thus, the gene corresponding to a gene may be the ortholog of that gene. Thus, for each human rhodopsin, the corresponding rhodopsin can be found in other animals (particularly mammals). Such corresponding genes can be identified using techniques well known in the art. Thus, for example, with regard to a corresponding gene in a certain animal (e.g., a mouse), the gene of reference for the corresponding gene (e.g., rhodopsin, etc.) can be found by searching a database containing the sequences of the animal, with a sequence of SEQ ID NO: 9 to 16 or the like used as a query sequence.

As used herein, "part", "fragment", or "fragments" refers to a polypeptide or polynucleotide having a sequence length from 1 to n-1 with respect to a full-length polypeptide or polynucleotide (having the length of n). The length of the fragment can be appropriately varied in accordance with its purpose. For example, the lower limit of the length, in the case of a polypeptide, includes 3, 4, 5, 6, 7, 8, 9, 10, 15, 20, 25, 30, 40, 50 and more amino acids, and other lengths represented by integers not specifically listed here (e.g., 11) may also be appropriate as the lower limit. Furthermore, in the case of a polynucleotide, included are 5, 6, 7, 8, 9, 10, 15, 20, 25, 30, 40, 50, 75, 100 and more nucleotides, and other lengths represented by integers not specifically listed here (e.g., 11) may also be appropriate as the lower limit. It is understood herein that any fragment may fall within the scope of the present disclosure when the full length one, for example, functions as a marker or target molecule and the fragment itself also functions as a marker or target molecule.

According to the present disclosure, the term "activity" as used herein refers to the function of a molecule in the broadest sense. The activity generally includes, without intention of limitation, the biological, biochemical, physical or chemical function of the molecule. The activity includes, for example, enzyme activity, ability to interact with other molecules, ability to activate, promote, stabilize, inhibit, suppress or destabilize the function of other molecules, stability, and ability to localize to a specific intracellular location. Where applicable, the term also relates to the function of protein complexes in the broadest sense. As used herein, "biological activity" includes activation of photochemical reactions and the like.

As used herein, a "functional equivalent" refers to any entity having the same target function but a different structure with respect to the original entity of interest. It is thus understood that the functional equivalent of "rhodopsin" or a chimera thereof includes, not the rhodopsin or chimera thereof itself, but a mutant or variant (e.g., an amino acid sequence variant, etc.) of the rhodopsin or chimera thereof having the biological activity of the rhodopsin or chimera thereof, and further includes one that, at the time of action, can be transformed into rhodopsin or an antibody thereof or a mutant or variant of the rhodopsin or a chimera thereof (including, for example, a nucleic acid encoding rhodopsin or a chimera thereof or a mutant or variant of rhodopsin or a chimera thereof, and a vector, cell, etc., containing the nucleic acid). As the functional equivalent of the present disclosure, an amino acid sequence in which one or more amino acids are inserted, substituted and/or deleted, or added to one or both ends thereof can be used. As used herein, an "amino acid sequence in which one or more amino acids are inserted, substituted and/or deleted, or added to one or both ends thereof" means that it has been modified with substitution or the like of a plurality of amino acids that can occur naturally, by a well-known technical method such as site-specific mutagenesis, or by a natural mutation. The modified amino acid sequence can be, for example, one in which 1 to 30, preferably 1 to 20, more preferably 1 to 9, still more preferably 1 to 5, and particularly preferably 1 to 2 amino acids have been inserted, substituted or deleted, or added to one or both ends thereof. The modified amino acid sequence may preferably be such an amino acid sequence that has one or more (preferably one or several or 1, 2, 3, or 4) conservative substitutions in the rhodopsin amino acid sequence.

As used herein, an "agent", "-agent" or "factor" (any of which corresponds to the word: *agent,* in English) may be used interchangeably in a broad sense, may be any substance or other element (e.g., energy, such as light, radioactivity, heat and electricity) that is capable of achieving the intended objective thereof. Examples of such substances include, without limitation, proteins, polypeptides, oligopeptides, peptides, polynucleotides, oligonucleotides, nucleotides, nucleic acids (including, for example, cDNA, DNA such as genomic DNA, RNA such as mRNA), polysaccharides, oligosaccharides, lipids, organic small molecules (e.g., hormones, ligands, messenger substances, organic small molecules, molecules synthesized by combinatorial chemistry, small molecules that can be used as pharmaceuticals (for example, small molecule ligands), etc.).

For oral administration, the agent may be formulated into various forms such as tablets, granules, fine granules, powders, capsules, etc., for use. Furthermore, additives commonly used in formulations such as binders, encapsulating agents, excipients, lubricants, disintegrants and wetting agents may be included therein. Furthermore, in addition to these, formulations for oral administration may be formulated in a liquid form such as an internal solution, suspension, emulsion or syrup, or may be formulated in a dry form to be re-dissolved at the time of use.

For parenteral administration, the agent may be formulated to be contained in a unit dose ampule or multidose container or tube. An additive such as a stabilizer, buffer, preservative, or isotonizing agent may also be included. A formulation for parenteral administration may also be formulated into a powder form that can be dissolved in a suitable carrier (sterilized water or the like) upon use.

Examples of parenteral administration include: intraocular administration such as intravitreal administration, subretinal administration and intracameral administration; and extraocular administration such as subconjunctival administration, subtenon administration and eye drop administration. The intravitreal administration is preferred. The composition and the like according to the present disclosure can be used for the treatment, prevention, suppression of progress, and the like, by administration to humans using the aforementioned method.

As used herein, "treatment" refers to preventing the exacerbation of a disease or disorder (e.g., vesicle transport disorder or apoptosis) in the event of such a condition, preferably maintaining the status quo, more preferably alleviating, and even more preferably resolving, of the disease or disorder, including the possible exertion of a symptom improving or preventing effect on the patient's disease or one or more symptoms associated with the disease. Conducting diagnosis in advance and appropriate treatment is called "companion treatment", and the diagnostic agent for that purpose is sometimes called "companion diagnostic agent". Since the present disclosure targets genetic disorders, the gene may be tested in advance to treat the patient.

As used herein, a "therapeutic drug (agent)" refers, in a broad sense, to any agent capable of treating a target condition (for example, retinal degenerative disease). In one embodiment of the present disclosure, the "therapeutic drug" may be a pharmaceutical composition comprising an active ingredient and one or more pharmacologically acceptable carriers. The pharmaceutical composition can be manufactured, for example, by mixing an active ingredient with the above carrier and using any method known in the technical field of pharmaceutics. Further, the therapeutic drug is not limited in the form of use as long as it is used for treatment, and may be an active ingredient alone or a mixture of an active ingredient and any component. Further, the shape of the carrier is not particularly limited, and may be, for example, a solid or a liquid (e.g., a buffer solution).

As used herein, "prevention" refers, with regard to a disease or disorder (e.g., retinal degenerative disease), to preventing one from having such a condition before being in such a condition. The agent of the present disclosure can be used for diagnosis, and if necessary, the agent of the present disclosure can be used to prevent, for example, retinal degenerative diseases, or to take preventive measures. As used herein, a "preventive drug (drug)" refers, in a broad sense, to any agent that can prevent a target condition (e.g., vesicle transport disorder or apoptosis).

As used herein, a "kit" refers to a unit that is usually divided into two or more compartments and provides portions to be provided (e.g., nucleic acids, nucleic acid constructs, cells into which the nucleic acid of interest has been gene-introduced, test agents, diagnostic agents, therapeutic agents, antibodies, labels, instruction manuals, etc.). Where it is preferable to administer a specific medicine (nucleic acid medicine, etc.) only to appropriate patients after identifying the patients to be administered using a reagent for determining the characteristics of the patients, such as a companion diagnostic agent, moreover, a kit may be provided when a diagnostic agent and a therapeutic agent are provided in combination. Alternatively, this form of the present kit is preferable when the purpose thereof is to provide a composition, such as certain unstable medicine, that should not be mixed and provided, but is preferably mixed and used immediately prior to use, for stability reasons or the like. It is advantageous for such a kit to comprise preferably an instruction, or a written explanation, describing how to use the portions to be provided (e.g., nucleic acids, nucleic acid constructs, cells into which the nucleic acid of interest has been gene-introduced, test agents, diagnostic agents, or therapeutic agents) or how the reagent should be processed. When the kit is used as a reagent kit in the present specification, the kit usually includes an instruction or the like describing how to use a test agent, a diagnostic agent, a therapeutic agent, an antibody, and the like.

As used herein, an "active ingredient" refers to an ingredient contained in an amount necessary for the composition of the present disclosure to attain a target effect, such as treatment, prevention or suppression of progress, and may also contain other ingredients as long as the effect is not compromised below the desired level. Further, the pharmaceuticals, compositions and the like of the present disclosure may be those that are formulated. In addition, the route of administration of the pharmaceuticals, compositions, etc. of the present disclosure may be oral or parenteral, and can be appropriately set according to the form of the formulation or the like.

As used herein, an "instruction" (including package inserts, labels used by the US FDA, etc.) refers to such an instruction that describes to a physician or other user how to use a method that uses the present disclosure. The instruction contains words instructing a detection method according to the present disclosure, how to use a diagnostic agent, or administration of pharmaceuticals or the like. In addition, the instruction may include words instructing for oral administration or administration to the retina (for example, by injection) as the administration site. This instruction is prepared in accordance with the format prescribed by the regulatory agency of the country in which the present disclosure is implemented (for example, the Ministry of Health, Labor and Welfare in Japan, the Food and Drug Administration (FDA) in the United States, etc.), and the instruction clearly states that it has been approved by the regulatory agency. The instruction is a so-called package insert or label and is usually provided in a paper medium; however, without limitation thereto, the instruction may also be provided in a form of, for example, an electronic medium (e.g., a website provided on the Internet, and e-mail).

### (Preferred Embodiments)

Preferred embodiments of the present disclosure will be described below. It is understood that the embodiments provided below are provided for a better understanding of the present disclosure and the scope of the present disclosure should not be limited to the following description. Therefore, it is clear that those skilled in the art can appropriately make modifications within the scope of the present disclosure in consideration of the description in the present specification. It is also understood that the following embodiments of the present disclosure may be used alone or in combination.

### (Retinal Modulating Agent)

In one aspect, the present disclosure provides novel uses for retinal modulating agents. In one embodiment of the present disclosure, the retinal modulating agent modulates the amount or concentration of retinal. Further, in one embodiment, the retinal modulating agent can modulate the amount or concentration of retinal in the retina. In other embodiments, the retinal modulating agent can also modulate the cis/trans ratio of retinal (ratio of 11-cis-retinal to all-trans retinal). In one embodiment, such retinal modulating agents that modulate the amount, concentration, or cis/trans ratio of retinal include vitamin A. Since vitamin A can be a source of retinal, it can modulate the amount of retinal in the retina. In other embodiments, such retinal modulating agents can include 9-cis-retinal, 9-cis-retinyl-acetate, and 9-cis-β-carotene. These substances bind to rhodopsin and receive light, but are metabolized to isorhodopsin instead of all-trans-retinal. Since this isorhodopsin does not enter the regeneration system of the visual cycle, the amount of all-trans-retinal is reduced and the burden on the visual cycle is reduced. This is thought to be particularly effective in retinitis pigmentosa with mutations in the visual cycle-related genes (ABCA4, LRAT, RPE65). In other embodiments, the retinal modulating agents can also include retinylamine and emixustat. These are substances that act as visual cycle modulators by acting on a metabolic circuit called the visual cycle in which all-trans-retinal photoisomerized from 11-cis-retinal to all-trans-retinal is returned to 11-cis-retinal again. By modulating the speed of the circuit, they suppress the accumulation of the toxic substance all-trans-retinal. In other embodiments, the retinal modulating agents further include: RBP4-TTRcomplex, which is a vitamin A remover; and Primary Amines, which is an all-trans retinal remover. As the retinal modulating agents, it is also possible to use: chimeric rhodopsins of ion-transporting receptor rhodopsin and G protein-coupled receptor rhodopsin; other types of opsins; or nucleic acid molecules encoding them. In other embodiments, the retinal modulating agents further include Zuretinolacetate (9-cis-retinol), which functions as a substitute for 11-cis-retinal, thereby substantially functioning as a modulating agent.

The isomerization of 11-cis-retinal to all-trans-retinal and the subsequent resynthesis of 11-cis-retinal, play an important function in maintaining visual substances in the visual cycle. Furthermore, visual substance regeneration also plays an important role in the process of recovery of visual cell sensitivity, that is, dark adaptation. On the other hand, all-trans-retinal produced in this visual cycle is rapidly metabolized by ATP-binding transporter ABCA4 and retinol dehydratase RDH8. However, any abnormality in this metabolic system causes an increase in the concentration of all-trans-retinal in visual cells. All-trans-retinal has a highly reactive aldehyde group, and thus, it has been suggested that all-trans-retinal may be involved in toxicity. In addition, a delayed metabolism of all-trans-retinal is known to produce A2E, an adduct thereof with ethanolamine. Accumulation of A2E is known in diseases such as age-related macular degeneration. Therefore, the metabolism of all-trans-retinal is extremely important.

In one embodiment, as for the retinal modulating agents as described above, it is possible to use retinal modulating agents that act on retinal, such as cis-retinal that does not produce all-trans-retinal upon photoreception, retinal that acts on the visual cycle to prevent the accumulation of all-trans-retinal, and retinal that directly removes all-trans-retinal. As such, in one embodiment of the present disclosure, it is possible to use a "relative retinal modulating agent" that directly or indirectly acts on retinal to relatively modulate cis-retinal and trans-retinal. As for the relative retinal modulating agent, since vitamin A, for example, can be a material for retinal, it can be said to be a relative retinal modulating agent in that vitamin A modulates the amount of retinal itself. Furthermore, 9-cis-retinal, 9-cis-retinyl-acetate, and 9-cis-β-carotene couple to opsin to form rhodopsin, which is metabolized to isorhodopsin rather than all-trans-retinal upon exposure to light. This thus can reduce the amount of all-trans-retinal. That is, these substances can be considered as relative retinal modulating agents in that they modulate the amount of all-trans-retinal. In addition, retinylamine and emixustat modulate the amount, concentration, or cis/trans ratio of retinal by modulating the speed of the visual cycle. They thus can be considered to act on retinal, and therefore, they can be considered as relative retinal modulating agent. Furthermore, RBP4-TTRcomplex, a vitamin A remover, and Primary Amines, an all-trans-retinal remover, modulate the amount, concentration, or cis/trans ratio of retinal through the removal of vitamin A and all-trans-retinal. These substances can thus be considered to act on retinal, and therefore, they can be considered as relative retinal modulating agent. Furthermore, Zuretinolacetate (9-cis-retinol) can be considered to act on retinal in that it functions as a substitute for 11-cis-retinal, and therefore, it can be considered as a relative retinal modulating agent.

In other embodiments, absolute retinal modulating agents, such as rhodopsin, which do not produce toxic metabolites such as all-trans-retinal, are also available. Such a rhodopsin, which does not produce toxic metabolites such as all-trans-retinal, can modulate the amount, concentration, or cis/trans ratio of retinal without producing all-trans-retinal in the first place, unlike the natural rhodopsin, in which 11-cis-retinal released by photoreception undergoes photoisomerization to all-trans-retinal, and the all-trans-retinal is converted back to 11-cis-retinal for use as a cofactor.

In one embodiment of the present disclosure, rhodopsin, which does not release retinal upon photoreception, or a nucleic acid molecule encoding it, can be used as a retinal modulating agent. The rhodopsin that does not release retinal upon photoreception includes microbial opsins typified by the ion-transporting receptor rhodopsin, and it may also be: vertebrate non-visual opsins; invertebrate opsins; or other opsins containing microbial-type opsin structures. There is no particular limitation to such substances as long as they do not release retinal upon photoreception.

Opsins (rhodopsins) can be broadly classified into microbial opsins and animal opsins, and the animal opsins can be further classified into vertebrate visual opsins, vertebrate non-visual opsins, and invertebrate opsins. Further, opsins (rhodopsins) can also be classified into Type I opsins and Type II opsins. The animal opsins are further classified into vertebrate visual opsins, vertebrate non-visual opsins, and invertebrate opsins; and the chimeric rhodopsin of the present disclosure is a chimeric protein of a microbial opsin and a vertebrate visual opsin. In addition, The vertebrate non-visual opsins and the invertebrate opsins are called bistable opsins, and include opsins that use all-trans-retinal as a chromophore, like microbial opsins. The opsins that do not release retinal upon photoreception (rhodopsins) can include microbial opsins as well as some of vertebrate non-visual opsins and invertebrate opsins.

In other embodiments, as for the opsins (rhodopsins) that do not release retinal upon photoreception, it is also possible to use: bistable opsins, which have 11-cis-retinal in their chromophore and are known to be difficult to release retinal even if they undergo photoreception and photoisomerization; opsins of the types that can be reversed by light of specific wavelengths; and special opsins that have all-trans-retinal in their chromophores and do not release retinal similar to the microbial forms, which have been recently discovered. In one embodiment, the opsins (rhodopsins) that do not release retinal upon photoreception can include non-bleaching pigments and photobleaching-resistant opsins, from the viewpoint of photobleaching. Opsins (rhodopsins) that do not release retinal upon photoreception include non-bleaching pigments and photobleaching-resistant opsins from the viewpoint of photobleaching. For example, microbial rhodopsin is also included in non-bleaching pigments. Therefore, the opsins that can be used in the present disclosure can include: microbial opsins, non-visual opsins, and some invertebrate opsins; from the chromophore point of view, opsins having all-trans-retinal as a chromophore, and bistable opsins among opsins having 11-cis-retinal as a chromophore; and from the point of view of photobleaching, bistable pigments and non-bleaching pigments, and chimeric proteins of these opsins and animal opsins.

As used herein, "rhodopsin" and "opsin" can be used interchangeably in accordance with the context, or a combination of the protein opsin and the chromophore retinal can be referred to as rhodopsin. The microbial type is often called rhodopsin because it does not release the chromophore retinal. On the other hand, rhodopsin narrowly refers to animal-type rod visual substances in the animal-type category. Thus, in order to make a distinction, those other than animal-type rod visual substances are often referred to as opsins. However, persons skilled in art can understand what is meant in accordance with the context.

In one embodiment of the present disclosure, the absolute retinal modulating agent is a chimeric rhodopsin, which can be, for example, a chimeric rhodopsin comprising at least a part of the ion-transporting receptor rhodopsin and at least a part of the G protein-coupled receptor rhodopsin. In other embodiments, the absolute retinal modulating agent can be a vertebrate non-visual opsin, an invertebrate opsin, or a microbial opsin. The chimeric rhodopsin of the present disclosure can be considered to be an absolute retinal modulating agent in that it becomes a rhodopsin that does not produce all-trans-retinal by the gene introduction thereof into the opsin that constitutes rhodopsin. Any chimeric rhodopsin may be used in the present disclosure as long as the purpose of the present disclosure can be achieved. A chimeric rhodopsin as used in the present disclosure is typically a chimeric protein comprising at least a part of the ion-transporting receptor rhodopsin and at least a part of the G protein-coupled receptor rhodopsin. To explain a typical example, fusing a part of the reusable microbial ion-transporting receptor rhodopsin to a part of the animal-derived G protein-coupled receptor rhodopsin can obtain high activity mediated by endogenous G proteins by the G protein-coupled receptor while retaining the function of repeated activation of the ion-transporting receptor, ion channel receptor rhodopsin derived from microorganisms. This can exert therapeutic, ameliorating, preventive, or progress-suppressing effects on diseases, disorders or symptoms associated with endoplasmic reticulum stress or all-trans-retinal. Use of a vertebrate non-visual opsin, invertebrate opsin, microbial opsin, or chimeric rhodopsin as an absolute retinal modulating agent eliminates the need for the visual cycle, which is a metabolic regeneration system for retinal, and thus eliminates the production of toxic all-trans-retinal. Therefore, this can exert therapeutic, ameliorating, preventive, or progress-suppressing effects on diseases, disorders or symptoms associated with all-trans-retinal. The present disclosure includes gene transfer therapy using optogenetics, which can be expected to have safe and long-term effects with minimal invasiveness. In some embodiments, the present disclosure utilizes endogenous G-protein signaling cascades and channels and uses the native physiological phototransduction pathways to enable highly efficient and safe therapeutics.

In one embodiment, ion pump receptor rhodopsin and ion channel receptor rhodopsin can be used as the ion-transporting receptor rhodopsin used in the chimeric rhodopsin of the present disclosure. In a preferred embodiment, the ion-transporting receptor rhodopsin is preferably of microbial origin, and those from cyanobacteria (blue-green bacteria), for example, are typical ones. Examples thereof include rhodopsin derived from microorganisms belonging to eubacteria, such as the genus *Gloeobacter,* and eukaryotes, such as the genus *Volvox,* genus *Chlamydomonas,* and genus *Guillardia.* Examples of the genus *Gloeobacter* include *Gloeobacter violaceus* and the like. Examples of the genus *Volvox* include *Volvox carteri* and the like. Examples of the genus *Chlamydomonas* include *Chlamydomonas reinhardtii* and the like. Examples of the genus *Guillardia* include *Guillardia theta* and the like. In a preferred embodiment, the chimeric rhodopsin of the present disclosure is such a chimeric protein in which the amino acid sequences of the second loop on the cytoplasm side and/or the third loop on the cytoplasm side, of the amino acid sequence of rhodopsin of *Guillardia theta,* are substituted by the amino acid sequences of the second loop on the cytoplasm side and/or the third loop on the cytoplasm side of the G protein-coupled receptor rhodopsin.

In one embodiment, the G protein-coupled receptor rhodopsin used in the chimeric protein of the present disclosure is typically derived from mammals, and rhodopsin derived from rodents, artiodactyls, cloven-hoofed animals, primates, carnivores, and the like is preferable, rhodopsin derived from artiodactyls or primates is more preferable, and rhodopsin derived from primates is still more preferable. In addition, preferable G protein-coupled receptor rhodopsin includes, for example, rhodopsin derived from bovine, human, mouse, rat, cat, dog, pig, sheep, horse and the like. Of these, bovine or human-derived rhodopsin is particularly preferable.

In a certain embodiment, the chimeric rhodopsin that the nucleic acid construct etc. of the present disclosure encodes is a chimeric protein comprising part of an ion-transporting receptor rhodopsin and part of a G protein-coupled receptor rhodopsin, and having a seven-time-transmembrane structure. In the present disclosure, the chimeric protein comprising part of an ion-transporting receptor rhodopsin and part of a G protein-coupled receptor rhodopsin is preferably designed to highly exert both: a function of repeatedly activating the ion-transporting receptor rhodopsin; and the G protein activity by the G protein-coupled receptor rhodopsin. From this point of view, the chimeric protein of the present disclosure maintains high activity of both, and particularly exhibits high visual function regeneration ability, and thus, the chimeric protein that the nucleic acid construct of the present disclosure preferably encodes a chimeric protein in which the amino acid sequences of the second loop on the cytoplasm side and/or the third loop on the cytoplasm side of the amino acid sequences of the ion-transporting receptor rhodopsin are substituted by the amino acid sequences of the second loop on the cytoplasm side and/or the third loop on the cytoplasm side of the G protein-coupled receptor rhodopsin. Note that the "second loop on the cytoplasm side" and the "third loop on the cytoplasm side" refer to loops located second from the N-terminal side and third from the N-terminal side of the seven loops, respectively.

In one embodiment, it is advantageous for the chimeric rhodopsin of the present disclosure to have an amino acid sequence in which glutamic acid corresponding to position 132 of the amino acid sequence of SEQ ID NO: 14 (GR) is substituted by glutamine. Examples of glutamine-substituted amino acid sequences include, but are not limited to, the amino acid sequences set forth in SEQ ID NO: 5 and the like.

The method for obtaining a nucleic acid, such as DNA, of the present disclosure is not particularly limited, and examples thereof include a method of obtaining cDNA by reverse transcription from mRNA (for example, RT-PCR method), a method of preparation from genomic DNA, a method of synthesis by chemical synthesis, a method of isolation from a genomic DNA library or a cDNA library, and other known methods (see, for example, Japanese Laid-Open Publication No. 11-295999).

Herein, the chimeric protein can be prepared, for example, by using a transformant into which an expression vector comprising nucleic acids, such as DNA, encoding the aforementioned chimeric protein has been introduced. For example, first, this transformant is cultured under appropriate conditions to synthesize a chimeric protein encoded by the nucleic acids such as DNA. Then, the synthesized protein is recovered from the transformant or the culture medium, so that the chimeric protein of the present disclosure can be obtained.

More specifically, the chimeric protein can be prepared by inserting the above-mentioned DNA encoding the chimeric protein into an appropriate expression vector. An "appropriate expression vector" may be any vector that can replicate, retain or self-proliferate in various hosts of prokaryotes and/or eukaryotes, and can be appropriately selected in accordance with the purpose of use. For example, a high copy vector can be selected when a large amount of nucleic acids, such as DNA, are to be obtained, while an expression vector can be selected when a polypeptide (chimeric protein) is to be obtained. Specific examples thereof include, without particular limitation, known vectors described in Japanese Laid-Open Publication No. 11-29599.

In addition, the expression vector can be used, not only for the synthesis of chimeric proteins, but also for the composition of the present disclosure or the like. Specifically, the composition of the present disclosure or the like may contain an expression vector in which the nucleic acid construct etc. of the present disclosure described above is incorporated, as an active ingredient. The direct introduction of such an expression vector into humans can be used for the treatment, prevention and suppression of progress of diseases, disorders or symptoms of the retina. As the vector in this case, a vector that can be introduced into human cells is used. As such a vector, preferable are, for example, an adeno-associated virus vector (AAV vector) and a lentiviral vector.

The method for introducing the vector can be appropriately selected in accordance with the type of vector and host, and the like. Specific examples thereof include, but are not limited to, known methods such as a protoplast method and a competent method when a bacterium is used as a host (see, for example, Japanese Laid-Open Publication No. 11-29599). When the expression vector is used as an active ingredient of the visual function regenerating agent or the visual function deterioration preventing agent of the present disclosure, the introduction can be achieved by injecting the above AAV vector or the like into the eye, for example.

The hosts into which the expression vector is introduced may be any hosts that are compatible with the expression vector and can be transformed. Specific examples thereof include, but are not particularly limited to, bacteria, yeast, animal cells, insect cells, and other known natural cells or artificially established cells (see Japanese Laid-Open Publication No. 11-29599), or humans, mice and other animals. The culturing of transformants can be performed by appropriately selecting a medium form from known nutrient media, and by appropriately adjusting the temperature, pH of the nutrient medium, culture time and the like, in accordance with the type of transformant, and the like (see, for example, Japanese Laid-Open Publication No. 11-29599).

Opsins of the present disclosure, in one preferred embodiment, are provided as nucleic acid molecules and provided as pharmaceuticals for achieving gene therapy. Nucleic acid molecules encoding opsins that can be used in this embodiment include, for example, nucleic acid molecules that encode rod opsin (rhodopsin) and cone opsin (e.g., blue opsin, green opsin, and red opsin), and the like. In addition, the nucleic acid molecules encoding opsins herein include, but are not limited to, nucleic acid molecules encoding melanopsin, encephalopsin, panopsin, and peropsin. In one embodiment, the nucleic acid molecules used in the present disclosure may be nucleic acid molecules encoding microbial opsins, animal opsins, and the like. More specifically, the animal opsin can also be a nucleic acid molecule encoding a vertebrate visual opsin, a vertebrate non-visual opsin, an invertebrate opsin, and the like. The nucleic acid molecules that can be used in the present disclosure can be nucleic acid molecules that encode bistable opsins, such as vertebrate non-visual opsins and invertebrate opsins. Even if the nucleic acid molecules encoding the opsins used in the present disclosure do not strictly correspond to microbial opsins, vertebrate non-visual opsins, or invertebrate opsins, such nucleic acid molecules can be advantageously used as long as they are such nucleic acid molecules that encode functional equivalents having equivalent functions to these types of opsins that are advantageously used.

The nucleic acid molecule used in the present disclosure may be, for example, a nucleic acid molecule encoding a chimeric rhodopsin of ion-transporting receptor rhodopsin and G protein-coupled receptor rhodopsin. The nucleic acid molecule encoding the ion-transporting receptor rhodopsin that can be used herein is preferably of microbial origin, and those that can be repeatedly used are utilized. Furthermore, when an animal-derived, preferably mammalian-derived nucleic acid molecule is used as the nucleic acid molecule encoding the G protein-coupled receptor rhodopsin, high activity via an endogenous G protein can be obtained, while the encoded protein retains its repetitive activation function.

The nucleic acid molecule for use in the present disclosure may be a nucleic acid molecule encoding an ion pump receptor rhodopsin, an ion channel receptor rhodopsin, or the like.

The nucleic acid molecule for use in the present disclosure may be, for example, a nucleic acid molecule encoding the ion-transporting receptor rhodopsin of algal or microbial origin. In a preferred embodiment, the nucleic acid molecule of the present disclosure is preferably of the genus *Gloeobacter* or *Guillardia,* among others. In particular, the nucleic acid molecule of the present disclosure is preferably of the *Gloeobacter violaceus,* among the microorganisms pertaining to the genus *Gloeobacter,* and *Guillardia theta* of the genus *Guillardia.* It is also preferable to combine and utilize a nucleic acid molecule encoding the rhodopsin (e.g., SEQ ID NO: 13) of microorganisms pertaining to the genus *Gloeobacter,* or a nucleic acid molecule encoding the rhodopsin (e.g., SEQ ID NO: 15) of microorganisms pertaining to the genus *Guillardia,* with a G protein-coupled receptor rhodopsin of mammalian origin, and preferably a G protein-coupled receptor rhodopsin of primates of a nucleic acid molecule encoding the rhodopsin of Artiodactyla, such as cow (e.g., SEQ ID NO: 11) or a nucleic acid molecule encoding the rhodopsin of humans (e.g., SEQ ID NO: 9) and the like, among the G protein-coupled receptor rhodopsins of animal origin. In addition nucleic acid molecules encoding the rhodopsin of the genus *Gloeobacter,* as well as the algae of the genus *Guillardia* etc., are also preferable in terms of having an important property of being expressed well in E. *coli,* which are eubacteria, and human cells, which are eukaryotes.

In addition, sequences of nucleic acid molecules that can be used in the present disclosure can include, for example: sequences of nucleic acid molecules for non-visual opsins such as melanopsin (OPN4 opsin 4 [*Homo sapiens* (human)], Gene ID: 94233), and parapinopsin (parapinopsin [*Pangasianodon hypophthalmus* (striped catfish)], Gene ID: 113533339); sequences of nucleic acid molecules for invertebrate opsins such as tachodopsin (LOC106878312 rhodopsin [*Octopus bimaculoides*]*,* GeneID: 106878312), icarodopsin (https://www.ncbi.nlm.nih.gov/protein/P31356.2?report=genba nk&log$=protalign&blast_rank=1&RID=P4MU7584014), *Drosophila melanogaster* rhodopsin (Rh2Rhodopsin 2 [*Drosophila melanogaster* (fruit fly)], Gene ID: 42261), and Jumping spider peropsin (Accession No. AB525082, https://www.ncbi.nlm.nih.gov/nuccore/AB525082), ACCESSION of WP_011140202 (https://www.ncbi.nlm.nih.gov/protein/WP_011140202.1? report=genpept); or sequences of nucleic acid molecules for GeneID such as 5953595, 1448071, 4193772, 30923462, 14652027, 20310963, 3852586, 14209775, 40751430, 55980348, 8412230, 56887267, 57560395, 56080414, 9621525, 4937479, 5727173, 27418605, 27674957, 42178991, 5724518, 105202290, 286789, 107361958, 107442358, 107364443, 110460528, 110452210, 110454859, 110451845, 42367, 105211548, 100492717, 20132, 107311309, 221391, 113533339, and 100331083. As for these nucleic acid molecules or proteins encoded thereby, those skilled in the art can approximately utilize preferable ones according to the purpose of various applications of the present disclosure.

The method for isolating and method for purifying the chimeric protein are not particularly limited, and examples thereof include known methods such as a method utilizing solubility, a method utilizing difference in molecular weight, and a method utilizing charge (see, for example, Japanese Laid-Open Publication No. 11-29599).

In one embodiment, the nucleic acid molecule (polynucleotide) encoding a chimeric rhodopsin of the present disclosure can be any of the following polynucleotides (note that: if the nucleic acid molecule is RNA, T means U in the nucleic acid sequence):
(A) a polynucleotide comprising the nucleic acid sequence set forth in SEQ ID NO: 1 or 3 or a fragment thereof;
(B) a polynucleotide comprising a nucleic acid sequence comprising one or more nucleotides or one or several nucleotide substitutions, additions, deletions and/or a combination thereof in the nucleic acid sequence set forth in (A);
(C) a polynucleotide comprising a polynucleotide comprising a nucleic acid sequence having at least 70%, at least 80%, at least 90%, or at least 95% or more sequence identity with the nucleic acid sequence set forth in (A) or (B);
(D) a polynucleotide comprising a nucleic acid sequence of a polynucleotide that hybridizes under stringent conditions to a polynucleotide comprising a nucleic acid sequence of any one of (A)-(C) or a complementary sequence thereof;
(E) a polynucleotide comprising a nucleic acid sequence of an allelic variant of the polynucleotide of any one of (A)-(D) ;
(F) a polynucleotide comprising a nucleic acid sequence encoding a polypeptide comprising the amino acid sequence set forth in SEQ ID NO: 2 or 4;
(G) a polynucleotide encoding a polypeptide comprising an amino acid sequence comprising one or more amino acids or one or several amino acid substitutions, additions, deletions and/or a combination thereof in the amino acid sequence of (F);
(H) a polynucleotide encoding a polypeptide having at least 70%, at least 80%, at least 90%, or at least 95% or more sequence identity with the amino acid sequence set forth in (F) or (G); or
(I) a polynucleotide encoding a polypeptide comprising a fragment of an amino acid sequence set forth in (F) to (H), and with regard to such a polynucleotide, the chimeric protein encoded by the polynucleotide has biological activity.

As used herein, representative examples of "biological activity" include functions of G protein-coupled receptors possessed by the loop thereof (e.g., membrane translocation efficiency), and in addition to the above, include functions capable of exhibiting effects of: treating, preventing, or suppressing the progress of diseases associated with endoplasmic reticulum transport; or treating, preventing, or suppressing the progress of diseases associated with all-trans-retinal. Biological activities for loops can include, but are not limited to, functions such as conformational compatibility and membrane translocation efficiency. Alternatively, the functions of the loop may be assessed by the functions of the entire integrated protein (which is the rhodopsin herein).

In a specific embodiment, the chimeric rhodopsin (polypeptide) of the present disclosure may be any of the polypeptides comprising the amino acid sequences below:
(a) a polypeptide comprising an amino acid sequence set forth in SEQ ID NO: 2 or 4 or a fragment thereof;
(b) a polypeptide comprising one or more amino acids or one or several amino acid substitutions, additions, deletions and/or a combination thereof, in the amino acid sequence of (a) ;
(c) a polypeptide having at least 70%, at least 80%, at least 90%, or at least 95% or more sequence identity with the amino acid sequence set forth in (a) or (b);
(d) a polypeptide encoded by a polynucleotide comprising an amino acid sequence set forth in SEQ ID NO: 1 or 3;
(e) a polypeptide encoded by a polynucleotide comprising one or more nucleotides or one or several nucleotide substitutions, additions, deletions and/or a combination thereof, in the amino acid sequence of (d);
(f) a polypeptide encoded by a polynucleotide having at least 70%, at least 80%, at least 90%, or at least 95% or more sequence identity with the nucleic acid sequence set forth in (d) or (e);
(g) a polypeptide encoded by a polynucleotide that hybridizes with a polynucleotide comprising a nucleic acid sequence set forth in any one of (d) to (f) or a complementary sequence thereof, under stringent conditions;
(h) a polypeptide encoded by an allelic mutant of a nucleic acid sequence of any one of (d) to (g); or
(i) a polypeptide comprising a fragment of an amino acid sequence set forth in (a) to (h),

and with regard to such a polypeptide, the polypeptide has biological activity; or
the chimeric protein of the present disclosure may include an amino acid sequence encoded by any of the following polynucleotides:
   (aa) a polynucleotide having a nucleic acid sequence encoding an amino acid sequence set forth in SEQ ID NO: 2 or 4, or a polynucleotide having an amino acid sequence set forth in SEQ ID NO: 1 or 3;
   (bb) a polynucleotide having a nucleic acid sequence that can hybridize under stringent conditions with a polynucleotide having a nucleic acid sequence encoding an amino acid sequence set forth in SEQ ID NO: 2 or 4, or with a polynucleotide complementary to an amino acid sequence set forth in SEQ ID NO: 1 or 3;
   (cc) a polynucleotide having a polynucleotide encoding an amino acid sequence in which one or more amino acids are substituted, deleted and/or added in the amino acid sequence set forth in SEQ ID NO: 2 or 4;
   (dd) a polynucleotide comprising a polynucleotide encoding an amino acid sequence having 90% or more homology with an amino acid sequence set forth in SEQ ID NO: 2 or, 4,
where the polynucleotide has biological activity; or
   (aaa) a polynucleotide having a nucleic acid sequence set forth in SEQ ID NO: 1 or 3 or a fragment thereof;
   (bbb) a polynucleotide comprising a nucleic acid sequence having at least 70%, at least 80%, at least 90%, or at least 95% identity to the nucleic acid sequence set forth in (aaa);
   (ccc) a polynucleotide comprising a nucleic acid sequence with one or more nucleotides substituted, added and/or deleted with respect to the nucleic acid sequence set forth in (aaa) or (bbb); and
   (ddd) a polynucleotide that hybridizes to a nucleic acid sequence set forth in any of (aaa) to (ccc) under stringent conditions,
and with regard to such a polynucleotide, the chimeric protein has biological activity.

In a particular embodiment, the nucleic acid encoding the chimeric rhodopsin of the present disclosure may be a nucleic acid that shares at least six triplets, or even more, in common with the nucleic acid sequence set forth in SEQ ID NO: 1 or 3. In another embodiment, the nucleic acid construct of the chimeric rhodopsin of the present disclosure may include a nucleic acid sequence sharing at least one of the triplets encoding amino acids 6, 9-13, 15, 16, 18-22, 27-29, 31-36, 39, 40, 43, 45, 48, 50, 51, 53-55, 58, 59, 61, 65-73, 75-84, 86, 88, 89, 93, 97, 98, 100, 101, 104, 106-108, 110, 112, 114, 115, 122, 123, 125, 128, 131, 133, 139, 143, 145, 146, 155, 157, 162, 165, 167, 169-171, 174, 176, 179, 182, 183, 186-189, 193-198, 204, 205, 207, 209, 212, 215, 216, 218-220, 224, 225, 227, 228, 230, 231, 233-235, 238, 240, 242, 243, 246, 247, 249, 251, 253-255, 257-259, 261-264, 266-270, 272, 273, 275, 276, 279, 281-287, 289-291, 296-299, 302-305, 307-316, 318, 319, and 321-330 in common with the nucleic acid sequence set forth in SEQ ID NO: 1 or 3, among the nucleic acid sequences encoding the same amino acids as SEQ ID NO: 2 or 4.

The nucleic acid molecule (polynucleotide) encoding the second loop on the cytoplasmic side of the G protein-coupled receptor rhodopsin described above may be any of the following (note that, when the nucleic acid molecule is RNA, T means U in the nucleic acid sequence):
(A) a polynucleotide comprising a nucleic acid sequence set forth in SEQ ID NO: 17 or 18 or a fragment thereof;
(B) a polynucleotide comprising a nucleic acid sequence comprising one or more nucleotides or one or several nucleotide substitutions, additions, deletions and/or a combination thereof, in the nucleic acid sequence set forth in (A);
(C) a polynucleotide comprising a polynucleotide comprising a nucleic acid sequence having at least 70%, at least 80%, at least 90%, or at least 95% or more sequence identity with a nucleic acid sequence set forth in (A) or (B);
(D) a polynucleotide comprising a nucleic acid sequence of polynucleotide that hybridizes under stringent conditions with a polynucleotide comprising a nucleic acid sequence set forth in any one of (A) to (C) or a complementary sequence thereof;
(E) a polynucleotide comprising a nucleic acid sequence of an allelic mutant of a polynucleotide of any one of (A) to (D) ;
(F) a polynucleotide comprising a nucleic acid sequence encoding a polypeptide comprising an amino acid sequence set forth in SEQ ID NO: 19 or 25;
(G) a polynucleotide encoding a polypeptide comprising an amino acid sequence comprising one or more amino acids or one or several amino acid substitutions, additions, deletions and/or a combination thereof, in the amino acid sequence of (F);
(H) a polynucleotide encoding a polypeptide having at least 70%, at least 80%, at least 90%, or at least 95% or more sequence identity with a nucleic acid sequence set forth in (F) or (G); or
(I) a polynucleotide encoding a polypeptide comprising a fragment of a nucleic acid sequence set forth in (F) to (H).

In a particular embodiment, the nucleic acid sequence encoding the second loop on the cytoplasmic side of the G protein-coupled receptor rhodopsin described above is preferably a nucleic acid sequence sharing at least two triplets in common with the nucleic acid sequence set forth in SEQ ID NO: 17.

Alternatively, the second loop on the cytoplasm side of the G protein-coupled receptor rhodopsin described above is preferably a loop having an amino acid sequence encoded by any of the nucleic acids described below:
(i) a polynucleotide having a nucleic acid sequence encoding an amino acid sequence set forth in SEQ ID NO: 19 or 25;
(ii) a polynucleotide having a nucleic acid sequence that can hybridize under stringent conditions with a polynucleotide complementary to a nucleic acid sequence encoding an amino acid sequence set forth in SEQ ID NO: 19 or 25;
(iii) a polynucleotide having a polynucleotide encoding an amino acid sequence in which one or more amino acids are substituted, deleted and/or added in an amino acid sequence set forth in SEQ ID NO: 19 or 25; and
(iv) a polynucleotide comprising a polynucleotide encoding an amino acid sequence having at least 70%, at least 80%, at least 90%, or at least 95% or more homology with an amino acid sequence set forth in SEQ ID NO: 19 or 25, or
the nucleic acid encoding the second loop on the cytoplasm side of the G protein-coupled receptor rhodopsin is preferably any of the below.

In a particular embodiment, the nucleic acid sequence encoding the third loop on the cytoplasmic side of the G protein-coupled receptor rhodopsin described above is preferably a nucleic acid sequence sharing at least one triplet in common with the nucleic acid sequence set forth in SEQ ID NO: 20 or 21.
(i) a polynucleotide having a nucleic acid sequence encoding an amino acid sequence set forth in SEQ ID NO: 19 or 25;
(ii) a polynucleotide having a nucleic acid sequence that can hybridize under stringent conditions with a polynucleotide complementary to a nucleic acid sequence encoding an amino acid sequence set forth in SEQ ID NO: 19 or 25;
(iii) a polynucleotide having a polynucleotide encoding an amino acid sequence in which one or more amino acids are substituted, deleted and/or added in an amino acid sequence set forth in SEQ ID NO: 19 or 25;
(iv) a polynucleotide comprising a polynucleotide encoding an amino acid sequence having 90% or more homology with an amino acid sequence set forth in SEQ ID NO: 19 or 25;
(x) a polynucleotide having a nucleic acid sequence set forth in SEQ ID NO: 20 or 21 or a fragment thereof;
(y) a polynucleotide comprising a nucleic acid sequence having at least 70%, at least 80%, at least 90%, or at least 95% or more identity to the nucleic acid sequence set forth in (x);
(z) a polynucleotide comprising a nucleic acid sequence with one or more nucleotides substituted, added and/or deleted with respect to a nucleic acid sequence set forth in (x) or (y); and
(w) a polynucleotide that hybridizes to a nucleic acid sequence set forth in any of (x) to (z) under stringent conditions, and
with regard to such a polynucleotide, the loop has biological activity.

The nucleic acid sequence (polynucleotide) encoding the third loop on the cytoplasmic side of the G protein-coupled receptor rhodopsin described above may be any of the following (note that, when the nucleic acid molecule is RNA, T means U in the nucleic acid sequence):
(A) a polynucleotide comprising a nucleic acid sequence set forth in SEQ ID NO: 20 or 21 or a fragment thereof;
(B) a polynucleotide comprising a nucleic acid sequence comprising one or more nucleotides or one or several nucleotide substitutions, additions, deletions and/or a combination thereof, in the nucleic acid sequence set forth in (A);
(C) a polynucleotide comprising a polynucleotide comprising a nucleic acid sequence having at least 70%, at least 80%, at least 90%, or at least 95% or more sequence identity with a nucleic acid sequence set forth in (A) or (B);
(D) a polynucleotide comprising a nucleic acid sequence of polynucleotide that hybridizes under stringent conditions with a polynucleotide comprising a nucleic acid sequence set forth in any one of (A) to (C) or a complementary sequence thereof;
(E) a polynucleotide comprising a nucleic acid sequence of an allelic mutant of a polynucleotide of any one of (A) to (D) ;
(F) a polynucleotide comprising a nucleic acid sequence encoding a polypeptide comprising the amino acid sequence set forth in SEQ ID NO: 22;
(G) a polynucleotide encoding a polypeptide comprising an amino acid sequence comprising one or more amino acids or one or several amino acid substitutions, additions, deletions and/or a combination thereof, in the amino acid sequence of (F);
(H) a polynucleotide encoding a polypeptide having at least 70%, at least 80%, at least 90%, or at least 95% or more sequence identity with a nucleic acid sequence set forth in (F) or (G); or
(I) a polynucleotide encoding a polypeptide comprising a fragment of a nucleic acid sequence set forth in (F) to (H).

The third loop on the cytoplasm side of the G protein-coupled receptor rhodopsin described above is preferably a loop having an amino acid sequence encoded by any of the following nucleic acids:
(l) a polynucleotide having a nucleic acid sequence encoding the amino acid sequence set forth in SEQ ID NO: 22;
(k) a polynucleotide having a nucleic acid sequence that can hybridize under stringent conditions with a polynucleotide complementary to a nucleic acid sequence encoding the amino acid sequence set forth in SEQ ID NO: 22;
(m) a polynucleotide having a polynucleotide encoding an amino acid sequence in which one or more amino acids are substituted, deleted and/or added in the amino acid sequence set forth in SEQ ID NO: 22; and
(n) a polynucleotide comprising a polynucleotide encoding an amino acid sequence having at least 70%, at least 80%, at least 90%, or at least 95% or more homology with the amino acid sequence set forth in SEQ ID NO: 22.

Alternatively, the nucleic acid encoding the third loop on the cytoplasm side of the G protein-coupled receptor rhodopsin is preferably any of the following:
(l) a polynucleotide having a nucleic acid sequence encoding the amino acid sequence set forth in SEQ ID NO: 22;
(k) a polynucleotide having a nucleic acid sequence that can hybridize under stringent conditions with a polynucleotide complementary to a nucleic acid sequence encoding the amino acid sequence set forth in SEQ ID NO: 22;
(m) a polynucleotide having a polynucleotide encoding an amino acid sequence in which one or more amino acids are substituted, deleted and/or added in the amino acid sequence set forth in SEQ ID NO: 22;
(n) a polynucleotide comprising a polynucleotide encoding an amino acid sequence having at least 70%, at least 80%, at least 90%, or at least 95% or more homology with the amino acid sequence set forth in SEQ ID NO: 22;
(xx) a polynucleotide having the nucleic acid sequence set forth in SEQ ID NO: 20 or a fragment thereof;
(yy) a polynucleotide comprising a nucleic acid sequence having at least 70%, at least 80%, at least 90% or at least 95% identity to the nucleic acid sequence set forth in (xx) ;
(zz) a polynucleotide comprising a nucleic acid sequence with one or more nucleotides substituted, added and/or deleted with respect to the nucleic acid sequence set forth in (xx) or (yy); or
(ww) a polynucleotide that hybridizes to a nucleic acid sequence set forth in any of (xx) to (zz) under stringent conditions, and
with regard to such a polynucleotide, the loop has biological activity.

In one embodiment, the nucleic acid molecule (polynucleotide) encoding a chimeric rhodopsin of the present disclosure can be any of the following polynucleotides (note that: if the nucleic acid molecule is RNA, T means U in the nucleic acid sequence):
(A) a polynucleotide comprising the nucleic acid sequence set forth in SEQ ID NO: 7 or a fragment thereof;
(B) a polynucleotide comprising a nucleic acid sequence comprising one or more nucleotides or one or several nucleotide substitutions, additions, deletions and/or a combination thereof in the nucleic acid sequence set forth in (A);
(C) a polynucleotide comprising a polynucleotide comprising a nucleic acid sequence having at least 70%, at least 80%, at least 90%, or at least 95% or more sequence identity with the nucleic acid sequence set forth in (A) or (B);
(D) a polynucleotide comprising a nucleic acid sequence of a polynucleotide that hybridizes under stringent conditions to a polynucleotide comprising a nucleic acid sequence of any one of (A)-(C) or a complementary sequence thereof;
(E) a polynucleotide comprising a nucleic acid sequence of an allelic variant of the polynucleotide of any one of (A)-(D) ;
(F) a polynucleotide comprising a nucleic acid sequence encoding a polypeptide comprising the amino acid sequence set forth in SEQ ID NO: 8;
(G) a polynucleotide encoding a polypeptide comprising an amino acid sequence comprising one or more amino acids or one or several amino acid substitutions, additions, deletions and/or a combination thereof in the amino acid sequence of (F);
(H) a polynucleotide encoding a polypeptide having at least 70%, at least 80%, at least 90%, or at least 95% or more sequence identity with the amino acid sequence set forth in (F) or (G); or
(I) a polynucleotide encoding a polypeptide comprising a fragment of an amino acid sequence set forth in (F) to (H), and with regard to such a polynucleotide, the chimeric protein encoded by the polynucleotide has biological activity.

In a particular embodiment, the nucleic acid of the chimeric rhodopsin of the present disclosure may be a nucleic acid sequence sharing at least fourteen or more triplets in common with the nucleic acid sequence set forth in SEQ ID NO: 7. In another embodiment, the nucleic acid construct of the present disclosure may include a nucleic acid sequence sharing at least one of the triplets encoding amino acids 1, 2, 4-9, 11-17, 21, 22, 27-30, 33, 34, 36-41, 43, 45, 48, 49, 51, 54, 56-58, 60, 63, 65, 68, 70, 71-75, 77-78, 81, 83, 84, 86, 89, 90, 92, 93, 95, 97-99, 102, 103, 111, 113, 114, 123, 125, 130, 131-137, 139, 142, 143, 146, 148-153, 156, 160, 161, 165, 167, 168, 170, 171, 174-176, 180, 182, 183, 187, 188, 190, 191, 196, 197, 199, 200, 202, 204, 208, 212-214, 217, 219, 226, 229, 232, 236-238, 240, 242, 243, 247, 248, 251, 252, 258, 263-265, 267, 269, 271, 272, 274, 276-280, 282-284, 289, 290, 291, 294, 297-299, 302, 304, 307 and 310 in common with the nucleic acid sequence set forth in SEQ ID NO: 7, among the nucleic acid sequences encoding the same amino acids as SEQ ID NO: 7.

In a specific embodiment, the chimeric rhodopsin (polypeptide) of the present disclosure may be any of the polypeptides comprising the amino acid sequences below:
(a) a polypeptide comprising an amino acid sequence set forth in SEQ ID NO: 8 or a fragment thereof;
(b) a polypeptide comprising an amino acid sequence comprising one or more amino acids or one or several amino acid substitutions, additions, deletions and/or a combination thereof in the amino acid sequence of (a);
(c) a polypeptide having at least 70%, at least 80%, at least 90%, or at least 95% or more sequence identity with a nucleic acid sequence set forth in (a) or (b);
(d) a polypeptide encoded by a polynucleotide comprising the amino acid sequence set forth in SEQ ID NO: 7;
(e) a polypeptide encoded by a polynucleotide comprising one or more nucleotides or one or several nucleotide substitutions, additions, deletions and/or a combination thereof in the amino acid sequence set forth in (d);
(f) a polypeptide encoded by a polynucleotide having at least 70%, at least 80%, at least 90%, or at least 95% or more sequence identity with a nucleic acid sequence set forth in (d) or (e);
(g) a polypeptide encoded by polynucleotide that hybridizes under stringent conditions with a polynucleotide comprising a nucleic acid sequence set forth in any one of (d) to (f) or a complementary sequence thereof;
(h) a polypeptide encoded by an allelic mutant of a nucleic acid sequence of any one of (d) to (g); or
(i) a polypeptide comprising a fragment of an amino acid sequence set forth in (a) to (h), and

with regard to such a polypeptide, the polypeptide has biological activity; or
the chimeric protein of the present disclosure may comprise an amino acid sequence encoded by any of the following nucleic acids:
   (aa) a polynucleotide having a nucleic acid sequence encoding the amino acid sequence set forth in SEQ ID NO: 8 or a polynucleotide having the nucleic acid sequence set forth in SEQ ID NO: 7;
   (bb) a polynucleotide having a nucleic acid sequence that can hybridize under stringent conditions with a polynucleotide having a nucleic acid sequence encoding the amino acid sequence set forth in SEQ ID NO: 8 or a polynucleotide complementary to the nucleic acid sequence set forth in SEQ ID NO: 7;
   (cc) a polynucleotide having a polynucleotide encoding an amino acid sequence in which one or more amino acids are substituted, deleted and/or added in the amino acid sequence set forth in SEQ ID NO: 8;
   (dd) a polynucleotide comprising a polynucleotide encoding an amino acid sequence having 90% or more homology with the amino acid sequence set forth in SEQ ID NO: 8, and
with regard to such a polynucleotide, the polynucleotide has biological activity; or
   (aaa) a polynucleotide comprising the nucleic acid sequence set forth in SEQ ID NO: 7 or a fragment thereof;
   (bbb) a polynucleotide comprising a nucleic acid sequence having at least 70%, at least 80%, at least 90%, or at least 95% identity to the nucleic acid sequence set forth in (aaa);
   (ccc) a polynucleotide comprising a nucleic acid sequence with one or more nucleotides substituted, added and/or deleted with respect to the nucleic acid sequence set forth in (aaa) or (bbb); and
   (ddd) a polynucleotide that hybridizes to a nucleic acid sequence set forth in any of (aaa) to (ccc) under stringent conditions, and
with regard to such a polynucleotide, the chimeric protein has biological activity.

### (Prevention or Suppression of Progress of Disease, Disorder or Symptom associated with Endoplasmic Reticulum Stress)

In another aspect, the present disclosure provides a novel pharmaceutical, composition, compound or method for preventing, or suppressing the progress of, a disease, disorder or symptom associated with an endoplasmic reticulum stress. The pharmaceutical, composition, compound or method of the present disclosure can, in one embodiment, utilize a retinal modulating agent. Without wishing to be bound by theory, the retinal modulating agent of the present disclosure can modulate the state of retinal, including the *in vivo* amount, concentration, or cis/trans ratio (ratio of 11-cis-retinal to all-trans-retinal) of retinal, which is believed to increase the ratio of 11-cis-retinal and ameliorate a transport disorder of rhodopsin from the endoplasmic reticulum. Furthermore, due to such a mechanism, it is believed that the progress of retinal degeneration is suppressed by the retinal modulating agent of the present disclosure.

In addition, it is believed that the retinal modulating agent of the present disclosure can treat or prevent a disease, disorder or symptom associated with an endoplasmic reticulum stress by modulating the retinal status. In the present disclosure, the endoplasmic reticulum stress ameliorating effect by the retinal modulating agent is shown in the Examples. Prior to the present disclosure, no association had been found between the modulation of the amount, concentration, or cis/trans ratio of retinal and the endoplasmic reticulum stress ameliorating effect. As such, the results of the present disclosure were surprising.

Therefore, in one aspect, the present disclosure can provide a composition for use in treating, preventing, or suppressing the progress of, a disease, disorder or symptom associated with an endoplasmic reticulum stress, the composition comprising a retinal modulating agent. In one aspect, the present disclosure provides a method of treating, preventing, or suppressing the progress of, a disease, disorder or symptom associated with an endoplasmic reticulum stress in a subject, the method comprising the step of administering an effective amount of a composition of the present disclosure to the subject. In one aspect, the present disclosure provides use of a retinal modulating agent for manufacture of a composition for use in treating, preventing, or suppressing the progress of, a disease, disorder or symptom associated with an endoplasmic reticulum stress in a subject. In other aspects, the present disclosure can provide a composition for use in treating or preventing a disease, disorder or symptom associated with an endoplasmic reticulum stress, the composition comprising an absolute retinal modulating agent. In one embodiment, examples of the "disease, disorder or symptom associated with an endoplasmic reticulum stress" include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

In other aspects, the present disclosure can provide a composition, compound, pharmaceutical, or method for reducing or eliminating an endoplasmic reticulum stress, comprising an absolute retinal modulating agent. The present disclosure can provide a method for reducing or eliminating an endoplasmic reticulum stress in a subject, the method comprising the step of administering an absolute retinal modulating agent to the subject. The present disclosure can further provide use of an absolute retinal modulating agent for manufacture of a composition or pharmaceutical for reducing or eliminating an endoplasmic reticulum stress. Without wishing to be bound by theory, the absolute retinal modulating agent has been unexpectedly found to be able to reduce or eliminate an endoplasmic reticulum stress and to be highly effective. As a related matter, since the absolute retinal modulating agent of the present disclosure is capable of reducing or eliminating an endoplasmic reticulum stress, it is believed that the absolute retinal modulating agent is capable of reducing or eliminating a disease, disorder or symptom associated with an associated endoplasmic reticulum stress.

In one embodiment, examples of the "disease, disorder or symptom associated with an endoplasmic reticulum stress" include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

In other aspects of the present disclosure, the composition of the present disclosure can also be provided as a kit with a companion diagnostic agent combined therein. For example, a composition comprising an active ingredient, such as the retinal modulating agent, absolute retinal modulating agent, or opsins or derivatives thereof, of the present disclosure can exert a therapeutic effect when administered to a subject when the subject has a disease or disorder such as a vesicle transport disorder, or when such a disease or disorder is expected to occur. Therefore, in combination with a companion diagnostic agent for a prior diagnosis of a vesicle transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Light-Absorbing Substances and Endoplasmic Reticulum Stress)

In another aspect, the present disclosure provides still another pharmaceutical, composition, compound or method for preventing, or suppressing the progress of, a disease, disorder or symptom associated with an endoplasmic reticulum stress. The pharmaceutical, composition, compound or method of the present disclosure can, in one embodiment, utilize a light absorbing substance. Without wishing to be bound by theory, since the light absorbing substance of the present disclosure can receive light in place of endogenous rhodopsin, the light absorbing substance takes over the photoreception by endogenous rhodopsin, where it is believed that the endoplasmic reticulum stress that can occur with the photoreception is reduced or eliminated. No agent with such a mechanism has been provided in the past, and it can be said that such an agent is provided for the first time in the present disclosure. It is believed that the light absorbing substance of the present disclosure ameliorates a transport disorder of rhodopsin from the endoplasmic reticulum. It is also believed that the light absorbing substance of the present disclosure suppresses the progress of retinal degeneration by taking over the photoreception by endogenous rhodopsin.

It is believed that the light absorbing substance of the present disclosure can treat or prevent a disease, disorder or symptom associated with an endoplasmic reticulum stress. In the present disclosure, the endoplasmic reticulum stress ameliorating effect by the light absorbing substance is shown in the Examples, and the results of the present disclosure were surprising.

Therefore, in one aspect, the present disclosure can provide a composition for use in treating, preventing, or suppressing the progress of, a disease, disorder or symptom associated with an endoplasmic reticulum stress, the composition comprising a light absorbing substance. In one aspect, the present disclosure provides a method of treating, preventing, or suppressing the progress of, a disease, disorder or symptom associated with an endoplasmic reticulum stress in a subject, the method comprising the step of administering an effective amount of a composition of the present disclosure to the subject. In one aspect, the present disclosure provides use of a light absorbing substance for manufacture of a composition for use in treating, preventing, or suppressing the progress of, a disease, disorder or symptom associated with an endoplasmic reticulum stress in a subject. In one embodiment, examples of the "disease, disorder or symptom associated with an endoplasmic reticulum stress" include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom. Preferably, a disease, disorder or symptom associated with photoreception may be a "disease, disorder or symptom associated with an endoplasmic reticulum stress".

In a preferred embodiment of the present disclosure, the light absorbing substance is biocompatible. The light absorbing substance can also include an autologous light absorbing substance. Alternatively, the light absorbing substance is preferably a non-intrinsic light absorbing substance. The light absorbing substance used in the present disclosure is preferably, but not limited to, a biocompatible light absorbing substance. The light absorbing substance used in the present disclosure may be, but is not limited to, a light absorbing substance that has neural activity-signaling activity. The light absorbing substance used in the present disclosure is preferably, but not limited to, a biocompatible light absorbing substance.

In one embodiment, examples of the "disease, disorder or symptom associated with an endoplasmic reticulum stress" include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

In other aspects of the present disclosure, the composition of the present disclosure can also be provided as a kit with a companion diagnostic agent combined therein. For example, a composition comprising an active ingredient, such as the light absorbing substance of the present disclosure, can exert a therapeutic effect when administered to a subject when the subject has a disease or disorder such as a vesicle transport disorder, or when such a disease or disorder is expected to occur. Therefore, in combination with a companion diagnostic agent for a prior diagnosis of a vesicle transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Neural Activity-Signaling Activity and Endoplasmic Reticulum Stress)

In another aspect, the present disclosure provides a novel pharmaceutical, composition, compound or method for preventing, or suppressing the progress of, a disease, disorder or symptom associated with an endoplasmic reticulum stress. The pharmaceutical, composition, compound or method of the present disclosure can, in one embodiment, utilize a neural activity signaling agent or device. Without wishing to be bound by theory, the neural activity signaling agent or device has been unexpectedly found to ameliorate an endoplasmic reticulum stress by transmitting nerve activity signals, including electrical stimulation, to nerves on the side closer to the center. It is believed that the neural activity signaling agent or device of the present disclosure ameliorates an endoplasmic reticulum stress, thereby ameliorating a transport disorder of rhodopsin from the endoplasmic reticulum. Furthermore, due to such a mechanism, it is believed that the progress of retinal degeneration is suppressed by the neural activity signaling agent or device of the present disclosure.

Furthermore, by modulating the retinal status, the neural activity signaling agent or device of the present disclosure is believed to be able to treat or prevent a disease, disorder or symptom associated with the endoplasmic reticulum stress. In the present disclosure, the endoplasmic reticulum stress ameliorating effect by the neural activity signaling agent or device is shown in the Examples, and the results of the present disclosure were surprising.

Therefore, in one aspect, the present disclosure can provide a composition for use in treating, preventing, or suppressing the progress of, a disease, disorder or symptom associated with an endoplasmic reticulum stress, the composition comprising a neural activity signaling agent or device. In one aspect, the present disclosure provides a method of treating, preventing, or suppressing the progress of, a disease, disorder or symptom associated with an endoplasmic reticulum stress in a subject, the method comprising the step of administering an effective amount of a composition of the present disclosure to the subject. In one aspect, the present disclosure provides use of a neural activity signaling agent or device for manufacture of a composition for use in treating, preventing, or suppressing the progress of, a disease, disorder or symptom associated with an endoplasmic reticulum stress in a subject. In one embodiment, examples of the "disease, disorder or symptom associated with an endoplasmic reticulum stress" include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

In a preferred embodiment of the present disclosure, the neural activity signaling agent or device is biocompatible. The neural activity signaling agent or device can also include an autologous, neural activity signaling agent or device. Alternatively, the neural activity signaling agent or device is preferably a non-intrinsic, neural activity signaling agent or device. The neural activity signaling agent or device used in the present disclosure is preferably, but not limited to, a biocompatible, neural activity signaling agent or device. The neural activity signaling agent or device used in the present disclosure may be, but is not limited to, a neural activity signaling agent or device that has a light absorption property. The neural activity signaling agent or device used in the present disclosure is preferably, but not limited to, a biocompatible neural activity signaling agent or device.

In one embodiment, examples of the "disease, disorder or symptom associated with an endoplasmic reticulum stress" include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

In other aspects of the present disclosure, the composition of the present disclosure can also be provided as a kit with a companion diagnostic agent combined therein. For example, a composition comprising an active ingredient, such as the neural activity signaling agent or device of the present disclosure, can exert a therapeutic effect when administered to a subject when the subject has a disease or disorder such as a vesicle transport disorder, or when such a disease or disorder is expected to occur. Therefore, in combination with a companion diagnostic agent for a prior diagnosis of a vesicle transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Opsins and Endoplasmic Reticulum Stress)

In another aspect, as novel use of opsins or derivatives thereof or nucleic acid molecules encoding them, the present disclosure provides a pharmaceutical, composition, compound or method, which comprises the opsins or derivatives thereof or nucleic acid molecules encoding them, for preventing, or suppressing the progress of a disease, disorder or symptom associated with an endoplasmic reticulum stress. Without wishing to be bound by theory, it is believed that the opsins or derivatives thereof of the present disclosure act, not on the retinal, but on the rhodopsin itself, and prevent the production of toxic metabolites such as all-trans-retinal, thereby ameliorating a transport disorder of rhodopsin from the endoplasmic reticulum.

Furthermore, the opsins or derivatives thereof of the present disclosure are believed to be able to treat or prevent a disease, disorder or symptom associated with the endoplasmic reticulum stress. In the present disclosure, the endoplasmic reticulum stress ameliorating effect by the opsins or derivatives thereof is shown in the Examples, and the results of the present disclosure were surprising.

Therefore, in one aspect, the present disclosure can provide a composition for use in treating, preventing, or suppressing the progress of, a disease, disorder or symptom associated with an endoplasmic reticulum stress, the composition comprising opsins or derivatives thereof or nucleic acid molecules encoding them. In one aspect, the present disclosure provides a method of treating, preventing, or suppressing the progress of, a disease, disorder or symptom associated with an endoplasmic reticulum stress in a subject, the method comprising the step of administering an effective amount of a composition of the present disclosure to the subject. In one aspect, the present disclosure provides use of opsins or derivatives thereof or nucleic acid molecules encoding them for manufacture of a composition for use in treating, preventing, or suppressing the progress of a disease, disorder or symptom associated with an endoplasmic reticulum stress in a subject. In one embodiment, examples of the "disease, disorder or symptom associated with an endoplasmic reticulum stress" include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

In a preferred embodiment, the opsins or derivatives thereof or nucleic acid molecules encoding them of the present disclosure may be autologous or non-autologous, and may also be of homologous or heterologous origin.

In one embodiment, examples of the "disease, disorder or symptom associated with an endoplasmic reticulum stress" include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

In other aspects of the present disclosure, the composition of the present disclosure can also be provided as a kit with a companion diagnostic agent combined therein. For example, a composition comprising an active ingredient, such as the opsins or derivatives thereof or nucleic acid molecules encoding them of the present disclosure, can exert a therapeutic effect when administered to a subject when the subject has a disease or disorder such as a vesicle transport disorder, or when such a disease or disorder is expected to occur. Therefore, in combination with a companion diagnostic agent for a prior diagnosis of a vesicle transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Prevention or Suppression of Progress of Disease, Disorder or Symptom Associated with Rhodopsin Transport Disorder)

In other aspects, the present disclosure can provide a pharmaceutical, compound, composition or method for ameliorating a rhodopsin transport disorder. The present disclosure can provide a method for ameliorating a rhodopsin transport disorder in a subject, the method comprising the step of administering a retinal modulating agent, an absolute retinal modulating agent, or opsins or derivatives thereof to the subject. The present disclosure can also provide use of a retinal modulating agent, an absolute retinal modulating agent, or opsins or derivatives thereof, for manufacture of a composition or pharmaceutical for ameliorating a rhodopsin transport disorder. The pharmaceutical, compound, composition or method of the present disclosure can be achieved with a retinal modulating agent. In this aspect, the present disclosure can provide a composition for use in ameliorating a rhodopsin transport disorder, the composition comprising an absolute retinal modulating agent. The retinal modulating agent, absolute retinal modulating agent, or opsins or derivatives thereof of the present disclosure can modulate the state of retinal, including the in vivo amount, concentration, or cis/trans ratio (ratio of 11-cis-retinal to all-trans-retinal) of retinal, thereby ameliorating a rhodopsin transport disorder. The rhodopsin transport disorder may be those in the endoplasmic reticulum as well as those at other sites.

Although the safety of ectopic expression of opsins such as Channelrhodopsin 2 has been previously reported, the present disclosure confirms the protective effect of the retinal modulating agent against retinal degeneration, as shown in the Examples. *In vitro* studies have shown that P23H opsin is misfolded and accumulates in the endoplasmic reticulum. Endoplasmic reticulum accumulation of P23H opsin may possibly induce an endoplasmic reticulum stress response and subsequent apoptosis. From the results of this experiment, the expression of coGHCR (chimeric rhodopsin) in the outer retinal layer is thought to induce suppression of endoplasmic reticulum stress and photoreceptor apoptosis leading to the above protective effect.

As used herein, the diseases, disorders or symptoms associated with the "rhodopsin transport disorder" can include, but are not limited to, retinal degenerative diseases, including retinitis pigmentosa and age-related macular degeneration. In fact, any disease, disorder or symptom in which the rhodopsin transport disorder is associated as a factor, is included in the disease, disorder or symptom.

In other aspects of the present disclosure, the composition of the present disclosure can also be provided as a kit with a companion diagnostic agent combined therein. For example, a composition comprising an active ingredient, such as the retinal modulating agent or the absolute retinal modulating agent of the present disclosure, can exert a therapeutic effect when administered to a subject when the subject has a disease or disorder such as a rhodopsin transport disorder, or when such a disease or disorder is expected to occur. Therefore, in combination with a companion diagnostic agent for a prior diagnosis of a rhodopsin transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Rhodopsin Transport Disorder and Light Absorbing Substance)

In other aspects, the present disclosure can provide a pharmaceutical, compound, composition or method for ameliorating a rhodopsin transport disorder. The present disclosure can provide a method for ameliorating a rhodopsin transport disorder in a subject, the method comprising the step of administering a light absorbing substance to the subject. The present disclosure can also provide use of a light absorbing substance for manufacture of a composition or pharmaceutical for ameliorating a rhodopsin transport disorder. The pharmaceutical, compound, composition or method of the present disclosure can be achieved with a light absorbing substance. Without wishing to be bound by theory, since the light absorbing substance of the present disclosure can receive light in place of endogenous rhodopsin, the light absorbing substance takes over the photoreception by endogenous rhodopsin, thereby ameliorating a rhodopsin transport disorder. The rhodopsin transport disorder may be those in the endoplasmic reticulum as well as those at other sites.

Although the safety of ectopic expression of opsins such as Channelrhodopsin 2 has been previously reported, the present disclosure confirms the protective effect of the light absorbing substance against retinal degeneration, as shown in the Examples. *In vitro* studies have shown that P23H opsin is misfolded and accumulates in the endoplasmic reticulum. Endoplasmic reticulum accumulation of P23H opsin may possibly induce an endoplasmic reticulum stress response and subsequent apoptosis. From the results of this experiment, the expression of coGHCR (chimeric rhodopsin) in the outer retinal layer is thought to induce suppression of endoplasmic reticulum stress and photoreceptor apoptosis leading to the above protective effect.

As used herein, the diseases, disorders or symptoms associated with the "rhodopsin transport disorder" can include, but are not limited to, retinal degenerative diseases, including retinitis pigmentosa and age-related macular degeneration. In fact, any disease, disorder or symptom in which the rhodopsin transport disorder is associated as a factor, is included in the disease, disorder or symptom.

In a preferred embodiment of the present disclosure, the light absorbing substance is biocompatible. The light absorbing substance can also include an autologous light absorbing substance. Alternatively, the light absorbing substance is preferably a non-intrinsic light absorbing substance. The light absorbing substance used in the present disclosure is preferably, but not limited to, a biocompatible light absorbing substance. The light absorbing substance used in the present disclosure may be, but is not limited to, a light absorbing substance that has neural activity-signaling activity. The light absorbing substance used in the present disclosure is preferably, but not limited to, a biocompatible light absorbing substance.

In other aspects of the present disclosure, the composition of the present disclosure can also be provided as a kit with a companion diagnostic agent combined therein. For example, a composition comprising an active ingredient, such as the light absorbing substance of the present disclosure, can exert a therapeutic effect when administered to a subject when the subject has a disease or disorder such as a rhodopsin transport disorder, or when such a disease or disorder is expected to occur. Therefore, in combination with a companion diagnostic agent for a prior diagnosis of a rhodopsin transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Rhodopsin Transport Disorder and Neural Activity-Signaling Activity)

In other aspects, the present disclosure can provide a novel pharmaceutical, compound, composition or method for ameliorating a rhodopsin transport disorder. The present disclosure can provide a method for ameliorating a rhodopsin transport disorder in a subject, the method comprising the step of administering a neural activity signaling agent or device to the subject. The present disclosure can also provide use of a neural activity signaling agent or device for manufacture of a composition or pharmaceutical for ameliorating a rhodopsin transport disorder. The pharmaceutical, compound, composition or method of the present disclosure can be achieved with a neural activity signaling agent or device. With regard to the neural activity signaling agent or device of the present disclosure, without wishing to be bound by theory, the neural activity signaling agent or device of the present disclosure can ameliorate a rhodopsin transport disorder by transmitting nerve activity signals, including electrical stimulation, to nerves on the side closer to the center. The rhodopsin transport disorder may be those in the endoplasmic reticulum as well as those at other sites.

Although the safety of ectopic expression of opsins such as Channelrhodopsin 2 has been previously reported, the present disclosure confirms the protective effect of the neural activity signaling agent or device against retinal degeneration, as shown in the Examples. *In vitro* studies have shown that P23H opsin is misfolded and accumulates in the endoplasmic reticulum. Endoplasmic reticulum accumulation of P23H opsin may possibly induce an endoplasmic reticulum stress response and subsequent apoptosis. From the results of this experiment, the expression of coGHCR (chimeric rhodopsin) in the outer retinal layer is thought to induce suppression of endoplasmic reticulum stress and photoreceptor apoptosis leading to the above protective effect.

As used herein, the diseases, disorders or symptoms associated with the "rhodopsin transport disorder" can include, but are not limited to, retinal degenerative diseases, including retinitis pigmentosa and age-related macular degeneration. In fact, any disease, disorder or symptom in which the rhodopsin transport disorder is associated as a factor, is included in the disease, disorder or symptom.

In a preferred embodiment of the present disclosure, the neural activity signaling agent or device is biocompatible. The neural activity signaling agent or device can also include an autologous, neural activity signaling agent or device. Alternatively, the neural activity signaling agent or device is preferably a non-intrinsic, neural activity signaling agent or device. The neural activity signaling agent or device used in the present disclosure is preferably, but not limited to, a biocompatible, neural activity signaling agent or device. The neural activity signaling agent or device used in the present disclosure may be, but is not limited to, a neural activity signaling agent or device that has a light absorption property. The neural activity signaling agent or device used in the present disclosure is preferably, but not limited to, a biocompatible neural activity signaling agent or device.

In other aspects of the present disclosure, the composition of the present disclosure can also be provided as a kit with a companion diagnostic agent combined therein. For example, a composition comprising an active ingredient, such as the neural activity signaling agent or device of the present disclosure, can exert a therapeutic effect when administered to a subject when the subject has a disease or disorder such as a rhodopsin transport disorder, or when such a disease or disorder is expected to occur. Therefore, in combination with a companion diagnostic agent for a prior diagnosis of a rhodopsin transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Rhodopsin Transport Disorder and Opsins)

In other aspects, as novel use of opsins or derivatives thereof, the present disclosure can provide a pharmaceutical, compound, composition or method for ameliorating a rhodopsin transport disorder. The present disclosure can provide a method for ameliorating a rhodopsin transport disorder in a subject, the method comprising the step of administering opsins or derivatives thereof or nucleic acid molecules encoding them to the subject. The present disclosure can also provide use of opsins or derivatives thereof or nucleic acid molecules encoding them, for manufacture of a composition or pharmaceutical for ameliorating a rhodopsin transport disorder. The pharmaceutical, compound, composition or method of the present disclosure can be achieved with a retinal modulating agent. Without wishing to be bound by theory, the opsins or derivatives thereof of the present disclosure can act, not on the retinal, but on the rhodopsin itself, and prevent the production of toxic metabolites such as all-trans-retinal, thereby ameliorating a rhodopsin transport disorder. The rhodopsin transport disorder may be those in the endoplasmic reticulum as well as those at other sites.

Although the safety of ectopic expression of opsins such as Channelrhodopsin 2 has been previously reported, the present disclosure confirms the protective effect of the opsins or derivatives thereof or nucleic acid molecules encoding them against retinal degeneration, as shown in the Examples. *In vitro* studies have shown that P23H opsin is misfolded and accumulates in the endoplasmic reticulum. Endoplasmic reticulum accumulation of P23H opsin may possibly induce an endoplasmic reticulum stress response and subsequent apoptosis. From the results of this experiment, the expression of coGHCR (chimeric rhodopsin) in the outer retinal layer is thought to induce suppression of endoplasmic reticulum stress and photoreceptor apoptosis leading to the above protective effect.

As used herein, the diseases, disorders or symptoms associated with the "rhodopsin transport disorder" can include, but are not limited to, retinal degenerative diseases, including retinitis pigmentosa and age-related macular degeneration. In fact, any disease, disorder or symptom in which the rhodopsin transport disorder is associated as a factor, is included in the disease, disorder or symptom.

In other aspects of the present disclosure, the composition of the present disclosure can also be provided as a kit with a companion diagnostic agent combined therein. For example, a composition comprising an active ingredient, such as the opsins or derivatives thereof or nucleic acid molecules encoding them of the present disclosure, can exert a therapeutic effect when administered to a subject when the subject has a disease or disorder such as a rhodopsin transport disorder, or when such a disease or disorder is expected to occur. Therefore, in combination with a companion diagnostic agent for a prior diagnosis of a rhodopsin transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Novel Use of Opsin)

In another aspect, as mentioned in part herein, the present disclosure provides a composition, compound, pharmaceutical or method for treating, preventing, or suppressing the progress of, a disease, disorder or symptom associated with an endoplasmic reticulum stress, comprising opsins or derivatives thereof or nucleic acid molecules encoding them. The present disclosure can provide a method for treating, preventing, or suppressing the progress of, a disease, disorder or symptom associated with an endoplasmic reticulum stress in a subject, the method comprising the step of administering opsins or derivatives thereof or nucleic acid molecules encoding them to the subject. The present disclosure can also provide use of opsins or derivatives thereof or nucleic acid molecules encoding them, for manufacture of a composition or pharmaceutical for treating, preventing, or suppressing the progress of, a disease, disorder or symptom associated with an endoplasmic reticulum stress.

In still another aspect, the present disclosure provides a composition, compound, pharmaceutical or method for reducing or eliminating an endoplasmic reticulum stress, comprising opsins or derivatives thereof or nucleic acid molecules encoding them. The opsins or derivatives thereof may have the activity of not releasing retinal upon photoreception.

In one embodiment, examples of the "disease, disorder or symptom associated with an endoplasmic reticulum stress" that may be targeted by the opsins or derivatives thereof or nucleic acid molecules encoding them of the present disclosure include, but are not limited to, vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease. In fact, any disease, disorder or symptom in which the endoplasmic reticulum stress is associated as a factor, is included in the disease, disorder or symptom.

The opsins or derivatives thereof or nucleic acid molecules encoding them of the present disclosure may be any of the opsins or rhodopsins or derivatives thereof or nucleic acid molecules encoding them that have been described in (Retinal Modulating Agent).

In other aspects of the present disclosure, the opsins or derivatives thereof or nucleic acid molecules encoding them of the present disclosure can also be provided as a kit with a companion diagnostic agent combined therein. For example, a composition comprising an active ingredient, such as the retinal modulating agent or absolute retinal modulating agent of the present disclosure, can exert a therapeutic effect when administered to a subject when the subject has a disease or disorder such as a vesicle transport disorder, or when such a disease or disorder is expected to occur. Therefore, in combination with a companion diagnostic agent for a prior diagnosis of a vesicle transport disorder or a disease or disorder associated therewith, and after diagnosing or testing the genetic statuses of subjects and the genes possessed by the subjects, the composition of the present disclosure can be administered only to subjects for whom the composition of the present disclosure can be expected to be effective.

### (Prevention or Suppression of Progress of Disease, Disorder or Symptom associated with All-Trans-Retinal)

In another aspect, the present disclosure provides a pharmaceutical, compound, composition, and method for preventing, or suppressing the progress of, a disease, disorder or symptom associated with all-trans-retinal. In one embodiment, the pharmaceutical, compound, composition, and method of the present disclosure are achieved with the retinal modulating agent, the absolute retinal modulating agent, or the opsins or derivatives thereof of the present disclosure. Without wishing to be bound by theory, the retinal modulating agent, the absolute retinal modulating agent, or the opsins or derivatives thereof of the present disclosure can modulate the state of retinal, including the *in vivo* amount, concentration, or cis/trans ratio (ratio of 11-cis-retinal to all-trans-retinal) of retinal, which is believed to decrease the proportion of all-trans-retinal and decrease apoptosis due to the cytotoxicity of all-trans-retinal or the toxicity due to its reactants such as A2E. Furthermore, due to such a mechanism, it is believed that the progress of retinal degeneration is suppressed by the retinal modulating agent, the absolute retinal modulating agent, or the opsins or derivatives thereof of the present disclosure; and due to a similar mechanism, it is believed that the disease, disorder or symptom associated with all-trans-retinal can be treated or prevented.

Therefore, in one aspect, the present disclosure can provide a composition for use in treating, preventing, or suppressing the progress of, a disease, disorder or symptom associated with all-trans-retinal, the composition comprising a retinal modulating agent, an absolute retinal modulating agent, or opsins or derivatives thereof. In one aspect, the present disclosure can provide a method for treating, preventing, or suppressing the progress of, a disease, disorder or symptom associated with all-trans-retinal, the method comprising the step of administering an effective amount of a retinal modulating agent, an absolute retinal modulating agent, or opsins or derivatives thereof, to a subject. In other aspects, the present disclosure provides use of a retinal modulating agent, an absolute retinal modulating agent, or opsins or derivatives thereof, for manufacture of a composition for use in treating, preventing, or suppressing the progress of, a disease, disorder or symptom associated with all-trans-retinal in a subject. In one embodiment, examples of the "disease, disorder or symptom associated with all-trans-retinal" include such a disease, disorder or symptom that develops due to apoptosis of visual cells due to the cytotoxicity of all-trans-retinal or the toxicity due to its reactants such as A2E. For example, the disease, disorder or symptom associated with all-trans-retinal includes, but is not limited to, glaucoma and various retinal diseases including age-related macular degeneration, Stargardt's disease, fundus flavum, and recessive retinitis pigmentosa. In fact, any disease, disorder or symptom that is directly or indirectly associated with all-trans-retinal toxicity is included in the disease, disorder or symptom.

Furthermore, in other aspects, in the present disclosure, a composition for use in reducing or eliminating all-trans-retinal toxicity, the composition comprising an absolute retinal modulating agent, can be provided. As described above, the absolute retinal modulating agent can modulate the amount, concentration, or cis/trans ratio of retinal by not producing all-trans-retinal. This is believed to lower the amount and ratio of all-trans-retinal and also reduce or eliminate its toxicity.

Without wishing to be bound by theory, there is an endogenous rhodopsin, that is, a natural rhodopsin that isomerizes released 11-cis-retinal upon photoreception to produce all-trans-retinal, present even when the absolute retinal modulating agent of the present disclosure is used. It was therefore not possible to assume efficient acquisition of the effect of treating or preventing a disease, disorder or symptom associated with all-trans-retinal, or the effect of inhibiting or reducing cell death or apoptosis, associated with reduction of all-trans-retinal, a toxic metabolite. In the present disclosure, however, even in the presence of endogenous rhodopsin, the use of the absolute retinal modulating agent allowed acquiring of the effect of treating or preventing a disease, disorder or symptom associated with all-trans-retinal as well as the effect of inhibiting or reducing cell death or apoptosis, as shown in the Examples. This is a surprising result.

Accordingly, in one aspect of the present disclosure, provided is a composition for use in inhibiting cell death (or apoptosis), comprising a retinal modulating agent, an absolute retinal modulating agent, or opsins or derivatives thereof.

Absence of 11-cis-retinal is known to induce cytotoxicity during the growth of the rod outer segment in P23H mice. As shown in the Examples, coGHCR has all-trans-retinal as a chromophore like microbial rhodopsin, so it does not consume cis-retinal and does not undergo photobleaching. Therefore, it is possible for the expressed coGHCR to suppress the consumption of cis-retinal by replacing visual cells. Accordingly, the retinal modulating agent of the present disclosure is believed to decrease apoptosis by increasing the proportion of 11-cis-retinal in the cis/trans ratio and decreasing the toxicity of all-trans-retinal.

Furthermore, in other aspects, the present disclosure provides a method of treating, preventing, or suppressing the progress of, a disease, disorder, or symptom associated with all-trans-retinal in a subject, the method comprising the step of administering a therapeutically effective amount of a composition of the present disclosure to the subject.

In one embodiment, examples of the "disease, disorder or symptom associated with all-trans-retinal" include such a disease, disorder or symptom that develops due to apoptosis of visual cells due to the cytotoxicity of all-trans-retinal or the toxicity due to its reactants such as A2E. For example, the disease, disorder or symptom associated with all-trans-retinal includes, but is not limited to, glaucoma and various retinal diseases including age-related macular degeneration, Stargardt's disease, fundus flavum, and recessive retinitis pigmentosa. In fact, any disease, disorder or symptom that is directly or indirectly associated with all-trans-retinal toxicity is included in the disease, disorder or symptom.

In other aspects of the present disclosure, the composition of the present disclosure can also be provided as a kit with a companion diagnostic agent combined therein. For example, a composition comprising a retinal modulating agent, an absolute retinal modulating agent, or opsins or derivatives thereof of the present disclosure, can exert a therapeutic effect when administered to a subject when the subject has a disease or disorder associated with all-trans-retinal or the like, or when such a disease or disorder is expected to occur. Therefore, in combination with a companion diagnostic agent for a prior diagnosis as to whether a subject is susceptible to a disease or disorder associated with all-trans-retinal, and after diagnosing or testing the genetic statuses of subjects and the genes possessed by the subjects, the composition of the present disclosure can be administered only to subjects for whom the composition of the present disclosure can be expected to be effective.

### (Disease or the like associated with All-Trans-Retinal, and Light Absorbing Substance)

In another aspect, the present disclosure still further provides a pharmaceutical, compound, composition, and method for preventing or suppressing the progress of a disease, disorder, or symptom associated with all-trans-retinal. In one embodiment, the pharmaceutical, compound, composition, and method of the present disclosure is achieved with the light absorbing substance of the present disclosure. Without wishing to be bound by theory, since the light absorbing substance of the present disclosure can receive light in place of endogenous rhodopsin, the light absorbing substance takes over the photoreception by endogenous rhodopsin, which is believed to decrease the proportion of all-trans-retinal and decrease apoptosis due to the cytotoxicity of all-trans-retinal or the toxicity due to its reactants such as A2E. Furthermore, due to such a mechanism, it is believed that the progress of retinal degeneration is suppressed by the light absorbing substance of the present disclosure; and due to a similar mechanism, it is believed that the disease, disorder or symptom associated with all-trans-retinal can be treated or prevented.

Therefore, in one aspect, the present disclosure can provide a composition for use in treating, preventing, or suppressing the progress of, a disease, disorder or symptom associated with all-trans-retinal, the composition comprising a light absorbing substance. In one aspect, the present disclosure can provide a method for treating, preventing, or suppressing the progress of, a disease, disorder or symptom associated with all-trans-retinal, the method comprising the step of administering an effective amount of a light absorbing substance to a subject. In other aspects, the present disclosure provides use of a light absorbing substance, for manufacture of a composition for use in treating, preventing, or suppressing the progress of, a disease, disorder or symptom associated with all-trans-retinal in a subject. In one embodiment, examples of the "disease, disorder or symptom associated with all-trans-retinal" include such a disease, disorder or symptom that develops due to apoptosis of visual cells due to the cytotoxicity of all-trans-retinal or the toxicity due to its reactants such as A2E. For example, the disease, disorder or symptom associated with all-trans-retinal includes, but is not limited to, glaucoma and various retinal diseases including age-related macular degeneration, Stargardt's disease, fundus flavum, and recessive retinitis pigmentosa. In fact, any disease, disorder or symptom that is directly or indirectly associated with all-trans-retinal toxicity is included in the disease, disorder or symptom.

Accordingly, in one aspect of the present disclosure, provided is a composition for use in inhibiting cell death (or apoptosis), comprising a light absorbing substance.

Absence of 11-cis-retinal is known to induce cytotoxicity during the growth of the rod outer segment in P23H mice. As shown in the Examples, coGHCR has all-trans-retinal as a chromophore like microbial rhodopsin, so it does not consume cis-retinal and does not undergo photobleaching. Therefore, it is possible for the expressed coGHCR to suppress the consumption of cis-retinal by replacing visual cells. Accordingly, the light absorbing substance of the present disclosure is believed to decrease apoptosis by various mechanisms.

Furthermore, in other aspects, the present disclosure provides a method of treating, preventing, or suppressing the progress of, a disease, disorder, or symptom associated with all-trans-retinal in a subject, the method comprising the step of administering a therapeutically effective amount of a composition of the present disclosure to the subject.

In one embodiment, examples of the "disease, disorder or symptom associated with all-trans-retinal" include such a disease, disorder or symptom that develops due to apoptosis of visual cells due to the cytotoxicity of all-trans-retinal or the toxicity due to its reactants such as A2E. For example, the disease, disorder or symptom associated with all-trans-retinal includes, but is not limited to, glaucoma and various retinal diseases including age-related macular degeneration, Stargardt's disease, fundus flavum, and recessive retinitis pigmentosa. In fact, any disease, disorder or symptom that is directly or indirectly associated with all-trans-retinal toxicity is included in the disease, disorder or symptom.

In a preferred embodiment of the present disclosure, the light absorbing substance is biocompatible. The light absorbing substance can also include an autologous light absorbing substance. Alternatively, the light absorbing substance is preferably a non-intrinsic light absorbing substance. The light absorbing substance used in the present disclosure is preferably, but not limited to, a biocompatible light absorbing substance. The light absorbing substance used in the present disclosure may be, but is not limited to, a light absorbing substance that has neural activity signaling activity. The light absorbing substance used in the present disclosure is preferably, but not limited to, a biocompatible light absorbing substance.

In other aspects of the present disclosure, the composition of the present disclosure can also be provided as a kit with a companion diagnostic agent combined therein. For example, a composition comprising a light absorbing substance of the present disclosure can exert a therapeutic effect when administered to a subject when the subject has a disease or disorder associated with all-trans-retinal or the like, or when such a disease or disorder is expected to occur. Therefore, in combination with a companion diagnostic agent for a prior diagnosis as to whether a subject is susceptible to a disease or disorder associated with all-trans-retinal, and after diagnosing or testing the genetic statuses of subjects and the genes possessed by the subjects, the composition of the present disclosure can be administered only to subjects for whom the composition of the present disclosure can be expected to be effective.

### (Disease associated with All-Trans-Retinal, and Neural-Activity-Signaling Activity)

In another aspect, the present disclosure provides a novel pharmaceutical, compound, composition, and method for preventing or suppressing the progress of a disease, disorder, or symptom associated with all-trans-retinal. In one embodiment, the pharmaceutical, compound, composition, and method of the present disclosure is achieved with the neural activity signaling agent or device of the present disclosure. Without wishing to be bound by theory, the neural activity signaling agent or device of the present disclosure transmits nerve activity signals, including electrical stimulation, to nerves on the side closer to the center, which unexpectedly decreases the proportion of all-trans-retinal, and which is believed to decrease apoptosis due to the cytotoxicity of all-trans-retinal or the toxicity due to its reactants such as A2E. Furthermore, due to such a mechanism, it is believed that the progress of retinal degeneration is suppressed by the neural activity signaling agent or device of the present disclosure; and due to a similar mechanism, it is believed that the disease, disorder or symptom associated with all-trans-retinal can be treated or prevented.

Therefore, in one aspect, the present disclosure can provide a composition for use in treating, preventing, or suppressing the progress of, a disease, disorder or symptom associated with all-trans-retinal, the composition comprising a neural activity signaling agent or device. In one aspect, the present disclosure can provide a method for treating, preventing, or suppressing the progress of, a disease, disorder or symptom associated with all-trans-retinal, the method comprising the step of administering an effective amount of a neural activity signaling agent or device to a subject. In other aspects, the present disclosure provides use of a neural activity signaling agent or device, for manufacture of a composition for use in treating, preventing, or suppressing the progress of, a disease, disorder or symptom associated with all-trans-retinal in a subject. In one embodiment, examples of the "disease, disorder or symptom associated with all-trans-retinal" include such a disease, disorder or symptom that develops due to apoptosis of visual cells due to the cytotoxicity of all-trans-retinal or the toxicity due to its reactants such as A2E. For example, the disease, disorder or symptom associated with all-trans-retinal includes, but is not limited to, glaucoma and various retinal diseases including age-related macular degeneration, Stargardt's disease, fundus flavum, and recessive retinitis pigmentosa. In fact, any disease, disorder or symptom that is directly or indirectly associated with all-trans-retinal toxicity is included in the disease, disorder or symptom.

Accordingly, in one aspect of the present disclosure, provided is a composition for use in inhibiting cell death (or apoptosis), comprising a neural activity signaling agent or device.

Absence of 11-cis-retinal is known to induce cytotoxicity during the growth of the rod outer segment in P23H mice. As shown in the Examples, coGHCR has all-trans-retinal as a chromophore like microbial rhodopsin, so it does not consume cis-retinal and does not undergo photobleaching. Therefore, it is possible for the expressed coGHCR to suppress the consumption of cis-retinal by replacing visual cells. Accordingly, the neural activity signaling agent or device of the present disclosure is believed to decrease apoptosis by various mechanisms. In a preferred embodiment of the present disclosure, the neural activity signaling agent or device is biocompatible. The neural activity signaling agent or device can also include an autologous, neural activity signaling agent or device. Alternatively, the neural activity signaling agent or device is preferably a non-intrinsic, neural activity signaling agent or device. The neural activity signaling agent or device used in the present disclosure is preferably, but not limited to, a biocompatible, neural activity signaling agent or device. The neural activity signaling agent or device used in the present disclosure may be, but is not limited to, a neural activity signaling agent or device that has a light absorption property. The neural activity signaling agent or device used in the present disclosure is preferably, but not limited to, a biocompatible neural activity signaling agent or device.

Furthermore, in other aspects, the present disclosure provides a method of treating, preventing, or suppressing the progress of, a disease, disorder, or symptom associated with all-trans-retinal in a subject, the method comprising the step of administering a therapeutically effective amount of a composition of the present disclosure to the subject.

In one embodiment, examples of the "disease, disorder or symptom associated with all-trans-retinal" include such a disease, disorder or symptom that develops due to apoptosis of visual cells due to the cytotoxicity of all-trans-retinal or the toxicity due to its reactants such as A2E. For example, the disease, disorder or symptom associated with all-trans-retinal includes, but is not limited to, glaucoma and various retinal diseases including age-related macular degeneration, Stargardt's disease, fundus flavum, and recessive retinitis pigmentosa. In fact, any disease, disorder or symptom that is directly or indirectly associated with all-trans-retinal toxicity is included in the disease, disorder or symptom.

In other aspects of the present disclosure, the composition of the present disclosure can also be provided as a kit with a companion diagnostic agent combined therein. For example, a composition comprising a neural activity signaling agent or device of the present disclosure can exert a therapeutic effect when administered to a subject when the subject has a disease or disorder associated with all-trans-retinal or the like, or when such a disease or disorder is expected to occur. Therefore, in combination with a companion diagnostic agent for a prior diagnosis as to whether a subject is susceptible to a disease or disorder associated with all-trans-retinal, and after diagnosing or testing the genetic statuses of subjects and the genes possessed by the subjects, the composition of the present disclosure can be administered only to subjects for whom the composition of the present disclosure can be expected to be effective.

### (Protection, and Suppression of Atrophy Progress, of Retinal Thickness)

In another aspect, the present disclosure provides a pharmaceutical, compound, composition, and method for protecting a retinal thickness, or suppressing the progress of atrophy of a retinal thickness. In one embodiment, the pharmaceutical, compound, composition, and method of the present disclosure are achieved with the retinal modulating agent, the absolute retinal modulating agent, the light absorbing substance, the neural activity signaling agent or device, or the opsins or derivatives thereof of the present disclosure. Without wishing to be bound by theory, the retinal modulating agent, the absolute retinal modulating agent, the light absorbing substance, the neural activity signaling agent or device, or the opsins or derivatives thereof of the present disclosure can: modulate the amount of retinal and the cis-trans ratio, reduce the amount of all-trans-retinal, a toxic photometabolite, and suppress apoptosis, as described above, by receiving light or by mediating neural activity; and suppress an endoplasmic reticulum stress, by relatively increasing 11-cis retinal. Furthermore, such a mechanism can suppress the progress of degeneration and disappearance of visual cells and protect the thickness of the outer retina centering on the visual cells. For example, the retinal modulating agent, the absolute retinal modulating agent, the light absorbing substance, the neural activity signaling agent or device, or the opsins or derivatives thereof of the present disclosure can exert effects as described above by receiving light in place of visual cells while the visual cells still remain.

Furthermore, the retinal modulating agent, the absolute retinal modulating agent, the light absorbing substance, the neural activity signaling agent or device, or the opsins or derivatives thereof of the present disclosure are thought to be able to receive light and continue sending light signals to the center. By such a mechanism, the retinal modulating agent, the absolute retinal modulating agent, the light absorbing substance, the neural activity signaling agent or device, or the opsins or derivatives thereof of the present disclosure can protect the central retina (inner layer of the retina), which is supposed to atrophy due to disuse, or suppress the progress of the disuse atrophy. Specifically, in the visual transmission system, visual cells sense light and transmit visual signals to the brain via neurons. For example, in retinitis pigmentosa, a disease in which visual cells and retinal pigment epithelial cells degenerate or disappear leading to blindness, when the visual cells disappear, the visual signal ceases, so the nerves on the central side also atrophy due to disuse. However, the retinal modulating agent, the absolute retinal modulating agent, the light absorbing substance, the neural activity signaling agent or device, or the opsins or derivatives thereof of the present disclosure continue sending light signals to the center, thus suppressing disuse atrophy, which can protect the visual pathway from the photoreceptors to the center, especially the inner retinal thickness.

Therefore, in one aspect, the present disclosure can provide a composition for use in protecting a retinal thickness, or suppressing the progress of atrophy of a retinal thickness, the composition comprising a retinal modulating agent, an absolute retinal modulating agent, a light absorbing substance, a neural activity signaling agent or device, or opsins or derivatives thereof. In one aspect, the present disclosure can provide a method for protecting a retinal thickness, or suppressing the progress of atrophy of a retinal thickness, the method comprising the step of administering an effective amount of a retinal modulating agent, an absolute retinal modulating agent, a light absorbing substance, a neural activity signaling agent or device, or opsins or derivatives thereof to a subject. In other aspects, the present disclosure provides use of a retinal modulating agent, an absolute retinal modulating agent, a light absorbing substance, a neural activity signaling agent or device, or opsins or derivatives thereof, for manufacture of a composition for use in protecting a retinal thickness, or a composition for use in suppressing the progress of atrophy of a retinal thickness. In one embodiment, the term "protecting a retinal thickness", "suppressing the progress of atrophy" or "suppressing atrophy" refers to: protecting, retaining, or maintaining the thickness of the inner or outer retinal layer; suppressing the progress of atrophy or decrease of the retina; or suppressing the atrophy itself of the retinal thickness. In addition, as used herein, "protecting a retinal thickness" may be used interchangeably with "retaining a retinal thickness" or "maintaining a retinal thickness" and both have the same meaning. In one embodiment, the term "protecting a retinal thickness" means that the retinal thickness is maintained within 70-130 (142)%, within 80-120 (125)%, preferably within 90-110 (111)%, and within 95-105%, compared to not administering the retinal modulating agent, absolute retinal modulating agent, or opsins or derivatives thereof of the present disclosure. In one embodiment, the term "suppressing the progress of atrophy" means that progress of atrophy of retinal thickness is suppressed by at least 10% or more, preferably 20% or more, more preferably 30% or more, still more preferably 40% or more, and even more preferably 50% or more, compared to not administering the retinal modulating agent, absolute retinal modulating agent, light absorbing substance, neural activity signaling agent or device, or opsins or derivatives thereof of the present disclosure.

In one embodiment, the term "suppressing atrophy" means that the retinal thickness is maintained at least 70% or more, preferably 80% or more, more preferably 90% or more, still more preferably 95% or more, and even still more preferably at the current level, compared to not administering the retinal modulating agent, absolute retinal modulating agent, or opsins or derivatives thereof of the present disclosure.

In other aspects of the present disclosure, the composition of the present disclosure can also be provided as a kit with a companion diagnostic agent combined therein. For example, a composition comprising a retinal modulating agent, an absolute retinal modulating agent, or opsins or derivatives thereof of the present disclosure, can exert a therapeutic effect when administered to a subject when retinal thickness atrophy develops, or when such a disorder is expected to occur. Therefore, in combination with a companion diagnostic agent for a prior diagnosis as to whether a subject is likely to develop retinal thickness atrophy, and after diagnosing or testing the genetic statuses of subjects and the genes possessed by the subjects, the composition of the present disclosure can be administered only to subjects for whom the composition of the present disclosure can be expected to be effective.

### (Pharmaceutical, and Treatment or Prevention Method)

In one aspect, the present disclosure provides a treatment or prevention method by means of administration of a composition or pharmaceutical of the present disclosure. In one embodiment, the composition or pharmaceutical of the present disclosure is administered by injection. In another embodiment, the composition or pharmaceutical of the present disclosure is administered intravitreally. In a particular embodiment, the composition or pharmaceutical of the present disclosure may be provided with a preservative solution. In some embodiments, the preservative solution may be a buffer solution. In other embodiments, the composition or pharmaceutical of the present disclosure may be provided in a container. In a particular embodiment, the container containing the composition or pharmaceutical of the present disclosure may be a syringe.

In another aspect, the present disclosure provides cells comprising the composition of the present disclosure. In some embodiments, the cells of the present disclosure can be retinal cells. In another embodiment, the cells of the present disclosure may be provided as a cell preparation. The cell preparation includes cells and a cell preservative solution. In some embodiments, the cell preservative solution may be a culture medium or a buffer solution. In other embodiments, the cells of the present disclosure may be provided in a container. In a particular embodiment, the container containing the cells of the present disclosure may be a syringe.

In other aspects, the present disclosure provides a pharmaceutical comprising a composition or cell of the present disclosure. In one embodiment, the pharmaceutical of the present disclosure may be for treating, preventing, or suppressing the progress of, a disease, disorder or symptom associated with an endoplasmic reticulum stress. In one embodiment, the pharmaceutical of the present disclosure may be for reducing or eliminating an endoplasmic reticulum stress. In one embodiment, the pharmaceutical of the present disclosure may be for ameliorating a rhodopsin transport disorder. In one embodiment, the pharmaceutical of the present disclosure may be for treating, preventing, or suppressing the progress of, a disease, disorder or symptom associated with all-trans-retinal. In one embodiment, the pharmaceutical of the present disclosure may be for reducing or eliminating all-trans-retinal toxicity. In one embodiment, the pharmaceutical of the present disclosure may be for inhibiting cell death (or apoptosis).

In one aspect of the present disclosure, the composition or pharmaceutical of the present disclosure can also be provided as a nucleic acid pharmaceutical. In one embodiment, in performing gene therapy or gene treatment using the nucleic acid pharmaceutical of the present disclosure, polynucleotides can be introduced into the genome of cells to restore or alter genes and/or gene expression. For example, it is possible to utilize treatment methods, etc., for introducing normal genes by vectors capable of transduction into human cells, such as various viral vectors, or by some other delivery systems. The method for introducing the vector can be appropriately selected in accordance with the type of vector and host, and the like. When the expression vector is used as an active ingredient of the composition of the present disclosure, the transduction can be achieved by injecting the above AAV vector or the like into the eye, for example.

As used herein, "gene therapy" is the insertion of nucleic acid sequences (e.g., transgenes as defined herein) into cells and/or tissues of an individual to treat disease. A transgene can be a functional mutant allele that replaces or complements a defective allele. The gene therapy also includes insertion of a transgene that inhibits, reduces or lowers the expression, activity or function of an endogenous gene or protein, such as a gene or protein that is naturally inhibitory, i.e., undesirable or aberrant (e.g., pathogenic). Such a transgene may be exogenous. An exogenous molecule or sequence is understood to be a molecule or sequence not normally present in the cell, tissue and/or individual to be treated. Both acquired and congenital diseases are amenable to gene therapy.

As used herein, a "gene therapy vector" is any vector capable of delivering a polynucleotide encoding a therapeutic protein (e.g., opsins, etc.) to a host, such as a patient. In some embodiments, gene therapy vectors are targeted to specific host cells or organs, for example, for local delivery, such as tissue-specific delivery. Typically, local delivery involves proteins (e.g., therapeutic proteins) encoded by mRNAs that are translated and expressed primarily in and/or by an organ, such as the liver, which thereby forms a depot, for example, a liver depot for the production (and secretion) of proteins. In some embodiments, the gene therapy vector is configured to deliver a polynucleotide of a therapeutic protein to the patient's eye. In some embodiments, the gene therapy vector delivers a polynucleotide encoding a therapeutic protein to other tissues in the patient. In some embodiments, the gene therapy vector delivers a polynucleotide encoding a therapeutic protein to the patient's optic nerve.

Any known or future developed gene therapy delivery vector, whether natural or engineered, can be used in the practice of the present disclosure. In some embodiments, gene therapy vectors are viral vectors, including, for example, viruses, viral capsids, viral genomes, and the like. In some embodiments, gene therapy vectors are naked polynucleotides, such as episomes. In some embodiments, a gene therapy vector comprises a polynucleotide complex. Exemplary, non-limiting polynucleotide complexes for use as gene therapy vectors include lipoplexes, polymersomes, polypexes, dendrimers, and inorganic nanoparticles (e.g., polynucleotide-coated gold, silica, iron oxide, calcium phosphate, etc.). In some embodiments, gene therapy vectors described herein comprise combinations of viral vectors, naked polynucleotides, and polynucleotide complexes.

In one embodiment, the gene therapy vector is a viral vector, including retroviruses, adenoviruses, herpes simplex viruses, poxviruses, vaccinia viruses, lentiviruses, or adeno-associated viruses. In one embodiment, the gene therapy vector is an adeno-associated virus (AAV), including serotypes AAV1, AAV2, AAV3, AAV4, AAV5, AAV6, AAV7, AAV8, AAV9, AAV10, and AAV11, or engineered or natural, selected variants thereof. In one embodiment, the polynucleotide also contains an adeno-associated virus (AAV) nucleic acid sequence. In one embodiment, the gene therapy vector is a chimeric adeno-associated virus containing genetic elements from two or more serotypes. For example, an AAV vector (referred to as AAV1/2 or AAV RC1/2) having an AAV1-derived rep gene and an AAV2-derived cap gene may be used as a gene therapy vector to deliver polynucleotides of therapeutic proteins of the present disclosure to cells or cells of a patient in need thereof. In one embodiment, the gene therapy vector is AAV1/2, AAV1/3, AAV1/4, AAV1/5, AAV1/6, AAV1/7, AAV1/8, AAV1/9, AAV1/10, AAV1/11, AAV2/1, AAV2/3, AAV2/4, AAV2/5, AAV2/6, AAV2/7, AAV2/8, AAV2/9, AAV2/10, AAV2/11, AAV3/1, AAV3/2, AAV3/4, AAV3/5, AAV3/6, AAV3/7, AAV3/8, AAV3/9, AAV3/10, AAV3/10, AAV4/1, AAV4/2, AAV4/3, AAV4/5, AAV4/6, AAV4/7, AAV4/8, AAV4/9, AAV4/10, AAV4/11, AAV5/1, AAV5/2, AAV5/3, AAV5/4, AAV5/6, AAV5/7, AAV5/8, AAV5/9, AAV5/10, AAV5/11, AAV6/1, AAV6/2, AAV6/3, AAV6/4, AAV6/5, AAV6/7, AAV6/8, AAV6/9, AAV6/10, AAV6/10, AAV7/1, AAV7/2, AAV7/3, AAV7/4, AAV7/5, AAV7/6, AAV7/8, AAV7/9, AAV7/10, AAV7/11, AAV8/1, AAV8/2, AAV8/3, AAV8/4, AAV8/5, AAV8/6, AAV8/7, AAV8/9, AAV8/10, AAV8/11, AAV9/1, AAV9/2, AAV9/3, AAV9/4, AAV9/5, AAV9/6, AAV9/7, AAV9/8, AAV9/10, AAV9/11, AAV10/1, AAV10/2, AAV10/3, AAV10/4, AAV10/5, AAV10/6, AAV10/7, AAV10/8, AAV10/9, AAV10/11, AAV11/1, AAV11/2, AAV11/3, AAV11/4, AAV11/5, AAV11/6, AAV11/7, AAV11/8, AAV11/9, AAV11/10, or a chimeric virion or derivative thereof. Gao et al., "Novel adeno-associated viruses from rhesus monkeys as vectors for human gene therapy", PNAS 99(18): 11854-11859, Sep. 3, 2002 is incorporated herein by reference for AAV vectors and chimeric virions useful as gene therapy vectors, and the construction pairs and uses thereof.

In some embodiments, gene therapy vectors are viral vectors that have been pseudotyped (e.g., engineered) to target specific cells (e.g., retinal cells). Many of the advances in targeted gene therapy using viral vectors have been non-recombinant (non-genetic) or recombinant (genetic) modifications of viral vectors, and as a result, they can be grouped together as those resulting in the natural tropism pseudotyping, expansion, and/or retargeting of viral vectors. (This is outlined in Nicklin and Baker (2002) Curr. Gene Ther. 2:273-93; Verheiji and Rottier (2012) Advances Virol 2012:1-15.) Non-genetic approaches typically utilize adapters that recognize both wild-type (unmodified) viral surface proteins and target cells. Soluble pseudo-receptors (against wild-type virus), polymers such as polyethylene glycol, and antibodies or portions thereof have been used as virus-binding domains of adapters, while natural peptides or vitamin ligands as well as antibodies and portions thereof have been used for the cell-binding domains of the adapters described above. For example, retargeting of a viral vector to a target cell can be accomplished by binding the vector:adaptor complex to a protein expressed on the surface of the target cell, such as a cell surface protein. Such approaches have been used for AAV (Bartlett et al. (1999) Nat. Biotechnol. 74: 2777-2785), adenoviruses (Hemminki et al. (2001) Cancer Res. 61: 6377-81; van Beusechem et al. (2003) Gene Therapy 10:1982-1991; Einfeld, et al. (2001) J. Virol. 75:11284-91; Glasgow et al. (2009) PLOS One 4:e8355), herpesviruses (Nakano et al. (2005) Mol. Ther. 11:617-24), paramyxoviruses (Bian et al. (2005) Cancer Gene Ther. 12:295-303; Bian et al. (2005) Int. J. Oncol. 29:1359-69), and coronaviruses (Haijema et al. (2003) J. Virol. 77:4528-4538; Wurdinger et al. (2005) Gene Therapy 12:1394-1404).

A common approach is the genetic modification by recombination of the viral capsid proteins and thus the surface of the viral capsid. In the indirect recombination approach, the viral capsid is modified with a heterologous "scaffold" and then ligated to an adaptor. The adapter binds to the scaffold and target cells. (See also Arnold et al. (2006) Mol. Ther. 5:125-132; Ponnazhagen et al. (2002) J. Virol. 76:12900-907; WO 97/05266.) Scaffolds, for example: (1) Fc binding molecules (e.g. Fc receptors, protein A, etc.) that bind to the Fc of the antibody adapter; (2) (strept) avidin that binds to the biotinylated adapter; (3) biotin binding to an adapter fused to (strept) avidin; and (4) SpyCatcher binding to SpyTag adapter, such as protein:protein binding pairs forming isometric peptide bonds, are incorporated into Ad (Pereboeva et al. (2007) Gene Therapy 14: 627-637; Park et al. (2008) Biochemical and Biophysical Research Communications 366: 769-774; Henning et al. (2002) Human Gene Therapy 13:1427-1439; Banerjee et al. (2011) Bioorganic and Medicinal Chemistry Letters 21:4985-4988), AAV (Gigout et al. (2005) Molecular Therapy 11:856-865; Stachler et al. (2008) Molecular Therapy 16:1467-1473), and Togavirus (Quetglas et al. (2010) Virus Research 153: 179-196; Ohno et al. (1997) Nature Biotechnology 15:763-767; Klimstra et al. (2005) Virology 338:9-21).

In a direct recombinant targeting approach, the targeting ligand is directly inserted or bound into the viral capsid, that is, the protein viral capsid is modified to express the heterologous ligand. The ligand redirects, e.g., binds, a receptor or marker that is preferentially or exclusively expressed on the target cell. It is used for pox virus (Guse et al. (2011) Expert Opinion on Biological Therapy 11:595-608; Galmiche et al. (1997) Journal of General Virology 78: 3019-3027; Paul et al. (2007) Viral Immunology 20:664-671), paramyxovirus (Nakamura and Russell (2004) Expert Opinion on Biological Therapy 4: 1685-1692; Hammond et al. (2001) Journal of Virology 75: 2087-2096; Galanis (2010) Clinical Pharmacology and Therapeutics 88:620-625; Blechacz and Russell (2008) Current Gene Therapy 8:162-175; Russell and Peng (2009) Current Topics in Microbiology and Immunology 330:213-241), and herpesvirus (Shah and Breakefield (2006) Current Gene Therapy 6:361-370; Campadelli-Fiume et al. (2011) Reviews in Medical Virology 21:213-226).

In some embodiments, gene therapy vectors described herein comprise naked polynucleotides. For example, in some embodiments, a polynucleotide encoding a therapeutic polypeptide may be injected directly into an organ, intravenously, for the formation of a depot, near the eyeball for example. Additional known methods for enhanced delivery of naked polynucleotides include, but are not limited to, electroporation, ultrasonic poration, use of gene guns to eject polynucleotide-coated gold particles, magnetic particles, and hydraulic delivery.

In some embodiments, therapy vectors described herein may be polynucleotide complexes, including, but not limited to, for example, nanoparticles (e.g., polynucleotide self-assembled nanoparticles, polymer-based self-assembled nanoparticles, inorganic nanoparticles, lipid nanoparticles, semiconducting/metallic nanoparticles), gels and hydrogels, polynucleotide complexes comprising cations and anions, microparticles, and any combinations thereof.

In some embodiments, the polynucleotides disclosed herein can be formulated as self-assembled nanoparticles. As a non-limiting example, polynucleotides may be used to make nanoparticles that can be used in delivery systems for polynucleotides (see, for example, International Patent Application Publication No. 2012125987, which is incorporated herein by reference in its entirety). In some embodiments, a polynucleotide self-assembled nanoparticle can comprise a polynucleotide core and a polymer shell disclosed herein. The polymer shell can be any of the polymers described herein, and such polymers are known in the art. In an additional embodiment, the polymer shell may be used to protect the polynucleotide within the core.

In some embodiments, these self-assembled nanoparticles can be microsponges formed of long polymeric polynucleotide hairpins, and the polynucleotide hairpins are formed into crystalline "pleated" sheets and then self-assembled into microsponges. These microsponges are densely packed, sponge-like microparticles that can act as efficient carriers and deliver cargo to cells. The microsponges can be from 300 nm to 1 um in diameter. The microsponges may be complexed with other agents known in the art to form larger microsponges. As a non-limiting example, the microsponges may be complexed with agents such as polycationic polyethyleneimine (PEI) for forming an outer layer and facilitating uptake by cells. This complex can form 250 nm diameter particles that can remain stable at high temperatures (150°C.) (Grabow and Jaegar, Nature Materials 2012, 11:269-269; incorporated herein by reference in its entirety). Additionally, these microsponges may be able to exert an exceptional degree of protection from degradation by ribonucleases. In another embodiment, polymer-based self-assembled nanoparticles, such as, but not limited to, microsponges, can be fully programmable nanoparticles. Nanoparticle geometry, size and stoichiometry can be precisely controlled to create the optimal nanoparticles for cargo delivery, including but not limited to polynucleotides.

In some embodiments, polynucleotides can be formulated into inorganic nanoparticles (US Patent No. 8,257,745, which is incorporated herein by reference in its entirety). The inorganic nanoparticles can include, but are not limited to, water-swellable clay-based materials. As a non-limiting example, the inorganic nanoparticles can include synthetic smectite clays made from simple silicates (see, for example, U.S. Patent Nos. 5,585,108 and 8,257,745, each of which is hereby incorporated by reference in its entirety).

In some embodiments, the polynucleotides may be formulated with water-dispersible nanoparticles comprising semiconducting or metallic materials (U.S. Patent Application Publication No. 20120228565, which is incorporated herein by reference in its entirety), or may be formed with magnetic nanoparticles (U.S. Patent Application Publication Nos. 20120265001 and 20120283503, which are incorporated herein by reference in their entireties).

In some embodiments, the polynucleotides disclosed herein can be encapsulated in any hydrogel known in the art that can form a gel when injected into a subject. The hydrogels are networks of hydrophilic polymer chains and are sometimes found as colloidal gels in which water is the dispersing medium. The hydrogels may comprise highly absorbent (capable of containing more than 99% water) natural or synthetic polymers. The hydrogels also have flexibility that is very similar to natural tissue due to their significantly higher water content. The hydrogels described herein may be used to encapsulate biocompatible, biodegradable, and/or porous lipid nanoparticles.

As a non-limiting example, the hydrogels may be an aptamer-functionalized hydrogel. The aptamer-functionalized hydrogels may be programmed to release one or more polynucleotides using polynucleotide hybridization (Battig et al., J. Am. Chem. Society. 2012 134:12410-12413; incorporated herein by reference in its entirety). In some embodiments, the polynucleotides may be encapsulated within lipid nanoparticles, and the lipid nanoparticles may then be encapsulated within a hydrogel.

In some embodiments, the polynucleotides may be encapsulated in a fibrin gel, fibrin hydrogel, or fibrin adhesive. In another embodiment, the polynucleotides may be formulated into lipid nanoparticles or rapid-clearing lipid nanoparticles prior to encapsulation in a fibrin gel, fibrin hydrogel, or fibrin adhesive. In yet another embodiment, the polynucleotides may be formulated as lipoplexes prior to encapsulation in a fibrin gel, hydrogel, or fibrin adhesive. The fibrin gel, hydrogel, and adhesives contain two components, a fibrinogen solution and a calcium-rich thrombin solution (see, for example, Spicer and Mikos, Journal of Controlled Release 2010. 148: 49-55; and Kidd et al. Journal of Controlled Release 2012. 157: 80-85, each of which is incorporated herein by reference in its entirety). The concentrations of components of the fibrin gel, hydrogel, and/or adhesive can be varied to alter the gel, hydrogel, and/or adhesive properties, network mesh size, and/or degradation properties; and for example, without limitation, the release properties of the fibrin gel, hydrogel, and/or adhesive can be altered (see, for example, Spicer and Mikos, Journal of Controlled Release 2010. 148: 49-55; Kidd et al. Journal of Controlled Release 2012. 157:80-85; Catelas et al. Tissue Engineering 2008. 14:119-128, each of which is incorporated herein by reference in its entirety). This feature can be advantageous when used to deliver the polynucleotides disclosed herein (see, for example, Kidd et al. Journal of Controlled Release 2012. 157:80-85; and Catelas et al. Tissue Engineering 2008. 14:119-128, each of which is incorporated herein by reference in its entirety).

In some embodiments, the polynucleotides disclosed herein can comprise cations or anions. In one embodiment, the formulation includes metal cations such as, but not limited to, Zn2+, Ca2+, Cu2+, Mg+, and combinations thereof. As a non-limiting example, the formulation can include a polymer and a polynucleotide complexed with a metal cation (see, for example, US Patent Nos. 6,265,389 and 6,555,525, each of which is hereby incorporated by reference in its entirety).

In some embodiments, the polynucleotides may be formulated into nanoparticles and/or microparticles. These nanoparticles and/or microparticles can be formed into any size shape and chemistry. As an example, nanoparticles and/or microparticles can be made using PRINT^{®} technology by LIQUIDA TECHNOLOGIES. RTM (Morrisville, N.C.) (see International Patent Application Publication No. 2007024323, which is incorporated herein by reference in its entirety).

In some embodiments, the polynucleotides may be formulated in nanojacket and nanoliposomes by Keystone Nano (State College, Pennsylvania). The nanojacket is made from compounds found naturally in the body, including calcium, phosphate, or compounds that also contain small amounts of silicate. The nanojacket can range in size from 5 to 50 nm and can be used to deliver hydrophilic and hydrophobic compounds such as, but not limited to, polynucleotides, primary constructs and/or polynucleotides. The nanoliposomes are made from lipids, including, but not limited to, lipids that occur naturally in the body. The nanoliposomes can range in size from 60-80 nm and can be used to deliver hydrophilic and hydrophobic compounds such as, but not limited to, polynucleotides, matrix constructs and/or polynucleotides. In one mode, the polynucleotides disclosed herein are formulated in, but not limited to, nanoliposomes, such as ceramide nanoliposomes.

In some embodiments, the polynucleotide, such as DNA, also contains a promoter operably linked to a nucleic acid sequence encoding a therapeutic protein. In certain embodiments, the promoter is a tissue-specific promoter that drives gene expression in specific tissues. In one embodiment, the tissue-specific promoter is a liver-specific enhancer/promoter from Serpina 1 and/or the TTR promoter. In other embodiments, the promoter is the CMV promoter. In other embodiments, the promoter is the ubiquitin C promoter.

In some embodiments, the polynucleotide also includes a "locus targeting nucleic acid sequence." A locus-targeting sequence allows a polynucleotide encoding a therapeutic protein to integrate into the genome of a recipient host cell. In some embodiments, the locus targeting sequence comprises flanking homology arms to allow homologous recombination. In some embodiments, the locus targeting sequence comprises a guide RNA sequence and a type II Cas enzyme (i.e., CRISPR-Cas9 method) that drives integration. In some embodiments, the locus targeting sequence comprises a guide zinc finger nuclease (ZFN) recognition sequence to drive integration. In some embodiments, the locus targeting sequence comprises a transcription activator-like effector nuclease (TALEN) recognition sequence to drive integration. In still other embodiments, the locus targeting sequence comprises a single residue-to-nucleotide code used by the BuD-derived nuclease to drive integration.

Furthermore, in one embodiment, cell therapy utilizing cells comprising the composition of the present disclosure, includes therapeutic methods of transplanting retinal cells comprising the composition of the present disclosure. In one embodiment, cells comprising the composition of the present disclosure may be administered with additional agents in addition to the cells. As for such additional agents, agents commonly used in ophthalmic therapy (such as steroids, antibiotics, antibacterials, and NSAIDs) may be used. Such additional agents may be included as pharmaceuticals in the cell pharmaceuticals of the present disclosure, or may be provided in a separately administered form. When provided or administered separately, such additional agents are provided as a kit or a combination agent. When used as a kit or a combination agent, an attached document or the like describing the method of use may be combined therewith.

In a preferred embodiment, the present disclosure is preferably administered to a subject prior to or immediately after onset of a disease, disorder or symptom as described above, for example, within 1 year, and preferably, but not limited to, within 6 months, 3 months, or 1 month, after the onset (for example, the appearance of subjective symptoms).

In one particular embodiment, the composition of the present disclosure is administered once during the treatment period. As described in the Examples, it has been confirmed that the composition of the present disclosure exerts its effect by administering it once, and it is considered that patient compliance is good.

In one particular embodiment, in the compositions of the present disclosure, the amount of vector used is a unit dose of 0.1 × 10¹¹ to 10 × 10¹¹ vg/eye. For example, the lower limit may be 0.01 ×10¹¹ vg/eye, 0.02 × 10¹¹ vg/eye, 0.03 × 10¹¹ vg/eye, 0.04 × 10¹¹ vg/eye, 0.05 × 10¹¹ vg/eye, 0.06 × 10¹¹ vg/eye, 0.07 × 10¹¹ vg/eye, 0.08 × 10¹¹ vg/eye, 0.09 × 10¹¹ vg/eye, 0.1 × 10¹¹ vg/eye, 0.2 × 10¹¹ vg/eye, 0.3 × 10¹¹ vg/eye, 0.4 × 10¹¹ vg/eye, 0.5 × 10¹¹ vg/eye or the like, and the upper limit may be 2 × 10¹¹ vg/eye, 3 × 10¹¹ vg/eye, 4 × 10¹¹ vg/eye, 5 × 10¹¹ vg/eye, 6 × 10¹¹ vg/eye, 7 × 10¹¹ vg/eye, 8 × 10¹¹ vg/eye, 9 × 10¹¹ vg/eye, 10 × 10¹¹ vg/eye, 15 × 10¹¹ vg/eye, 20 × 10¹¹ vg/eye, 30 × 10¹¹ vg/eye, 40 × 10¹¹ vg/eye, 50 × 10¹¹ vg/eye, or the like.

### (Combinations)

In one aspect of the present disclosure, of the pharmaceutical, composition or compound for preventing, or suppressing the progress of, a disease, disorder or symptom associated with an endoplasmic reticulum stress; the pharmaceutical, composition or compound for ameliorating a rhodopsin transport disorder; the pharmaceutical, composition or compound for suppressing apoptosis; and the pharmaceutical, composition or compound for preventing, or suppressing the progress of, a disease, disorder or symptom associated with All-trans-retinal-related disorder, two or more can be used in combination. In one embodiment, when each of the above uses is used in combination, the same active ingredient can be used for the combined use, or different active ingredients can be used in combination.

### (General Technology)

The molecular biology approaches, biochemical approaches, and microbiological approaches as used herein are those well known and commonly practiced in the art, which are described in documents such as Current Protocols in Molecular Biology (http://onlinelibrary.wiley.com/book/10.1002/0471142727) and Molecular Cloning: A Laboratory Manual (Fourth Edition) (http://www.molecularcloning.com), the relevant parts (which may be all the parts) of which are incorporated herein by reference.

As used herein, the term, "or", is used when "at least one or more" of the matters listed in the sentences can be employed. When explicitly described herein as "within the range of two of the values", the range also includes the two values themselves.

Reference literatures such as scientific literatures, patents, and patent applications cited herein are incorporated herein by reference to the same extent that the entirety of each document is specifically described.

As described above, the present disclosure has been explained while showing preferred embodiments to facilitate understanding. The present disclosure is explained hereinafter based on Examples. The above explanation and the following Examples are not provided to limit the present disclosure, but for the sole purpose of exemplification. Thus, the scope of the present disclosure is not limited to the embodiments or the Examples specifically described herein and is limited only by the scope of claims.

### [Examples]

Examples will be described hereinafter. The handling of animals used in the following examples was carried out, if necessary, based on the Declaration of Helsinki, in compliance with the standards and other relevant ethical standards and guidelines as stipulated by Keio University and others. As for reagents, while those specifically described in Examples were used, these reagents can be substituted by equivalent products of other manufacturers (such as, Sigma-Aldrich, Wako Pure Chemical, Nacalai, R & D Systems and USCN Life Science Inc.). All animal experiments were performed in accordance with protocols approved by the Institutional Animal Care and Use Committee of Keio University School of Medicine.

### (Example 1: Vector Preparation)

The DNA encoding the chimeric protein (GR/BvRh) was produced as follows. The sequence corresponding to the 137th to 145th amino acids from the N-terminal, which corresponds to the second loop on the cytoplasm side of *Gloeobacter violaceus* Rhodopsin (GR) (SEQ ID NO: 14), was substituted by the sequence corresponding to the 137th to 145th amino acids of bovine rhodopsin (BvRh) (SEQ ID NO: 12), and the sequence corresponding to 198th to 206th amino acids from the N-terminal, which corresponds to the third loop on the cytoplasm side of GR, was substituted by the sequence corresponding to the 225th to 252nd amino acids of the bovine rhodopsin. Furthermore, DNA encoding a chimeric protein, in which glutamic acid, or the 132nd amino acid of GR, was substituted by glutamine, was inserted into the pCDNA3.1 vector. Alternatively, nucleic acids having the base sequence set forth in SEQ ID NO: 23 were generated and inserted, as the DNA encoding the chimeric protein, into the pCDNA3.1 vector HindIII/XbaI site. The base sequence set forth in SEQ ID NO: 23 was generated as follows: the sequence corresponding to the 137th to 145th amino acids from the N-terminal, which corresponds to the second loop on the cytoplasm side of *Gloeobacter violaceus* Rhodopsin (GR) (SEQ ID NO: 14), was substituted by the base sequence set forth in SEQ ID NO: 18 corresponding to the second loop of bovine rhodopsin (BvRh) (SEQ ID NO: 12) (the encoding of the amino acid sequence set forth in SEQ ID NO: 19), and the sequence corresponding to 198th to 206th amino acids from the N-terminal, which corresponds to the third loop on the cytoplasm side of GR, was substituted by the base sequence set forth in SEQ ID NO: 20 corresponding to the third loop of the bovine rhodopsin (the encoding of the amino acid sequence set forth in SEQ ID NO: 22), thereby producing the base sequence. In addition, the base sequence set forth in SEQ ID NO: 3 was prepared by changing part of the base without changing the amino acids to be encoded. Specifically, the preparation was performed by mutating the nucleic acids encoding the amino acids 6, 9-13, 15, 16, 18-22, 27-29, 31-36, 39, 40, 43, 45, 48, 50, 51, 53-55, 58, 59, 61, 65-73, 75-84, 86, 88, 89, 93, 97, 98, 100, 101, 104, 106-108, 110, 112, 114, 115, 122, 123, 125, 128, 131, 133, 139, 143, 145, 146, 155, 157, 162, 165, 167, 169-171, 174, 176, 179, 182, 183, 186-189, 193-198, 204, 205, 207, 209, 212, 215, 216, 218-220, 224, 225, 227, 228, 230, 231, 233-235, 238, 240, 242, 243, 246, 247, 249, 251, 253-255, 257-259, 261-264, 266-270, 272, 273, 275, 276, 279, 281-287, 289-291, 296-299, 302-305, 307-316, 318, 319, and 321-330, without changing the amino acids to be encoded. The production of the mutant was conducted using the quick change method. Note that the sequence portion adopted for bovine rhodopsin completely matches the amino acid sequence of human rhodopsin, and thus, the sequence portion may be referred to as human rhodopsin without any problem.

The EGFP or GR/BvRh gene was subcloned into the AAV2 shuttle plasmid, and AAV2-CAGGS-EGFP-WPRE-pA (vector for the expression of EGFP) and AAV2-CAGGS-GR/BvRh-WPRE-pA (vector for the expression of chimeric protein) were produced as virus expression constructs. Viral vector packaging was performed by transfecting HEK293 cells with three types of plasmids, vector plasmid, AAV vector plasmid and adenovirus helper plasmid; and the cesium chloride method was used to purify the viral vector. Note that, with regard to the vector, the "ITR" is an abbreviation for "Inverted Terminal Repeat". The "CAGGS" is a sequence of regions of the CAG promoter. The "WPRE" is an abbreviation for "woodchuck hepatitis virus post-transcriptional regulatory element". The "pA" means a peptide tag. The "EGFP" is an abbreviation for "enhanced green fluorescent protein". The construct thus obtained is called GHCR (Gloeobacter-human rhodopsin Chimeras) in the following Examples.

### (Example 2: Restoration of Photic Evocation Activity from the Retina by GHCR)

Viral vectors (rAAV-DJ and rAAV-2) containing the GHCR coding sequence under the control of the CAGGS promoter (CAGGS-GHCR; Fig. **1A****)** were injected into the vitreous of rd1 mice at 10 weeks. The AAV-DJ vector was adopted for more efficient and widespread gene transfer, and AAV-2 was adopted as a benchmark already clinically applied. Retinas were obtained after 2-4 months. Expression of a reporter gene (EGFP) appeared throughout the retina and in both the ganglion cell layer (GCL) and inner nuclear layer (INL) (Fig. **1B****).** To assess the function of GHCR ectopically induced in the mouse retina, a multi-electrode array (MEA) test was performed that can record extracellular potentials of RGCs. As a result of photoreceptor degeneration, control retinas without treatment showed no response from RGCs detected by MEA (Fig. **1C****).** In contrast, the treated retina exhibited a clear photic evocation response, which was obtained with white LED illumination up to 10¹⁴ photons/cm²/s (Fig. **1D****).**

### (Example 3: Wavelength Sensitivity Evaluation)

Next, to create a stable vector for human gene therapy, a codon-optimized version of GHCR (coGHCR) was designed to fuse the ER2 endoplasmic reticulum trafficking signal to the C-terminus to enhance gene expression levels. As a result, a photoresponse up to 10¹³ photons/cm²/s, which was not observed before codon optimization, was confirmed (Figs. **1E** and **1F****).** Furthermore, the number of firing cells per unit area was also significantly increased (Fig. 1G). The rhodopsin exhibits selectivity for Gi/o class G proteins in heterologous expression. Therefore, Gi/o activation was measured by a homogeneous time-resolved fluorescence (HTRF) cAMP assay, which showed a 5-fold increase in activation (Fig. **1G****).** As a result of measuring the spectral sensitivity by wavelength-specific stimulation, the maximum sensitivity was about 500 nm, and a photoresponse was obtained even with stimulation of 600 nm or more (Fig. **1H****).**

### (Example 4: Evaluation of Visual Evoked Potential)

To investigate whether light received in the retina is transmitted to the visual cortex, visual evoked potentials (VEP) from the visual cortex were examined (Fig. **2A****).** In these experiments, used were: rd1 mice treated with AAV-DJ-CAGGS-GHCR, AAV-DJ-CAGGS-coGHCR in both eyes; and a control EGFP virus (AAV-DJ-CAGGS-EGFP). No significant VEP was detected in either control or GHCR-treated mice. In contrast, VEP was observed in coGHCR-treated mice (Fig. **2B****).** In response to a light stimulus of 3 cds/m², the mean VEP amplitude of coGHCR-treated mice was significantly higher (56.4 µν; n=8) than GHCR-treated mice (22.1 µV; n=8) at the same level as the control mice (17.9 µV; n=6) (Fig. **2C****)**. From this result, subsequent experiments used coGHCR.

### (Example 5: Evaluation of Light-Dark Recognition Function)

Next, a light-dark transition test (LDT) was performed to investigate whether ectopic expression of coGHCR in the degenerating retina leads to behavioral changes following visual recovery (Fig. **3A****).** Rodents are nocturnal and feel anxious in bright environments, so they tend to stay in dark places according to their visual function. On the other hand, the blind animals spent approximately half the time in a bright place and half in a dark place. The coGHCR-treated mice significantly reduced their time spent in the bright place compared to the untreated rd1 mutant mice (Fig. **3B****),** which indicated that visual recovery in behavior was confirmed. Furthermore, in order to compare the differences in the effects, the rd1 mice were treated with chimeric rhodopsin (AAV-6-CAGGS-coGHCR), microbial rhodopsin (AAV-6-CAGGS-ChrimsonR26), animal rhodopsin (AAV-6-CAGGS-human rhodopsin), and a control EGFP virus (AAV-6-CAGGS-EGFP). At illuminance of 3,000 lux, a significant change in time spent in the bright place was observed in the coGHCR-treated mice (0.32; n=6) compared to the control mice (0.50; n=8) (Fig. **3C****).** A similar trend was observed in the ChrimsonR-treated mice (0.36; n=6). On the other hand, no obvious changes were observed in the human rhodopsin-treated mice (0.48; n=6). Conversely, when the experiment was conducted with illumination of 10 lux, the human rhodopsin-treated mice showed a significant change in time spent (0.40; n=6), whereas the ChrimsonR-treated mice showed no apparent change (0.55; n=6) (Fig. **3D****).** The GHCR-treated mice showed significant changes even at 10 lux illumination (0.40; n=6).

### (Example 6: Evaluation of Object Recognition Function)

Since the LDT only measures light-dark discrimination, a visual recognition test (VRT) was performed to assess whether chimeric rhodopsin improves an object recognition function. Mice use vision for cognitive functions and are known to be attracted to fighting videos. Mice were tested with a place selection device using a tablet displaying a fighting video (Fig. **3E****).** The proportion of time spent in the fighting video playing area to the time spent in the control area (an empty cage at the same illuminance) during 15 minutes was measured. As a result, the time spent in the fighting video playing area was significantly increased with the coGHCR-treated (AAV-DJ-CAGGS-coGHCR) mice (0.55, n=33) compared to the control (untreated rd1) mice (0.50, n=30). On the other hand, no significant changes were observed in AAV2-treated (AAV-2-CAGGS-coGHCR) mice (0.48, n=30) or microbial rhodopsin-treated (AAV-DJ-CAGGS-C1V1) mice (0.49, n=20) (Fig. **3F****).**

### (Example 7: Protective Effect of GHCR against Retinal Degeneration)

Model mice of retinal degeneration with P23H RHO mutation were used, which are referred to as P23H mice. P23H mice were used for assessment of protective effects because retinal degeneration progresses more slowly than rd1 mice. AAV DJ-CAGGS-coGHCR and controls (AAV DJ-CAGGS-EGFP) were delivered subretinally, targeting the outer retina of postnatal (PND) day 0-1 mice. As such, the protective effect was quantified morphologically and electrophysiologically.

Subretinal injection of the AAV-DJ vector efficiently induced the gene in the outer retina of mice (Fig. **4A****).** By optical coherence tomography (OCT), it was found that the outer retina thickness (ORT; thickness from outer nuclear layer (ONL) to rod outer segment (ROS)) of coGHCR-treated mice (50.0 um; n=13) was significantly thicker than that of PND30 control mice (42.7 um; n=10) (Figs. **4B** and **4C****).** In the subsequent follow-up studies, the ORT in coGHCR-treated mice was also significantly thicker until PND50.

As a result of electroretinogram (ERG) measurement, coGHCR mice exhibited greater amplitudes in all rod, mixed, and cone responses at PND30 (141.2 µV, 271.4 µV, and 159.0 µV, respectively; n=9) than control mice (70.4 µV, 158.7 µV, and 99.1 µV, respectively; n=14) (Figs. **4D** and **4E****).** As a result of observing changes over time, all amplitudes in control mice gradually decreased, while all amplitudes in coGHCR-treated mice continued to increase until PND42. Thereafter, the amplitudes of coGHCR-treated mice gradually decreased and were significantly higher than those of control mice until PND66.

### (Example 8: Apoptosis Detection)

Terminal deoxynucleotidyl transferase dUTP nick end labeling (TUNEL) was also performed to detect retinal apoptosis. The number of TUNEL-positive cells in the retina of coGHCR-treated mice (289.7 cells; n=3) was significantly lower at PND31 compared to that of the control mice (67.3 cells; n=3) (Figs. **5A, 5B,** and **5C****).**

### (Example 9: Evaluation of ER stress)

Cross-sectional transmission electron microscopy (TEM) images of PND31 mice were also obtained. Consistent with the OCT results, ONL (Fig. **5D****)** and ROS (Fig. **5E****)** in coGHCR-treated mice were preserved compared to those in controls, and the rod outer segment structure was not so disturbed (Fig. **5F**). Furthermore, coGHCR-treated mice had reduced endoplasmic reticulum (ER) swelling indicative of ER stress (Fig. **5G****).**

To further evaluate ER stress, Western blotting was performed. ATF4, an ER stress marker, showed a significant decrease, and BiP, PERK, ATF6, and pIRE1 also showed a decreasing trend at PND14.

### (Example 10: Evaluation with Other Opsins 1 - Protective Effect against Retinal Degeneration)

In order to confirm the protective effect against retinal degeneration by other opsins, P23H mice are used in the same manner as in Example 7, where AAV DJ-CAGGS-GRsm/m1AchR-3rd-Venus (chimeric rhodopsin of *Gloeobacter* rhodopsin and muscarinic receptor, followed by gene encoding fluorescent protein Venus), AAV DJ-CAGGS-GRsm/A1R-3rd-Venus (chimeric rhodopsin of Gloeobacter rhodopsin and adenosine receptor), AAV DJ-CAGGS-EGFP-GtACR2sm/BvRh-double (chimera of *Guillardia theta's* anion-conducting channelrhodopsins (another microbial rhodopsin) and bovine (human) rhodopsin, with fluorescent protein EGFP in front), AAV 6-CAGGS-ChrimsonR-tdT (microbial rhodopsin and fluorescent protein tdT), and a control (AAV DJ-CAGGS-EGFP) are delivered subretinally, targeting the outer retina of postnatal day (PND) 0-1 mice. As such, the protective effect is quantified morphologically and electrophysiologically.

Subretinal injection of the AAV-DJ vector confirmed efficient induction of genes in the outer retina of mice; and optical coherence tomography (OCT) confirmed that the outer retina thickness (ORT; thickness from outer nuclear layer (ONL) to rod outer segment (ROS)) of: AAV DJ-CAGGS-GRsm/m1AchR-3rd-Venus injected mice; AAV DJ-CAGGS-GRsm/A1R-3rd-Venus injected mice; AAV DJ-CAGGS-EGFP-GtACR2sm/BvRh-double injected mice; and AAV 6-CAGGS-ChrimsonR-tdT injected mice, was thicker than that of control mice at PND30.

The thickness of the outer retina at PND30 after administration of each vector is shown in Figure **6** (n=6 each). As a result, compared to AAV DJ-CAGGS-EGFP-injected mice (52.4±2.42 um) as a control, the outer retinal thickness was greater in all of: AAV DJ-CAGGS-GRsm/m1AchR-3rd-Venus injected mice (58.2±1.35 µm); AAV 6-CAGGS-ChrimsonR-tdT injected mice (58.4±1.47 µm); AAV DJ-CAGGS-GRsm/A1R-3rd-Venus injected mice (54.8±0.58 µm); and AAV DJ-CAGGS-EGFP-GtACR2sm/BvRh-double injected mice (57.8±1.36 µm). *p<0.05, One-way analysis of variance and Tukey's multiple comparison test.

Electroretinogram (ERG) measurements were performed to confirm that AAV DJ-CAGGS-GRsm/m1AchR-3rd-Venus injected mice, AAV DJ-CAGGS-GRsm/A1R-3rd-Venus injected mice, AAV DJ-CAGGS-EGFP-GtACR2sm/BvRh-double injected mice, and AAV 6-CAGGS-ChrimsonR-tdT injected mice exhibited greater amplitudes at PND30 than control mice for all rod, mixed, and cone responses.

Electroretinogram amplitudes at PND30 after each vector administration are shown in Figures **7** to **9** (n=6 each). As a result, compared to AAV DJ-CAGGS-EGFP-injected mice (rod response 161.5±19.7 µV, mixed response 382.5±74.4 µν, and cone response 183.8±33.9 µV) as a control, many of: AAV DJ-CAGGS-GRsm/m1AchR-3rd-Venus injected mice (rod response 162.6±25.9 µV, mixed response 442.8±16.8 µν, cone response 197.1±28.6 µV); AAV 6-CAGGS-ChrimsonR-tdT injected mice (rod response 147.2±49.9 µV, mixed response 596.8±8.9 µν, cone response 308.1±33.0 µV); AAV DJ-CAGGS-GRsm/A1R-3rd-Venus injected mice (rod response 176.0±24.8 µV, mixed response 420.7±22.3 µν, cone response 204.0129.8 µV); and AAV DJ-CAGGS-EGFP-GtACR2sm/BvRh-double injected mice (rod response 223.0±25.7 µV, mixed response 518.5±18.4 µν, cone response 249.2±16.4 µV), exceeded the responses of the control. *p<0.05, One-way analysis of variance and Tukey's multiple comparison test.

Furthermore, while changes over time are observed, the following is confirmed: all amplitudes in control mice gradually decrease, while all amplitudes in AAV DJ-CAGGS-EGFP-GtACR2sm/BvRh-double injected mice, and AAV 6-CAGGS-ChrimsonR-tdT injected mice continue to increase until around PND42. Subsequently, the following is confirmed: the amplitudes of AAV DJ-CAGGS-GRsm/m1AchR-3rd-Venus injected mice, AAV DJ-CAGGS-GRsm/A1R-3rd-Venus injected mice, AAV DJ-CAGGS-EGFP-GtACR2sm/BvRh-double injected mice, and AAV 6-CAGGS-ChrimsonR-tdT injected mice gradually decrease and are significantly higher than that of control mice until around PND66.

### (Example 11: Evaluation with Other Opsins 2 - Apoptosis Detection)

Furthermore, in order to detect retinal apoptosis, terminal deoxynucleotidyl transferase dUTP nick end labeling (TUNEL) is performed. It is confirmed that the number of TUNEL-positive cells in the retina of AAV DJ-CAGGS-GRsm/m1AchR-3rd-Venus injected mice, AAV DJ-CAGGS-GRsm/A1R-3rd-Venus injected mice, AAV DJ-CAGGS-EGFP-GtACR2sm/BvRh-double injected mice, and AAV 6-CAGGS-ChrimsonR-tdT injected mice, is significantly lower than that of control mice at about PND31.

### (Example 12: Evaluation with Other Opsins 3 - Evaluation of ER stress)

Furthermore, transmission electron microscopy (TEM) images of retinal sections of mice of around PND31 were acquired. It was confirmed that: similar to the OCT results, ONL and ROS in AAV DJ-CAGGS-GRsm/m1AchR-3rd-Venus injected mice, AAV DJ-CAGGS-GRsm/A1R-3rd-Venus injected mice, AAV DJ-CAGGS-EGFP-GtACR2sm/BvRh-double injected mice, and AAV 6-CAGGS-ChrimsonR-tdT injected mice were preserved compared to those in controls; and the rod outer segment structures were less disturbed. Furthermore, it was confirmed that: AAV DJ-CAGGS-GRsm/m1AchR-3rd-Venus injected mice, AAV DJ-CAGGS-GRsm/A1R-3rd-Venus injected mice, AAV DJ-CAGGS-EGFP-GtACR2sm/BvRh-double injected mice, and AAV 6-CAGGS-ChrimsonR-tdT injected mice had reduced swelling of the endoplasmic reticulum (ER) indicative of ER stress.

Western blotting was performed to further evaluate ER stress. It was confirmed that: ATF4, an ER stress marker, showed a significant decrease; and BiP, PERK, ATF6, and pIRE1 also showed a decreasing trend at PND30.

The results of Western blotting at PND30 after administration of each vector are shown in Figure **10** (n=3 each). As a result, it was confirmed that all markers tended to decrease in AAV DJ-CAGGS-GRsm/m1AchR-3rd-Venus-administered mice, but consistent results could not be confirmed in the other administered mice.

### (Example 13: Preventive Effect against Diseases other than Retinitis Pigmentosa)

The following are used in experiments: diabetes model mice obtained by administering streptozotocin to wild-type mice; laser-induced CNV model mice produced by inducing pathological angiogenesis by irradiating the retina of a wild-type mouse known as a model of age-related macular degeneration, with a laser; photodamaged model mice obtained by inducing degeneration by irradiating wild-type mice known as another age-related macular degeneration model, with strong light; myopia model mice induced by shielding the eyes of wild-type mice or attaching strong concave lenses thereto; and NMDA (N-methyl-D-aspartate)-administered glaucoma model mice. AAV DJ-CAGGS-coGHCR and control (AAV DJ-CAGGS-EGFP) are transduced into the retina by intravitreal or subretinal injection and protective effects are quantified.

It is confirmed that intravitreal or subretinal injection of AAV-DJ vectors efficiently induces genes in the outer retina of mice; and it is also confirmed that the retinal thickness of AAV DJ-CAGGS-coGHCR-injected mice is thicker than that of controls, by optical coherence tomography (OCT).

Electroretinogram (ERG) measurements are performed to confirm that AAV DJ-CAGGS-coGHCR-injected mice exhibit greater amplitudes than control mice in all rod, mixed, and cone responses.

Furthermore, in order to detect retinal apoptosis, terminal deoxynucleotidyl transferase dUTP nick end labeling (TUNEL) is performed. It is confirmed that the number of TUNEL-positive cells in the retina of AAV DJ-CAGGS-coGHCR-injected mice is significantly lower than that of control mice.

Furthermore, transmission electron microscopy (TEM) images of retinal sections of mice are acquired. It is confirmed that: similar to the OCT results, ONL and ROS in AAV DJ-CAGGS-coGHCR injected mice are preserved compared to those in controls; and the rod outer segment structures are less disturbed. Furthermore, it is confirmed that AAV DJ-CAGGS-coGHCR injected mice have reduced swelling of the endoplasmic reticulum (ER) indicative of ER stress.

Western blotting was performed to further evaluate ER stress. It was confirmed that: ATF4, an ER stress marker, showed a significant decrease; and BiP, PERK, ATF6, and pIRE1 also showed a decreasing trend at around PND14.

The results of Western blotting on the retina of glaucoma and diabetes-induced model mice in which GHCR was transfected into the retina (n=3 each) are shown in Figure 11. As a result, a decrease in ATF4 was observed in glaucoma and diabetes-induced models. In addition, reductions in XBP1 and pIRE1 were observed in the diabetes induction model.

By ligating the sciatic nerve of wild-type mice, sciatic nerve neuropathy model mice are produced and used for experiments. AAV DJ-CAGGS-coGHCR and a control (AAV DJ-CAGGS-EGFP) are transfected into the sciatic nerve to quantify the protective effect.

Furthermore, in order to detect sciatic nerve apoptosis, terminal deoxynucleotidyl transferase dUTP nick end labeling (TUNEL) is performed. It is confirmed that the number of TUNEL-positive cells in the sciatic nerve of AAV DJ-CAGGS-coGHCR-injected mice is significantly lower than that of control mice.

Furthermore, transmission electron microscopy (TEM) images of mouse sciatic nerve sections are acquired. It is confirmed that AAV DJ-CAGGS-coGHCR injected mice have reduced swelling of the endoplasmic reticulum (ER) indicative of ER stress.

Western blotting is performed to further evaluate ER stress. It is confirmed that: the ER stress marker ATF4 shows a significant decrease; and BiP, PERK, ATF6, and pIRE1 show a decrease.

### (Example 14: Effect of Suppressing Progress of Disuse Atrophy of Retinal Thickness)

AAV DJ-CAGGS-coGHCR and a control (AAV DJ-CAGGS-EGFP) are transfected into the retina of rd1 mice by intravitreal or subretinal injection, and the protective effect is quantified.

It is confirmed that the gene is efficiently induced in the outer retina of mice by intravitreal or subretinal injection of AAV-DJ vectors; and it is also confirmed that the retinal thickness (inner retinal layer thickness) of AAV DJ-CAGGS-coGHCR-injected mice is thicker than that of controls, by optical coherence tomography (OCT) or by the observation of sections with an optical microscope.

Furthermore, in order to detect retinal apoptosis, terminal deoxynucleotidyl transferase dUTP nick end labeling (TUNEL) is performed. It is confirmed that the number of TUNEL-positive cells in the retina of AAV DJ-CAGGS-coGHCR-injected mice is significantly lower than that of control mice.

### (Example 15: Evaluation 1 with Light Absorbing Substance - Protective Effect against Retinal Degeneration)

In order to confirm the photoreception of light absorbing substances, the following experiments are performed using carbon black (carbon fine particles), melanin, carotenoids, polyphenols, retinoids, photoactivated adenylate cyclase, acrylamide-azobenzene-quaternaryammonium, diethylammonium substituted for the acrylamide, and other azobenzene compounds, and diarylethene.

In this example, in order to confirm the protective effect of light absorbing substances against retinal degeneration, and in the same manner as in Example 7, P23H mice are used. A solution containing carbon black (carbon fine particles), melanin, carotenoids, polyphenols, retinoids, azobenzene compounds, diarylethene, and physiological saline as a control are intravitreally administered to mice aged 2 weeks or older, and the protective effect is quantified morphologically and electrophysiologically.

By optical coherence tomography (OCT), it is confirmed that the outer retina thickness (ORT; thickness from outer nuclear layer (ONL) to rod outer segment (ROS)) of: carbon black (carbon fine particles)-administered mice; melanin-administered mice; carotenoid-administered mice; polyphenol-administered mice; and retinoid-administered mice, is thicker than that of control mice at PND30 or PND50.

Electroretinogram (ERG) measurements are performed to confirm that the carbon black (carbon fine particles)-administered mice; melanin-administered mice; carotenoid-administered mice; polyphenol-administered mice; and retinoid-administered mice exhibit greater amplitudes at PND30 than control mice for all rod, mixed, and cone responses. Subsequently, the following is confirmed: the amplitudes of carbon black (carbon fine particles)-administered mice, melanin-administered mice, carotenoid-administered mice, polyphenol-administered mice, retinoid-administered mice, azobenzene compound-administered mice, and diarylethene-administered mice gradually decrease and are significantly higher than that of control mice until around PND66.

### (Example 16: Evaluation with Other Opsins 2 - Apoptosis Detection)

Furthermore, in order to detect retinal apoptosis, terminal deoxynucleotidyl transferase dUTP nick end labeling (TUNEL) is performed. It is confirmed that the number of TUNEL-positive cells in the retina of carbon black (carbon fine particles)-administered mice, melanin-administered mice, carotenoid-administered mice, polyphenol-administered mice, retinoid-administered mice, azobenzene compound-administered mice, and diarylethene-administered mice, is significantly lower than that of control mice at about PND31.

### (Example 17: Evaluation with Other Opsins 3 - Evaluation of ER stress)

Furthermore, transmission electron microscopy (TEM) images of retinal sections of mice of around PND31 are acquired. It is confirmed that: similar to the OCT results, ONL and ROS in carbon black (carbon fine particles)-administered mice, melanin-administered mice, carotenoid-administered mice, polyphenol-administered mice, and retinoid-administered mice are preserved compared to those in controls; and the rod outer segment structures are less disturbed. Furthermore, it is confirmed that: carbon black (carbon fine particles)-administered mice, melanin-administered mice, carotenoid-administered mice, polyphenol-administered mice, retinoid-administered mice, azobenzene compound-administered mice, and diarylethene-administered mice have reduced swelling of the endoplasmic reticulum (ER) indicative of ER stress.

Western blotting is performed to further evaluate ER stress. It is confirmed that: ATF4, an ER stress marker, shows a significant decrease; and BiP, PERK, ATF6, and pIRE1 also show a decreasing trend at around PND14.

In order to confirm the protective effect of light absorbing substances against retinal degeneration, and in the same manner as in Example 7, P23H mice are used. AAV DJ-CAGGS-photoactivated adenylate cyclase and a control (AAV DJ-CAGGS-EGFP) are delivered subretinally, targeting the outer retina of postnatal day (PND) 0-1 mice. As such, the protective effect is quantified morphologically and electrophysiologically.

It is confirmed that the subretinal injection of the AAV-DJ vector efficiently induces the genes into the outer retina of mice. Furthermore, by optical coherence tomography (OCT), it is confirmed that the outer retina thickness (ORT; thickness from outer nuclear layer (ONL) to rod outer segment (ROS)) of AAV DJ-CAGGS-photoactivated adenylate cyclase-injected mice is thicker than that of control mice at PND30 or PND50.

Electroretinogram (ERG) measurements are performed to confirm that the AAV DJ-CAGGS-photoactivated adenylate cyclase-injected mice exhibit greater amplitudes at PND30 than control mice for all rod, mixed, and cone responses.

### (Example 18: Evaluation with Other Opsins 2 - Apoptosis Detection)

Furthermore, in order to detect retinal apoptosis, terminal deoxynucleotidyl transferase dUTP nick end labeling (TUNEL) is performed. It is confirmed that the number of TUNEL-positive cells in the retina of AAV DJ-CAGGS-photoactivated adenylate cyclase-injected mice is significantly lower than that of control mice at about PND31.

### (Example 19: Evaluation with Other Opsins 3 - Evaluation of ER stress)

Furthermore, transmission electron microscopy (TEM) images of retinal sections of mice of around PND31 are acquired. It is confirmed that: similar to the OCT results, ONL and ROS in AAV DJ-CAGGS-photoactivated adenylate cyclase-injected mice are preserved compared to those in controls; and the rod outer segment structures are less disturbed. Furthermore, it is confirmed that: AAV DJ-CAGGS-photoactivated adenylate cyclase-injected mice, AV DJ-CAGGS-azobenzene compound-injected mice, and AAV DJ-CAGGS-diarylethene-injected mice have reduced swelling of the endoplasmic reticulum (ER) indicative of ER stress.

Western blotting is performed to further evaluate ER stress. It is confirmed that: ATF4, an ER stress marker, shows a significant decrease; and BiP, PERK, ATF6, and pIRE1 also show a decreasing trend at around PND14.

In order to confirm the neural activity-imparting activity, photoactivated adenylate cyclase, azobenzene derivatives such as acrylamide-azobenzene-quaternary ammonium and diethylammonium substituted for the acrylamide, diarylethene, etc., are used, and devices such as artificial retinas represented by Argus II are used, to conduct the following experiments.

### (Example 20: Effect of Suppressing Progress of Disuse Atrophy of Retinal Thickness)

AAV DJ-CAGGS-photoactivated adenylate cyclase and a control (AAV DJ-CAGGS-EGFP) are transfected into the retina of rd1 mice by intravitreal or subretinal injection, and the protective effect is quantified.

It is confirmed that the gene is efficiently induced in the outer retina of mice by intravitreal or subretinal injection of AAV-DJ vectors; and it is also confirmed that the retinal thickness (inner retinal layer thickness) of AAV DJ-CAGGS-photoactivated adenylate cyclase-injected mice is thicker than that of controls, by optical coherence tomography (OCT) or by the observation of sections with an optical microscope.

Furthermore, in order to detect retinal apoptosis, terminal deoxynucleotidyl transferase dUTP nick end labeling (TUNEL) is performed. It is confirmed that the number of TUNEL-positive cells in the retina of AAV DJ-CAGGS-photoactivated adenylate cyclase-injected mice is significantly lower than that of control mice.

### (Example 21: Effect of Suppressing Progress of Disuse Atrophy of Retinal Thickness)

An azobenzene derivative and/or diarylethene are delivered to the retina of rd1 mice by intravitreal injection, and the protective effect is quantified.

It is confirmed that the retinal thickness (inner retinal layer thickness) of azobenzene derivative and/or diarylethene-injected mice is thicker than that of controls by optical coherence tomography (OCT) or by the observation of sections with an optical microscope.

Furthermore, in order to detect retinal apoptosis, terminal deoxynucleotidyl transferase dUTP nick end labeling (TUNEL) is performed. It is confirmed that the number of TUNEL-positive cells in the retina of azobenzene derivative and/or diarylethene-injected mice is significantly lower than that of control mice.

### (Example 22: Effect of Suppressing Progress of Disuse Atrophy of Retinal Thickness)

Artificial retina implantation surgery is performed on rd1 mice. The protective effect is quantified in a light-stimulated group and a non-light-stimulated control group.

It is confirmed that the retinal thickness (inner retinal layer thickness) of the artificial retina light-stimulated group is thicker than that of controls by optical coherence tomography (OCT) or by the observation of sections with an optical microscope.

Furthermore, in order to detect retinal apoptosis, terminal deoxynucleotidyl transferase dUTP nick end labeling (TUNEL) is performed. It is confirmed that the number of TUNEL-positive cells in the retina of the artificial retina light-stimulated group is significantly lower than that of control mice.

### (Example 23: Effect of Suppressing Progress of Disuse Atrophy of Retinal Thickness)

Photoreceptor cell transplantation surgery is performed on rd1 mice. The protective effect is quantified in a light-stimulated group and a non-light-stimulated control group.

It is confirmed that the retinal thickness (inner retinal layer thickness) of the artificial retina light-stimulated group is thicker than that of controls by optical coherence tomography (OCT) or by the observation of sections with an optical microscope.

Furthermore, in order to detect retinal apoptosis, terminal deoxynucleotidyl transferase dUTP nick end labeling (TUNEL) is performed. It is confirmed that the number of TUNEL-positive cells in the retina of the artificial retina light-stimulated group is significantly lower than that of control mice.

### (Note)

As described above, the present disclosure has been illustrated using the preferred embodiments of the present disclosure; however, it is understood that the scope of the present disclosure should be interpreted only by the Claims thereof. It is understood that the contents of patents, patent applications and other documents cited herein should be incorporated herein by reference in the same way that the contents themselves thereof are specifically described herein. The present application claims priority to Japanese Patent Application No. 2020-156757 (filed on September 17, 2020) filed with the Japan Patent Office, the contents of which are incorporated herein by reference in the same manner as all of them are described in the present specification.

### [Industrial Applicability]

Compositions for treating or preventing a disease, disorder or symptom associated with endoplasmic reticulum stress or all-trans-retinal, as well as compositions for preserving a retinal thickness have been provided. Techniques are provided that are applicable to industries (pharmaceuticals, etc.) based on such techniques as described above.

### [Sequence Listing Free Text]

SEQ ID NO: 1: an example of the nucleic acid sequence consisting of a chimeric rhodopsin (GR/BvRh) and an endoplasmic reticulum export signal sequence
SEQ ID NO: 2: an example of the amino acid sequence consisting of a chimeric rhodopsin (GR/BvRh) and an endoplasmic reticulum export signal sequence
SEQ ID NO: 3: an example of the nucleic acid sequence consisting of a chimeric rhodopsin (GR/BvRh), an endoplasmic reticulum export signal sequence, and a FLAG tag
SEQ ID NO: 4: an example of the amino acid sequence consisting of a chimeric rhodopsin (GR/BvRh), an endoplasmic reticulum export signal sequence, and a FLAG tag
SEQ ID NO: 5: an example of the amino acid sequence of a chimeric rhodopsin (GR/BvRh)
SEQ ID NO: 6: an example of the nucleic acid sequence of a chimeric rhodopsin (GtACR2/BvRh)
SEQ ID NO: 7: an example of the nucleic acid sequence of a chimeric rhodopsin (GtACR2/BvRh)
SEQ ID NO: 8: an example of the amino acid sequence of a chimeric rhodopsin (GtACR2/BvRh)
SEQ ID NO: 9: the nucleic acid sequence of a human rhodopsin (huRh)
SEQ ID NO: 10: the amino acid sequence of a human rhodopsin (huRh)
SEQ ID NO: 11: the nucleic acid sequence of a bovine rhodopsin (BvRh)
SEQ ID NO: 12: the amino acid sequence of a bovine rhodopsin (BvRh)
SEQ ID NO: 13: the nucleic acid sequence of *Gloeobacter violaceus* Rhodopsin (GR)
SEQ ID NO: 14: the amino acid sequence of *Gloeobacter violaceus* Rhodopsin (GR)
SEQ ID NO: 15: the nucleic acid sequence of *Guillardia* theta anion channelrhodopsin2 (GtACR2)
SEQ ID NO: 16: the amino acid sequence of *Guillardia theta* anion channelrhodopsin2 (GtACR2)
SEQ ID NO: 17: an example of the nucleic acid sequence of the second loop on the cytoplasm side of the G protein-coupled receptor rhodopsin
SEQ ID NO: 18: an example of the nucleic acid sequence of the second loop on the cytoplasm side of the G protein-coupled receptor rhodopsin
SEQ ID NO: 19: an example of the amino acid sequence of the second loop on the cytoplasm side of the G protein-coupled receptor rhodopsin (corresponding to SEQ ID NO: 18)
SEQ ID NO: 20: an example of the nucleic acid sequence of the third loop on the cytoplasm side of the G protein-coupled receptor rhodopsin
SEQ ID NO: 21: an example of the nucleic acid sequence of the third loop on the cytoplasm side of the G protein-coupled receptor rhodopsin
SEQ ID NO: 22: an example of the amino acid sequence of the third loop on the cytoplasm side of the G protein-coupled receptor rhodopsin
SEQ ID NO: 23: an example of the nucleic acid sequence of the chimeric rhodopsin (GR/BvRh) (corresponding to SEQ ID NO: 8), where the start codon corresponds to nucleotides 43-45 and the stop codon corresponds to nucleotides 994-996
SEQ ID NO: 24: an example of the nucleic acid sequence of the second loop on the cytoplasm side of the G protein-coupled receptor rhodopsin
SEQ ID NO: 25: an example of the amino acid sequence of the second loop on the cytoplasm side of the G protein-coupled receptor rhodopsin (corresponding to SEQ ID NO: 24)

## Claims

1. A composition for use in treating or preventing a disease, disorder or symptom associated with an endoplasmic reticulum stress, the composition comprising a retinal modulating agent.

2. A composition for use in treating or preventing a disease, disorder or symptom associated with an endoplasmic reticulum stress, the composition comprising a light absorbing substance.

3. A composition for use in treating or preventing a disease, disorder or symptom associated with an endoplasmic reticulum stress, the composition comprising a neural activity signaling agent or device.

4. A composition for use in reducing or eliminating an endoplasmic reticulum stress, the composition comprising a light absorbing substance.

5. A composition for use in reducing or eliminating an endoplasmic reticulum stress, the composition comprising a neural activity signaling agent or device.

6. A composition for use in treating or preventing a disease, disorder or symptom associated with an endoplasmic reticulum stress, the composition comprising an opsin or a derivative thereof, or a nucleic acid molecule encoding the same.

7. A composition for use in ameliorating a rhodopsin transport disorder, the composition comprising a retinal modulating agent.

8. A composition for use in ameliorating a rhodopsin transport disorder, the composition comprising a light absorbing substance.

9. A composition for use in ameliorating a rhodopsin transport disorder, the composition comprising a neural activity signaling agent or device.

10. The composition of any one of claims 1 to 9, wherein the retinal modulating agent, the light absorbing substance, the neural activity signaling agent or device, or the opsin or derivative thereof modulates a cis/trans ratio of retinal in an organism body of interest.

11. The composition of any one of claims 1 to 10, wherein the retinal modulating agent, the light absorbing substance, the neural activity signaling agent or device, or the opsin or derivative thereof modulates a retinal concentration in an organism body of interest.

12. The composition of any one of claims 1 to 3, wherein the disease, disorder or symptom associated with the endoplasmic reticulum stress includes vesicle transport disorder, diabetes, diabetic retinopathy, myopia, macular degeneration (e.g., age-related macular degeneration), glaucoma, cataract, viral infection, corneal dystrophy, retinoblastoma, Alzheimer's disease, Parkinson's disease, and lifestyle-related disease.

13. The composition of any one of claims 1 to 12, **characterized in that** the composition is for treating or preventing a disease, disorder or symptom associated with an endoplasmic reticulum stress, for reducing or eliminating an endoplasmic reticulum stress, or for ameliorating a rhodopsin transport disorder, in the retina.

14. The composition of any one of claims 1 to 13, **characterized in that** the composition is administered to a subject prior to or after onset of the disease, disorder or symptom.

15. The composition of any one of claims 1 to 14, **characterized in that** the composition is administered once during a treatment period.

16. The composition of any one of claims 1 to 15, wherein the retinal modulating agent, the light absorbing substance, the neural activity signaling agent or device, or the opsin or derivative thereof is a rhodopsin that does not release retinal upon photoreception.

17. The composition of any one of claims 1 to 16, wherein the retinal modulating agent, the light absorbing substance, the neural activity signaling agent or device, or the opsin or derivative thereof is an ion-transporting receptor rhodopsin.

18. The composition of any one of claims 1 to 17, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.

19. The composition of any one of claims 1 to 18, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.

20. The composition of any one of claims 17 to 19, wherein the ion-transporting receptor rhodopsin is derived from cyanobacteria (blue-green bacteria).

21. The composition of claim 18 or 19, wherein the G protein-coupled receptor rhodopsin is derived from a mammal.

22. A composition for use in treating or preventing a disease, disorder or symptom associated with all-trans-retinal, the composition comprising a retinal modulating agent.

23. A composition for use in treating or preventing a disease, disorder or symptom associated with all-trans-retinal, the composition comprising a light absorbing substance.

24. A composition for use in treating or preventing a disease, disorder or symptom associated with all-trans-retinal, the composition comprising a neural activity signaling agent or device.

25. A composition for use in reducing or eliminating all-trans-retinal toxicity, the composition comprising a light absorbing substance.

26. A composition for use in reducing or eliminating all-trans-retinal toxicity, the composition comprising a neural activity signaling agent or device.

27. A composition for use in treating or preventing a disease, disorder or symptom associated with all-trans-retinal, the composition comprising an opsin or a derivative thereof, or a nucleic acid molecule encoding the same.

28. A composition for use in inhibiting cell death or apoptosis, the composition comprising a retinal modulating agent.

29. A composition for use in inhibiting cell death or apoptosis, the composition comprising a light absorbing substance.

30. A composition for use in inhibiting cell death or apoptosis, the composition comprising a neural activity signaling agent or device.

31. A composition for use in inhibiting cell death or apoptosis, the composition comprising an opsin or a derivative thereof, or a nucleic acid molecule encoding the same.

32. The composition of any one of claims 22 to 31, wherein the retinal modulating agent, the light absorbing substance, the neural activity signaling agent or device, or the opsin or derivative thereof modulates a cis/trans ratio of retinal in an organism body of interest.

33. The composition of any one of claims 22 to 32, wherein the retinal modulating agent, the light absorbing substance, the neural activity signaling agent or device, or the opsin or derivative thereof modulates a retinal concentration in an organism body of interest.

34. The composition of any one of claims 22 to 24 and 27, wherein the disease, disorder or symptom associated with all-trans-retinal comprises cell death or apoptosis.

35. The composition of any one of claims 22 to 34, **characterized in that** the composition is for treating or preventing a disease, disorder or symptom associated with all-trans-retinal, for reducing or eliminating all-trans-retinal toxicity, or for inhibiting cell death or apoptosis.

36. The composition of any one of claims 22 to 35, **characterized in that** the composition is administered to a subject prior to or after onset of the disease, disorder or symptom.

37. The composition of any one of claims 22 to 36, **characterized in that** the composition is administered once during a treatment period.

38. The composition of any one of claims 22 to 37, wherein the retinal modulating agent, the light absorbing substance, the neural activity signaling agent or device, or the opsin or derivative thereof is a rhodopsin that does not release retinal upon photoreception.

39. The composition of any one of claims 22 to 38, wherein the retinal modulating agent, the light absorbing substance, the neural activity signaling agent or device, or the opsin or derivative thereof is an ion-transporting receptor rhodopsin.

40. The composition of any one of claims 22 to 39, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.

41. The composition of any one of claims 22 to 40, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.

42. The composition of any one of claims 39 to 41, wherein the ion-transporting receptor rhodopsin is derived from cyanobacteria (blue-green bacteria).

43. The composition of claim 40 or 41, wherein the G protein-coupled receptor rhodopsin is derived from a mammal.

44. A composition for use in protecting a retinal thickness, the composition comprising a retinal modulating agent.

45. A composition for use in protecting a retinal thickness, the composition comprising a light absorbing substance.

46. A composition for use in protecting a retinal thickness, the composition comprising a neural activity signaling agent or device.

47. A composition for use in suppressing atrophy, or progress of atrophy, of a retinal thickness, the composition comprising a retinal modulating agent.

48. A composition for use in suppressing atrophy, or progress of atrophy, of a retinal thickness, the composition comprising a light absorbing substance.

49. A composition for use in suppressing atrophy, or progress of atrophy, of a retinal thickness, the composition comprising a neural activity signaling agent or device.

50. A composition for use in protecting a retinal thickness, the composition comprising an opsin or a derivative thereof, or a nucleic acid molecule encoding the same.

51. A composition for use in suppressing atrophy, or progress of atrophy, of a retinal thickness, the composition comprising an opsin or a derivative thereof, or a nucleic acid molecule encoding the same.

52. The composition of any one of claims 44 to 51, wherein the retinal modulating agent, the light absorbing substance, the neural activity signaling agent or device, or the opsin or derivative thereof modulates a cis/trans ratio of retinal in an organism body of interest.

53. The composition of any one of claims 44 to 52, wherein the retinal modulating agent, the light absorbing substance, the neural activity signaling agent or device, or the opsin or derivative thereof modulates a retinal concentration in an organism body of interest.

54. The composition of any one of claims 44 to 53, wherein the protection of the retinal thickness, or the suppression of atrophy, or progress of atrophy, of the retinal thickness, comprises: protection of an inner retinal layer thickness; or suppression of atrophy, or progress of atrophy, of an inner retinal layer thickness.

55. The composition of any one of claims 44 to 54, **characterized in that** the composition is administered to a subject prior to or after onset of atrophy of a retinal thickness.

56. The composition of any one of claims 44 to 55, **characterized in that** the composition is administered once during a treatment period.

57. The composition of any one of claims 44 to 56, wherein the retinal modulating agent, the light absorbing substance, the neural activity signaling agent or device, or the opsin or derivative thereof is a rhodopsin that does not release retinal upon photoreception.

58. The composition of any one of claims 44 to 57, wherein the retinal modulating agent, the light absorbing substance, the neural activity signaling agent or device, or the opsin or derivative thereof is an ion-transporting receptor rhodopsin.

59. The composition of any one of claims 44 to 58, wherein the retinal modulating agent, the light absorbing substance, the neural activity signaling agent or device, or the opsin or derivative thereof is: a chimeric protein of an ion-transporting receptor rhodopsin and a G protein-coupled receptor rhodopsin; a vertebrate non-visual opsin; an invertebrate opsin; or a microbial opsin.

60. The composition of any one of claims 44 to 59, wherein the retinal modulating agent, the light absorbing substance, the neural activity signaling agent or device, or the opsin or derivative thereof comprises a nucleic acid encoding: a chimeric protein of an ion-transporting receptor rhodopsin and a G protein-coupled receptor rhodopsin; a vertebrate non-visual opsin; an invertebrate opsin; or a microbial opsin.

61. The composition of any one of claims 58 to 60, wherein the ion-transporting receptor rhodopsin is derived from cyanobacteria (blue-green bacteria).

62. The composition of claim 59 or 60, wherein the G protein-coupled receptor rhodopsin is derived from a mammal.
